(19) Europäisches Patentamt
European Patent Office
Office européen des brevets

(11) EP 4 431 486 A1

(12) EUROPEAN PATENT APPLICATION
published in accordance with Art. 153(4) EPC

(43) Date of publication:
**18.09.2024 Bulletin 2024/38**

(21) Application number: **22892546.7**

(22) Date of filing: **21.10.2022**

(51) International Patent Classification (IPC):
***C07C 15/38*** *(2006.01)* ***C07C 15/27*** *(2006.01)*
***C07D 213/16*** *(2006.01)* ***C07D 221/18*** *(2006.01)*
***C07D 307/91*** *(2006.01)* ***C07D 333/76*** *(2006.01)*
***C07D 401/04*** *(2006.01)* ***C09K 11/06*** *(2006.01)*
***H05B 33/12*** *(2006.01)* ***H10K 50/00*** *(2023.01)*

(52) Cooperative Patent Classification (CPC):
**C07C 15/27; C07C 15/38; C07D 213/16; C07D 221/18; C07D 307/91; C07D 333/76; C07D 401/04; C09K 11/06; H05B 33/12; H10K 50/00**

(86) International application number:
**PCT/JP2022/039309**

(87) International publication number:
**WO 2023/085046 (19.05.2023 Gazette 2023/20)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **12.11.2021 JP 2021184897**

(71) Applicant: **CANON KABUSHIKI KAISHA**
**Tokyo 146-8501 (JP)**

(72) Inventors:
- **MIYASHITA Hirokazu**
  **Tokyo 146-8501 (JP)**
- **YAMADA Naoki**
  **Tokyo 146-8501 (JP)**
- **OHRUI Hiroki**
  **Tokyo 146-8501 (JP)**
- **IWAWAKI Hironobu**
  **Tokyo 146-8501 (JP)**
- **NISHIDE Yosuke**
  **Tokyo 146-8501 (JP)**
- **KAMATANI Jun**
  **Tokyo 146-8501 (JP)**

(74) Representative: **TBK**
**Bavariaring 4-6**
**80336 München (DE)**

(54) **ORGANIC COMPOUND, ORGANIC LIGHT EMITTING ELEMENT, DISPLAY DEVICE, PHOTOELECTRIC CONVERSION DEVICE, ELECTRONIC APPARATUS, ILLUMINATION DEVICE, MOVABLE BODY, AND EXPOSURE LIGHT SOURCE**

(57) The present disclosure provides an organic compound represented by the following general formula [1].

EP 4 431 486 A1

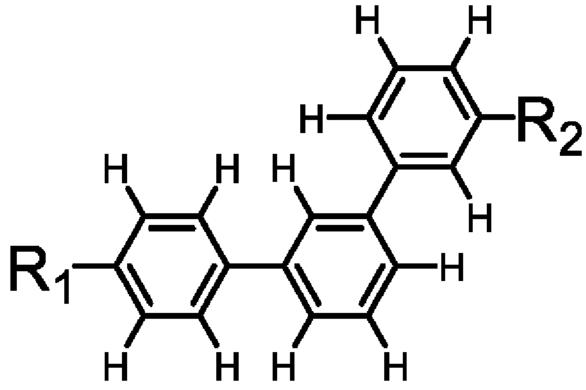

[1]

## Description

### Technical Field

The present invention relates to an organic compound, an organic light-emitting element, a display apparatus, a photoelectric conversion apparatus, electronic equipment, a lighting apparatus, a moving body, and an exposure light source.

### Background Art

An organic light-emitting element (hereinafter sometimes referred to as an "organic electroluminescent element" or an "organic EL element") is an electronic element that includes a pair of electrodes and an organic compound layer between the electrodes. Electrons and holes are injected from the pair of electrodes to generate an exciton of a light-emitting organic compound in the organic compound layer. When the exciton returns to its ground state, the organic light-emitting element emits light.

With recent significant advances in organic light-emitting elements, it is characteristically possible to realize low drive voltage, various emission wavelengths, high-speed responsivity, and thin and lightweight light-emitting devices.

Compounds suitable for organic light-emitting elements have been actively developed. This is because a compound that provides an element with good lifetime characteristics is important for high-performance organic light-emitting elements.

As compounds that have been developed, Patent Literature 1 describes the following compound 1-A, and Patent Literature 2 describes the following compound 1-B. Both of them are compounds with a fused polycyclic group substituted at an end of a phenylene chain.

[Chem. 1]

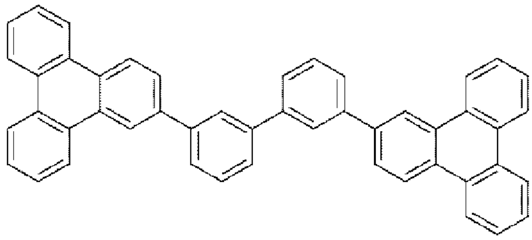

1 − A

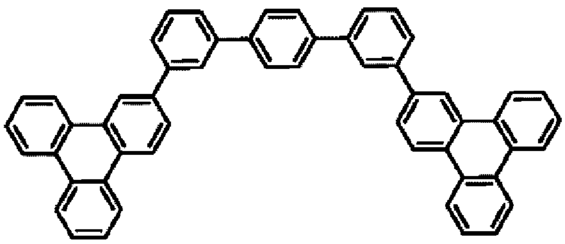

1 − B

### Citation List

### Patent Literature

PTL 1: International Publication No. WO 2006/130598

PTL 2: International Publication No. WO 2012/050008

## Summary of Invention

### Technical Problem

Investigation by the present inventors showed that the compound 1-A described in Patent Literature 1 and the compound 1-B described in Patent Literature 2 have room for improvement in element life characteristics when used in an organic light-emitting element.

### Solution to Problem

In view of such a situation, it is an object of the present invention to provide an organic compound that can be used for an organic light-emitting element with good element life characteristics. It is another object of the present invention to provide an organic light-emitting element with good element life characteristics.

An organic compound according to one aspect of the present invention is represented by the following general formula [1]:

[Chem. 2]

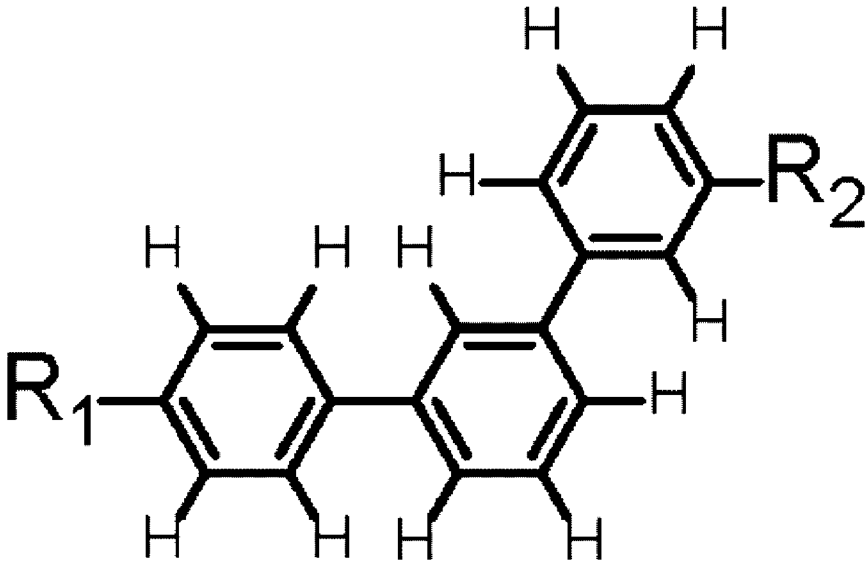

[1]

(In the general formula [1], $R_1$ and $R_2$ are each independently selected from the following substituent A group.

(Substituent A Group)

[Chem. 3]

A:

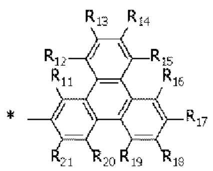

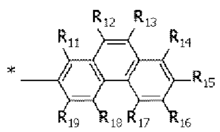

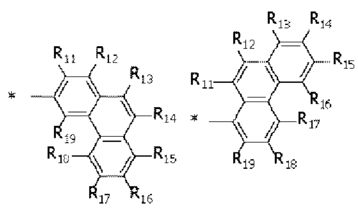

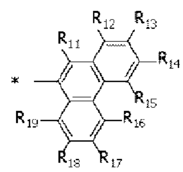

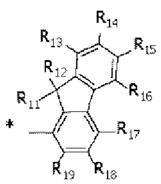

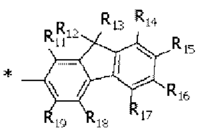

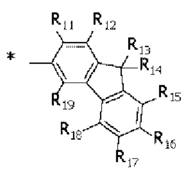

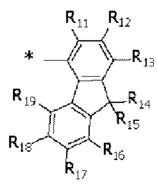

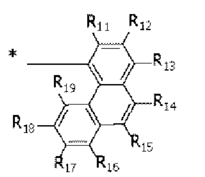

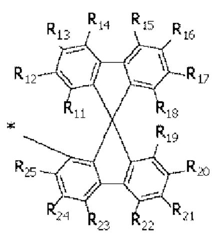

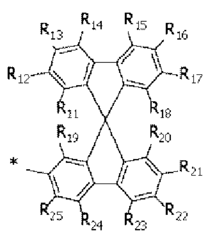

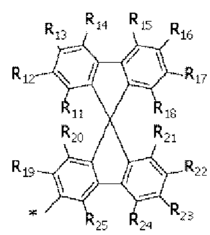

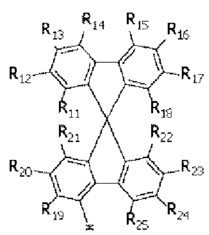

[Chem. 4]

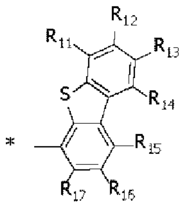
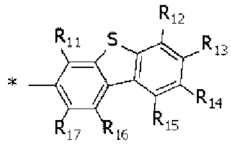
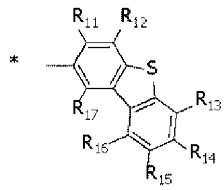
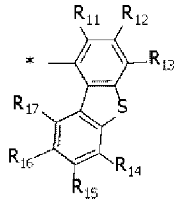
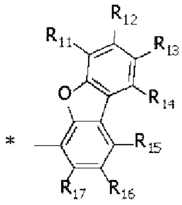
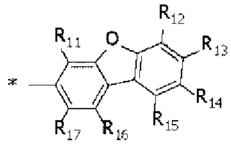
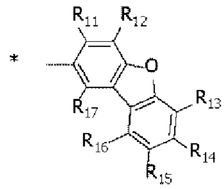
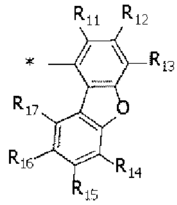
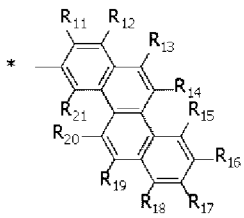
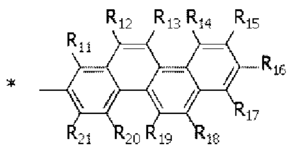
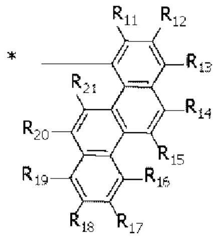
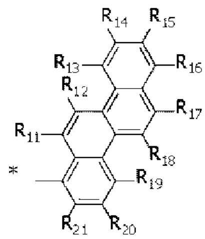
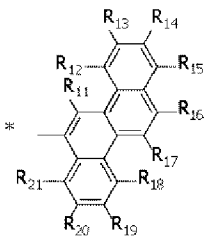

[Chem. 5]

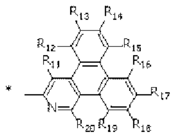
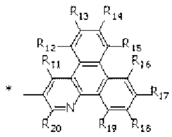
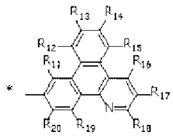
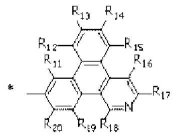

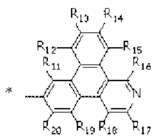
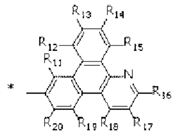

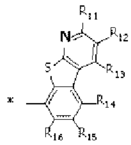
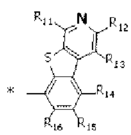
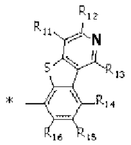
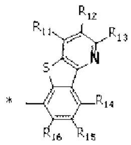
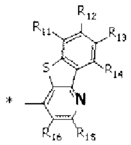
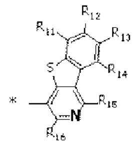
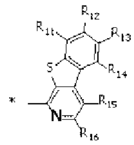

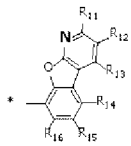
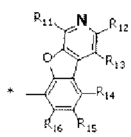
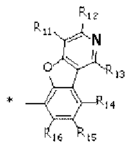
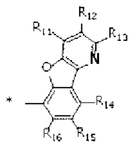
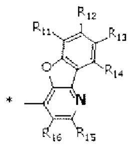
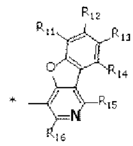
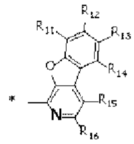

In the substituent A group, $R_{101}$ to $R_{506}$ are each independently selected from a hydrogen atom, a deuterium atom, a halogen atom, a substituted or unsubstituted alkyl group, a substituted or unsubstituted alkoxy group, a substituted or unsubstituted amino group, an aryloxy group, a silyl group, an aromatic hydrocarbon group, a heterocyclic group, and a cyano group.)

Advantageous Effects of Invention

The present invention can provide an organic compound that can be used for an organic light-emitting element with good element life characteristics.

Brief Description of Drawings

[Fig. 1A] Fig. 1A is a schematic cross-sectional view of an example of a pixel of a display apparatus according to an embodiment of the present invention.
[Fig. 1B] Fig. 1B is a schematic cross-sectional view of an example of a display apparatus including an organic light-emitting element according to an embodiment of the present invention.
[Fig. 2] Fig. 2 is a schematic view of an example of a display apparatus according to an embodiment of the present invention.
[Fig. 3A] Fig. 3A is a schematic view of an example of an imaging apparatus according to an embodiment of the present invention.
[Fig. 3B] Fig. 3B is a schematic view of an example of a mobile device according to an embodiment of the present invention.
[Fig. 4A] Fig. 4A is a schematic view of an example of a display apparatus according to an embodiment of the present invention.
[Fig. 4B] Fig. 4B is a schematic view of an example of a foldable display apparatus.
[Fig. 5A] Fig. 5A is a schematic view of an example of a lighting apparatus according to an embodiment of the present invention.
[Fig. 5B] Fig. 5B is a schematic view of an automobile as an example of a moving body according to an embodiment of the present invention.
[Fig. 6A] Fig. 6A is a schematic view of an example of a wearable device according to an embodiment of the present invention.
[Fig. 6B] Fig. 6B is a schematic view of an example of a wearable device according to an embodiment of the present invention including an imaging apparatus.

[Fig. 7] Fig. 7 is a schematic view of an example of an image-forming apparatus according to an embodiment of the present invention.

[Fig. 8A] Fig. 8A is a schematic view of an example of an exposure light source for an image-forming apparatus according to an embodiment of the present invention.

[Fig. 8B] Fig. 8B is a schematic view of an example of an exposure light source for an image-forming apparatus according to an embodiment of the present invention. Description of Embodiments

<<Organic Compound>>

First, an organic compound according to the present embodiment is described below.

The organic compound according to the present embodiment is an organic compound represented by the following general formula [1].

[Chem. 6]

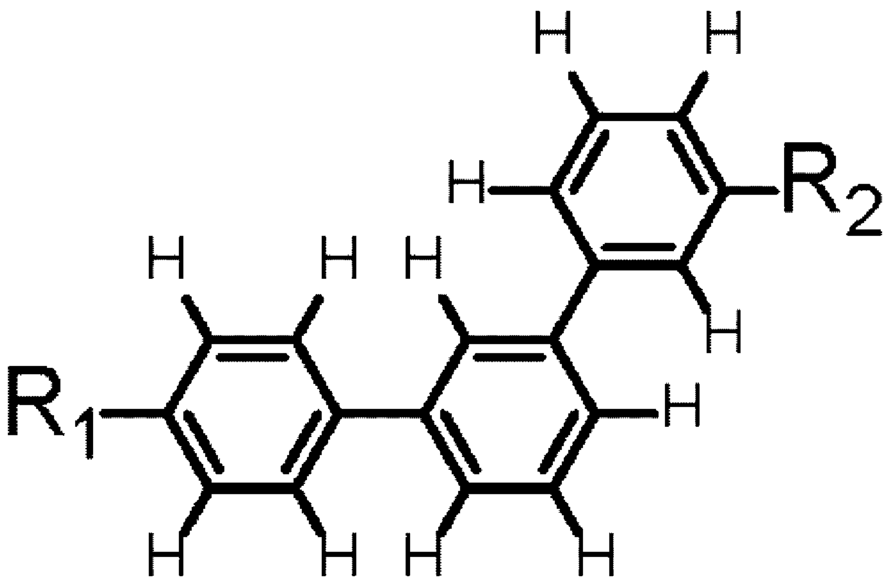

[1]

In the general formula [1], $R_1$ and $R_2$ are each independently selected from the following substituent A group.

(Substituent A Group)

[Chem. 7]

A: 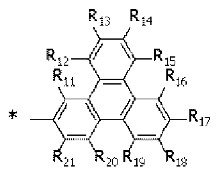 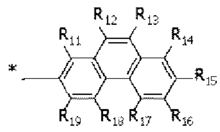 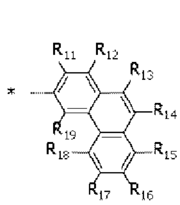 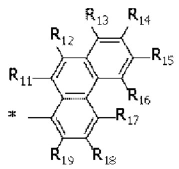 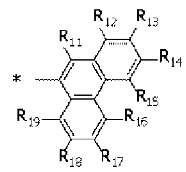

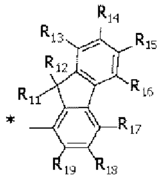 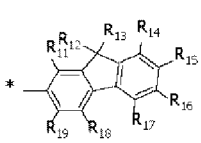 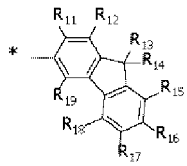 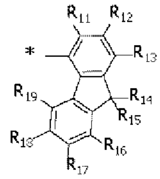 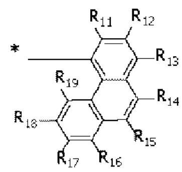

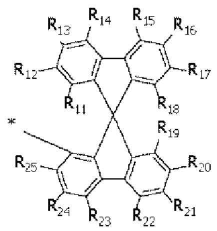 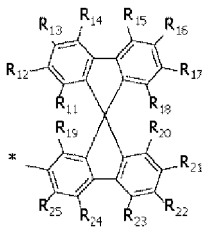 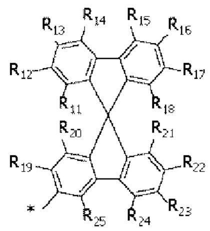 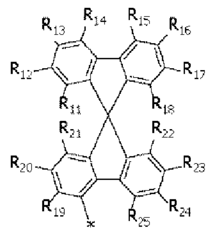

[Chem. 8]

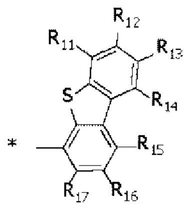
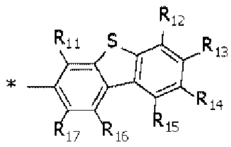
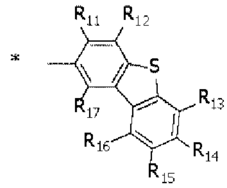
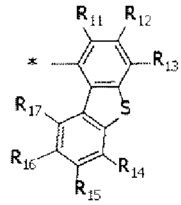
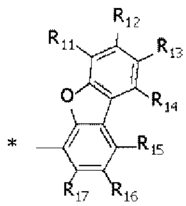
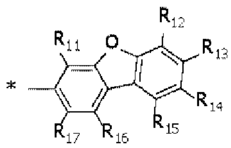
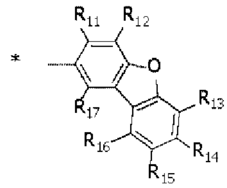
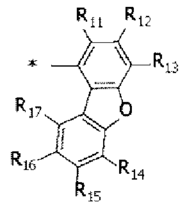
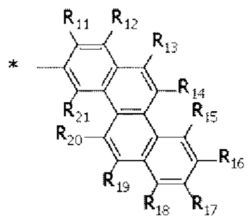
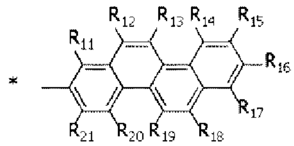
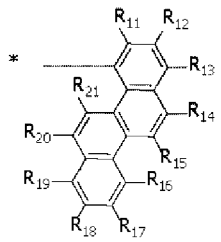
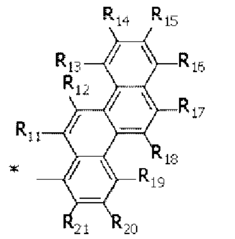
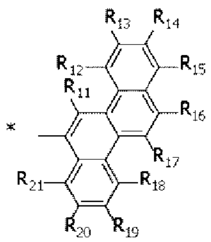

[Chem. 9]

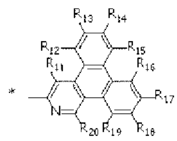
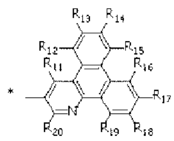
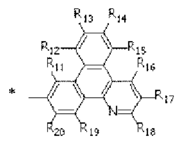
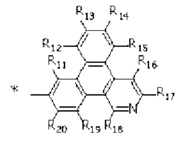

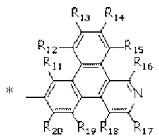
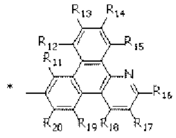

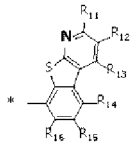
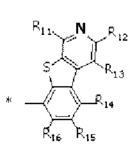
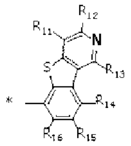
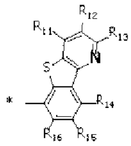
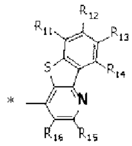
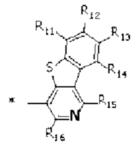
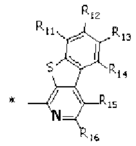

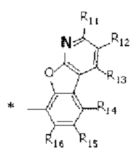
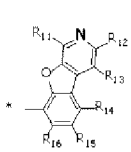
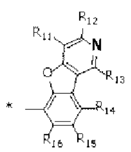
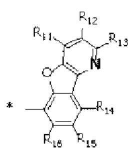
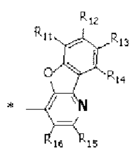
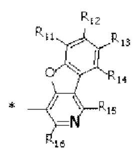
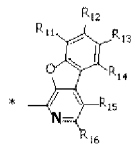

In the substituent A group, $R_{101}$ to $R_{506}$ are each independently selected from a hydrogen atom, a deuterium atom, a halogen atom, a substituted or unsubstituted alkyl group, a substituted or unsubstituted alkoxy group, a substituted or unsubstituted amino group, an aryloxy group, a silyl group, an aromatic hydrocarbon group, a heterocyclic group, and a cyano group.

In the general formula [1], at least one of $R_1$ and $R_2$ is preferably each independently selected from the following substituent B group.

(Substituent B Group)

[Chem. 10]

B : 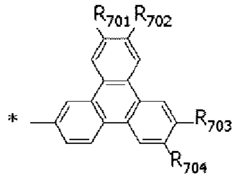

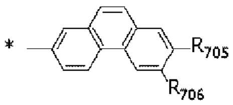 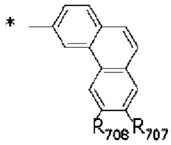 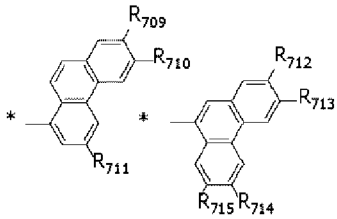 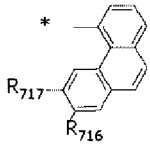

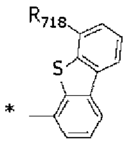 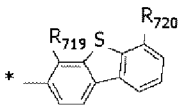 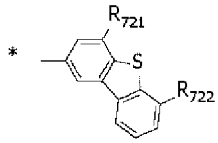 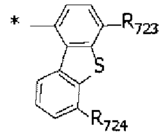

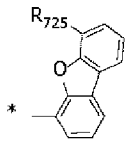 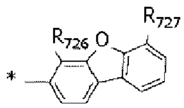 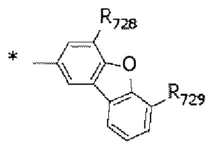 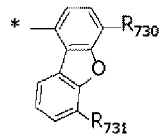

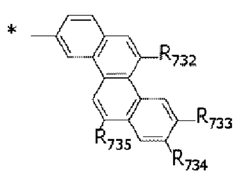 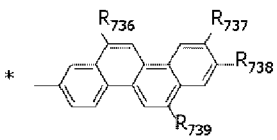 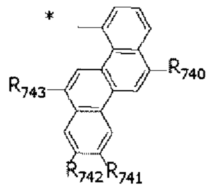 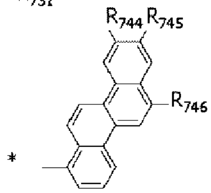

In the substituent B group, $R_{701}$ to $R_{746}$ are each independently selected from a hydrogen atom, a deuterium atom, a halogen atom, a substituted or unsubstituted alkyl group, a substituted or unsubstituted alkoxy group, a substituted or unsubstituted amino group, an aryloxy group, a silyl group, an aromatic hydrocarbon group, a heterocyclic group, and a cyano group.

In the general formula [1], at least one of $R_1$ and $R_2$ is preferably each independently selected from the following substituent C group.

(Substituent C Group)

[Chem. 11]

C :

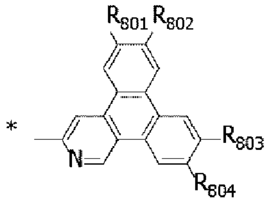 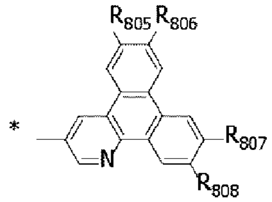 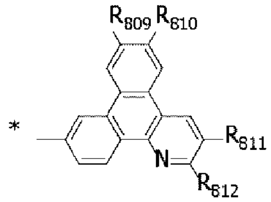

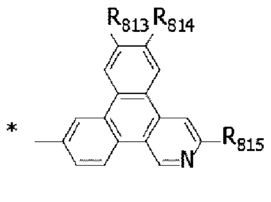 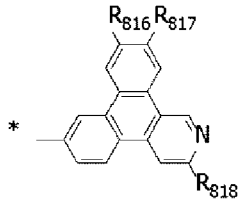 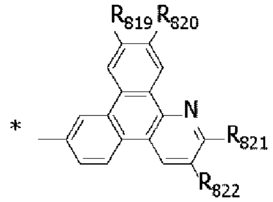

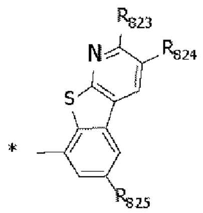 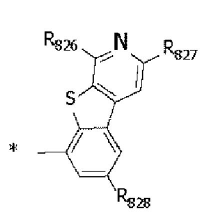 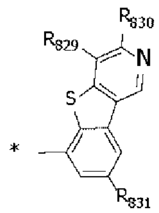 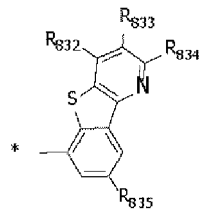

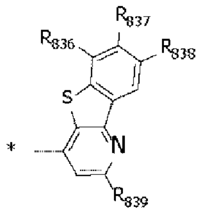 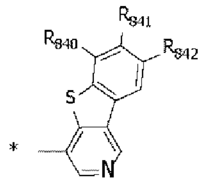 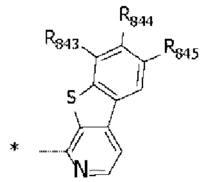

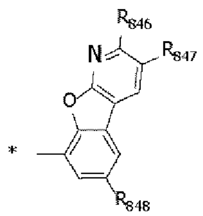 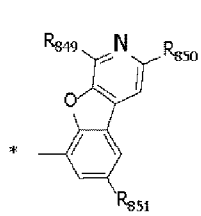 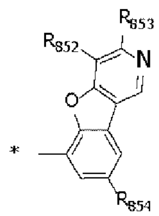 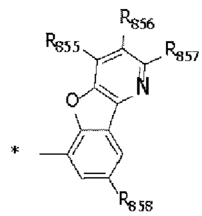

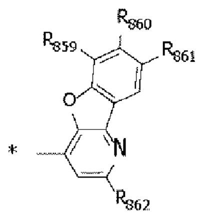 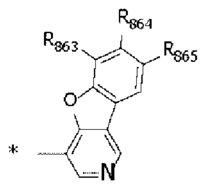 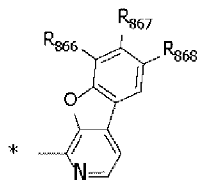

In the substituent C group, $R_{801}$ to $R_{868}$ are each independently selected from a hydrogen atom, a deuterium atom, a halogen atom, a substituted or unsubstituted alkyl group, a substituted or unsubstituted alkoxy group, a substituted or unsubstituted amino group, an aryloxy group, a silyl group, an aromatic hydrocarbon group, a heterocyclic group, and a cyano group.

In the general formula [1], at least one of $R_1$ and $R_2$ is particularly preferably each independently selected from the following substituent D group.

(Substituent D Group)

[Chem. 12]

D :

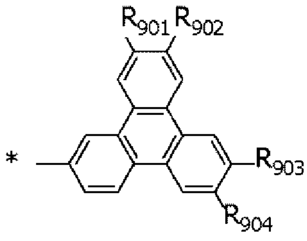

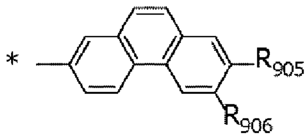

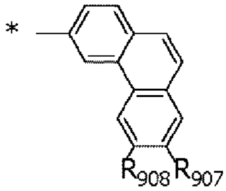

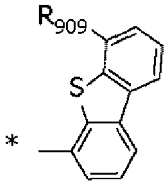

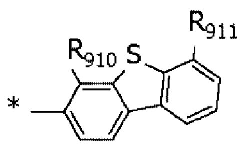

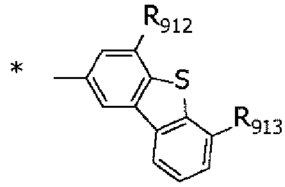

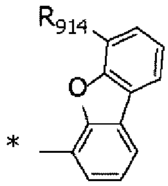

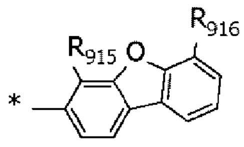

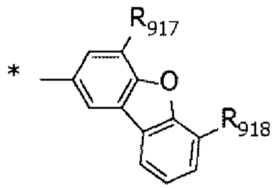

In the substituent D group, $R_{901}$ to $R_{918}$ are each independently selected from a hydrogen atom, a deuterium atom, a halogen atom, a substituted or unsubstituted alkyl group, a substituted or unsubstituted alkoxy group, a substituted or unsubstituted amino group, an aryloxy group, a silyl group, an aromatic hydrocarbon group, a heterocyclic group, and a cyano group.

The halogen atom represented by each of $R_{101}$ to $R_{506}$, $R_{701}$ to $R_{746}$, $R_{801}$ to $R_{868}$, and $R_{901}$ to $R_{918}$ may be, but is not limited to, fluorine, chlorine, bromine, iodine, or the like.

The alkyl group represented by each of $R_{101}$ to $R_{506}$, $R_{701}$ to $R_{746}$, $R_{801}$ to $R_{868}$, and $R_{901}$ to $R_{918}$ may be, but is not limited to, a methyl group, an ethyl group, a n-propyl group, an isopropyl group, a n-butyl group, a t-butyl group, a sec-butyl group, an octyl group, a cyclohexyl group, a 1-adamantyl group, a 2-adamantyl group, or the like.

The alkoxy group represented by each of $R_{101}$ to $R_{506}$, $R_{701}$ to $R_{746}$, $R_{801}$ to $R_{868}$, and $R_{901}$ to $R_{918}$ may be, but is not limited to, a methoxy group, an ethoxy group, a propoxy group, a 2-ethyl-octyloxy group, a benzyloxy group, or the like.

The amino group represented by each of $R_{101}$ to $R_{506}$, $R_{701}$ to $R_{746}$, $R_{801}$ to $R_{868}$, and $R_{901}$ to $R_{918}$ may be, but is not limited to, an N-methylamino group, an N-ethylamino group, an N,N-dimethylamino group, an N,N-diethylamino group, an N-methyl-N-ethylamino group, an N-benzylamino group, an N-methyl-N-benzylamino group, an N,N-dibenzylamino group, an anilino group, an N,N-diphenylamino group, an N,N-dinaphthylamino group, an N,N-difluorenylamino group, an N-phenyl-N-tolylamino group, an N,N-ditolylamino group, an N-methyl-N-phenylamino group, an N,N-dianisolylamino group, an N-mesityl-N-phenylamino group, an N,N-dimesitylamino group, an N-phenyl-N-(4-t-butylphenyl)amino group, an N-phenyl-N-(4-trifluoromethylphenyl)amino group, an N-piperidyl group, or the like.

The aryloxy group or heteroaryloxy group represented by each of $R_{101}$ to $R_{506}$, $R_{701}$ to $R_{746}$, $R_{801}$ to $R_{868}$, and $R_{901}$ to $R_{918}$ may be, but is not limited to, a phenoxy group, a thienyloxy group, or the like.

The silyl group represented by each of $R_{101}$ to $R_{506}$, $R_{701}$ to $R_{746}$, $R_{801}$ to $R_{868}$, and $R_{901}$ to $R_{918}$ may be, but is not limited to, a trimethylsilyl group, a triphenylsilyl group, or the like.

The aromatic hydrocarbon group represented by each of $R_{101}$ to $R_{506}$, $R_{701}$ to $R_{746}$, $R_{801}$ to $R_{868}$, and $R_{901}$ to $R_{918}$ may be, but is not limited to, a phenyl group, a naphthyl group, an indenyl group, a biphenyl group, a terphenyl group, a fluorenyl group, a phenanthryl group, a fluoranthenyl group, a triphenylenyl group, or the like.

The heterocyclic group represented by each of $R_{101}$ to $R_{506}$, $R_{701}$ to $R_{746}$, $R_{801}$ to $R_{868}$, and $R_{901}$ to $R_{918}$ may be, but is not limited to, a pyridyl group, an oxazolyl group, an oxadiazolyl group, a thiazolyl group, a thiadiazolyl group, a carbazolyl group, an acridinyl group, a phenanthrolyl group, a dibenzofuranyl group, a dibenzothiophenyl group, or the like.

The additional optional substituent of the alkyl group, alkoxy group, amino group, aryloxy group, silyl group, aromatic hydrocarbon group, and heterocyclic group may be, but is not limited to, a halogen atom, such as fluorine, chlorine, bromine, or iodine; an alkyl group, such as a methyl group, an ethyl group, a n-propyl group, an isopropyl group, a n-butyl group, or a t-butyl group; an alkoxy group, such as a methoxy group, an ethoxy group, or a propoxy group; an amino group, such as a dimethylamino group, a diethylamino group, a dibenzylamino group, a diphenylamino group, or a ditolylamino group; an aryloxy group, such as a phenoxy group; an aromatic hydrocarbon group, such as a phenyl group or a biphenyl group; a heterocyclic group, such as a pyridyl group or a pyrrolyl group; or a cyano group.

(Method for Synthesizing Organic Compound)

Next, a method for synthesizing the organic compound according to the present embodiment is described. For example, the organic compound according to the present embodiment is synthesized in accordance with the following reaction scheme.

[Chem. 13]

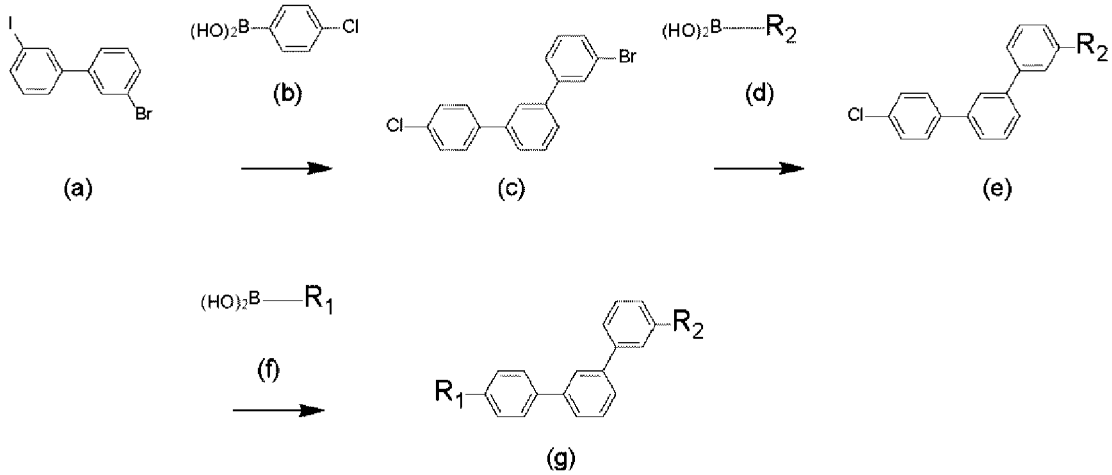

(a) (b) (c) (d) (e) (f) (g)

The compounds represented by (d) and (f) can be appropriately chosen to produce various compounds. The present invention is not limited to the synthesis scheme described above, and various synthesis schemes and reagents can be used. The synthesis method is described in detail in exemplary embodiments.

Characteristics of Organic Compounds According to the Present Embodiment

Next, characteristics of the organic compound according to the present embodiment are described.

The organic compound according to the present embodiment has the following characteristics and therefore has a small energy difference $\Delta ST$ between $S_1$ (excited singlet state) and $T_1$ (excited triplet state). It also has the following characteristics and therefore has good amorphousness. It also has the following characteristics and therefore has high sublimability. Furthermore, the organic compound can be used to provide an organic light-emitting element with high light emission efficiency and durability.

(1) A low $\Delta ST$ due to a substituent composed of a tricyclic or higher polycyclic fused ring present at positions 4 and 3" of 1,1':3',1"-terphenyl.
(2) Low symmetry due to a substituent composed of a tricyclic or higher polycyclic fused ring present at positions 4 and 3" of 1,1':3',1"-terphenyl.
(3) High stability as a compound due to no substituent at a position other than positions 4 and 3" of 1,1':3',1"-terphenyl.

These characteristics are described below with reference to comparative compounds 1-A to 1-C as comparison targets.

(1) A low ΔST due to a substituent composed of a tricyclic or higher polycyclic fused ring present at positions 4 and 3" of 1,1':3',1"-terphenyl.

The present inventors have focused on the structure of a linking group composed of a phenylene chain in the invention of an organic compound according to the present invention. The organic compound according to the present embodiment has a structure with a substituent composed of a tricyclic or higher polycyclic fused ring at positions 4 and 3" of 1,1':3',1"-terphenyl. In other words, the organic compound according to the present embodiment has a structure with two fused rings linked by a linking group 1,1':3',1"-terphenyl. The linking group 1,1':3',1"-terphenyl decreases the $S_1$ energy level and increases the $T_1$ energy level of the organic compound. This results in the organic compound with a lower ΔST (= $S_1$ - $T_1$).

Table 1 shows comparison results of the $S_1$ energy level and the $T_1$ energy level between an exemplary compound A4, which is an example of the organic compound according to the present embodiment, and the comparative compounds 1-A and 1-B. The $S_1$ energy level was measured by photoluminescence (PL) measurement of an evaporated film using F-4500 manufactured by Hitachi, Ltd. at room temperature at an excitation wavelength of 350 nm and was calculated from an emission edge at a rising portion of an emission spectrum. The $T_1$ energy level was measured by photoluminescence (PL) measurement of an evaporated film using F-4500 manufactured by Hitachi, Ltd. at 77 K at an excitation wavelength of 350 nm and was calculated from an emission edge at a rising portion on the short-wavelength side of the resulting emission spectrum. The $S_1$ energy level and the $T_1$ energy level were measured using an evaporated film sample deposited on a quartz substrate in a vacuum of 5 x $10^{-4}$ Pa or less.

[Table 1]

Table 1

| Compound | Structure | $S_1$ [eV] | $T_1$ [eV] | ΔST [eV] |
|---|---|---|---|---|
| Exemplary compound A4 | 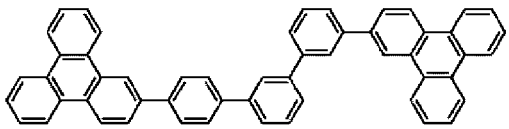 | 3.28 | 2.50 | 0.78 |
| Comparative compound 1-A | 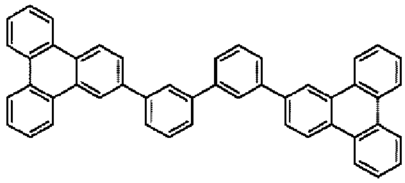 | 3.46 | 2.50 | 1.04 |
| Comparative compound 1-B | 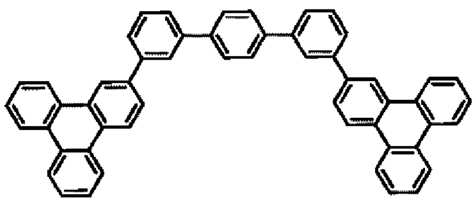 | 3.48 | 2.52 | 1.06 |

As shown in Table 1, the exemplary compound A4 had an $S_1$ energy level of 3.28 eV, a $T_1$ energy level of 2.50 eV, and a ΔST of 0.78 eV. On the other hand, the comparative compound 1-A had an $S_1$ energy level of 3.46 eV, a $T_1$ energy level of 2.50 eV, and a ΔST of 1.04 eV. The comparative compound 1-B had an $S_1$ energy level of 3.48 eV, a $T_1$ energy level of 2.52 eV, and a ΔST of 1.06 eV. This shows that the exemplary compound A4 has a lower ΔST than the comparative compounds 1-A and 1-B.

This is probably due to the structure with two fused rings linked at the positions 4 and 3" of the linking group 1,1'3',1"-terphenyl. The 1,1'3',1"-terphenyl linking the two structures at the positions 4 and 3" has three phenylene chains as shown below. Of the three phenylene chains, one phenylene chain is a phenylene chain linked at the para position

(a portion surrounded by a broken line rectangle below), and two phenylene chains are phenylene chains linked at the meta position (a portion surrounded by a broken line circle below). Conjugation extended by the phenylene chain linked at the para position lowers the $S_1$ energy level. On the other hand, conjugation shortened by the phenylene chains linked at the meta position can maintain a high $T_1$ energy level.

[Chem. 14]

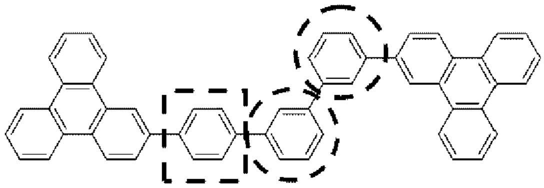

The effect of a low ΔST is described below.

In an organic light-emitting element utilizing phosphorescence, $T_1$ of a phosphorescent material (guest) emitting phosphorescence is used for light emission. Thus, a light-emitting layer host material of the organic light-emitting element utilizing phosphorescence needs to have a higher $T_1$ energy level than the phosphorescent material (guest) emitting phosphorescence.

In general, the $S_1$ energy level tends to increase with the $T_1$ energy level. A high $S_1$ energy level results in a large band gap. A large band gap results in a decrease in the injectability of a hole or an electron from a peripheral layer of a light-emitting layer. This may result in an increase in the voltage of the element or a decrease in element durability due to unnecessary charge accumulation. Thus, a lower $S_1$ energy level is preferred from the perspective of enhancing the injectability of a hole or an electron from the peripheral layer of the light-emitting layer.

Thus, a light-emitting layer host material of an organic light-emitting element utilizing phosphorescence preferably has a high $T_1$ energy level and a low $S_1$ energy level. In other words, an organic compound with a low ΔST (= $S_1$ - $T_1$) is preferred.

As described above, the organic compound according to the present embodiment has the linking group 1,1':3',1"-terphenyl, has a substituent at the positions 4 and 3", and therefore has a low ΔST. Thus, the organic compound used as a light-emitting layer host material of an organic light-emitting element enables low-voltage driving and improves element life characteristics. It also improves light emission efficiency.

(2) Low symmetry due to a substituent composed of a tricyclic or higher polycyclic fused ring present at positions 4 and 3" of 1,1'3',1"-terphenyl.

In the invention of an organic compound according to the present invention, the present inventors have focused on the symmetry of the organic compound. The organic compound according to the present embodiment has a structure with a tricyclic or higher polycyclic fused ring at the positions 4 and 3" of 1,1'3',1"-terphenyl. Thus, it has a structure with low symmetry.

Table 2 shows comparison results of the symmetry between the exemplary compound A4, which is an example of the organic compound according to the present embodiment, and the comparative compounds 1-A and 1-B.

[Table 2]

| | Structure | Glass transition temperature (°C) | Crystallization temperature (°C) |
|---|---|---|---|
| Exemplary compound A4 | 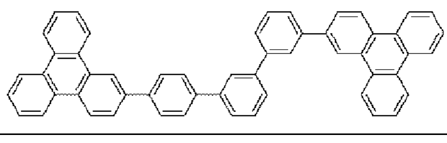 | 141 | Not observed |
| Comparative compound 1-A | 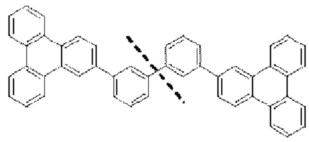 | 130 | 218 |

(continued)

| | Structure | Glass transition temperature (°C) | Crystallization temperature (°C) |
|---|---|---|---|
| Comparative compound 1-B | 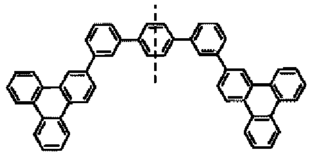 | 141 | 236 |

As shown in Table 2, each of the exemplary compound A4, the comparative compound 1-A, and the comparative compound 1-B has a structure with two triphenylene rings linked by a linking group composed of a phenylene chain. When the molecular structure is viewed as a plane, the symmetry of the molecular structure changes with the binding position of the phenylene chain in the linking group that links the triphenylene rings at both ends.

As shown in Table 2, the comparative compounds 1-A and 1-B have high symmetry due to a structure with twofold symmetry with respect to an axis passing through the middle of the two phenylene chains constituting the linking group and perpendicular to the molecular plane. On the other hand, the exemplary compound A4 has a planar structure with no rotation axis or symmetry axis and therefore has lower symmetry than the comparative compounds 1-A and 1-B.

A compound with low symmetry has the following effects.

The first effect is to prevent the overlapping of molecules in molecular packing, reduce crystallization, and increase amorphousness. Enhanced amorphousness, that is, better film properties are preferred for an organic light-emitting element.

This is because high amorphousness reduces the generation of crystal grain boundaries, trap levels, and quenchers associated with fine crystallization even during the operation of the element, and high carrier transport ability and efficient emission properties can be maintained. Consequently, an organic light-emitting element with high durability and efficiency can be provided.

Table 2 shows evaluation results of the glass transition temperature and the crystallization temperature of the exemplary compound A4, which is an example of the organic compound according to the present embodiment, and the comparative compounds 1-A and 1-B by differential scanning calorimetry (DSC).

It can be said that a higher glass transition temperature and a higher crystallization temperature or no crystallization temperature observed result in higher amorphousness and thermal stability. In the DSC measurement, the glass transition temperature and the crystallization temperature were measured by rapidly cooling approximately 2 mg of a sample sealed in an aluminum pan from a high temperature exceeding the melting point to bring the sample into an amorphous state and then heating the sample at a heating rate of 10°C/min. DSC 204F1 manufactured by NETZSCH was used as a measuring apparatus.

The comparative compound 1-A had a glass transition temperature of 130°C and had a crystallization temperature observed at 218°C during the temperature rise. Thus, it can be said that the comparative compound 1-A is a compound with low amorphousness and low thermal stability.

By contrast, the exemplary compound A4 had a glass transition temperature of 141°C and had no crystallization temperature observed during the temperature rise. Thus, the exemplary compound A4 is a compound with high amorphousness and higher thermal stability. Thus, the exemplary compound A4 can be used for an organic light-emitting element to maintain a stable amorphous film even during the operation of the element and to provide a long-life organic light-emitting element.

The second effect is to decrease the sublimation temperature and enhance sublimability.

This is because an organic compound with lower symmetry is less likely to aggregate. Table 3 shows comparison results of the sublimability of the exemplary compound A4, which is an example of the organic compound according to the present embodiment, and the comparative compound 1-B.

The sublimability was evaluated by the temperature difference $\Delta T$ between the sublimation temperature and the decomposition temperature (= decomposition temperature - sublimation temperature). A higher temperature difference indicates higher sublimability. The decomposition temperature is a temperature at which the weight loss reaches 5% in TG/DTA measurement. The sublimation temperature is a temperature at which a sufficient sublimation rate is achieved while the temperature is slowly increased in a vacuum of 1 x $10^{-1}$ Pa in an Ar flow to perform sublimation purification.

Table 3]

| | Structure | Molecular weight | ΔT = decomposition temperature - sublimation temperature |
|---|---|---|---|
| Exemplary compound A4 | 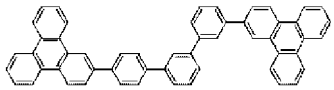 | 682 | 70 |
| Comparative compound 1-B | 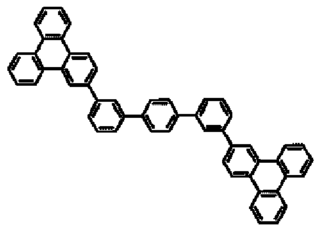 | 682 | 60 |

Table 3 shows that, although the exemplary compound A4 and the comparative compound 1-B have the same molecular weight, the exemplary compound A4 has a larger temperature difference ΔT between the sublimation temperature and the decomposition temperature and is a material with high sublimability.

Due to high sublimability, sublimation purification can be stably performed without decomposition. High sublimability results in high vapor deposition stability in the production of an organic light-emitting element. More specifically, a high-purity evaporated film can be formed without decomposition during vapor deposition, and a long-life organic light-emitting element can be provided.

(3) High stability as a compound due to no substituent at a position other than positions 4 and 3" of 1,1':3',1"-terphenyl.

The present inventors have focused on the structure of a coupler composed of a phenylene chain in the invention of an organic compound according to the present invention. More specifically, the organic compound according to the present embodiment has a structure with two fused rings linked by a linking group 1,1':3',1"-terphenyl. In the present embodiment, the linking group 1,1'3',1"-terphenyl is characterized by no substituent other than the fused rings at the positions 4 and 3". With such a structure, the compound is stable due to no substituent that increases the bond length.

For example, as shown in Table 4, the maximum bond length is compared between the exemplary compound A4, which is an example of the organic compound according to the present embodiment, and the comparative compound 1-C. The comparative compound 1-C is a compound in which 1,1':3',1"-terphenyl, which is the linking group of the exemplary compound A4, has a substituent (methyl group) at a position other than the positions 4 and 3".

[Table 4]

| | Structure | Maximum bond length |
|---|---|---|
| Exemplary compound A4 | 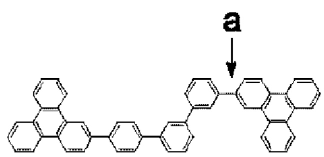 | 1.485 |
| Comparative compound 1-C | 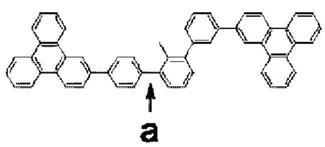 | 1.494 |

In Table 4, "a" denotes the bond with the maximum bond length in each compound. The maximum bond length is 1.485 angstroms in the exemplary compound A4 and 1.494 angstroms in the comparative compound 1-C. Such a substituent that increases the maximum bond length is undesirable. This is because a bond with a long bond length is likely to be cleaved and reduces operational durability. In other words, a short maximum bond length can enhance the operational durability.

The bond length was determined by molecular orbital calculation. The calculation method in the molecular orbital calculation method utilized a widely used density functional theory (DFT). B3LYP was used as the functional, and

6-31G* was used as the basis function. The molecular orbital calculation method was performed using widely used Gaussian 09 (Gaussian 09, Revision C. 01, M.J. Frisch, G.W. Trucks, H.B. Schlegel, G.E. Scuseria, M.A. Robb, J.R. Cheeseman, G. Scalmani, V. Barone, B. Mennucci, G.A. Petersson, H. Nakatsuji, M. Caricato, X. Li, H.P. Hratchian, A.F. Izmaylov, J. Bloino, G. Zheng, J.L. Sonnenberg, M. Hada, M. Ehara, K. Toyota, R. Fukuda, J. Hasegawa, M. Ishida, T. Nakajima, Y. Honda, O. Kitao, H. Nakai, T. Vreven, J.A. Montgomery, Jr., J.E. Peralta, F. Ogliaro, M. Bearpark, J.J. Heyd, E. Brothers, K.N. Kudin, V.N. Staroverov, T. Keith, R. Kobayashi, J. Normand, K. Raghavachari, A. Rendell, J.C. Burant, S.S. Iyengar, J. Tomasi, M. Cossi, N. Rega, J.M. Millam, M. Klene, J.E. Knox, J.B. Cross, V. Bakken, C. Adamo, J. Jaramillo, R. Gomperts, R.E. Stratmann, O. Yazyev, A.J. Austin, R. Cammi, C. Pomelli, J.W. Ochterski, R.L. Martin, K. Morokuma, V.G. Zakrzewski, G.A. Voth, P. Salvador, J.J. Dannenberg, S. Dapprich, A.D. Daniels, O. Farkas, J.B. Foresman, J.V. Ortiz, J. Cioslowski, and D.J. Fox, Gaussian, Inc., Wallingford CT, 2010.).

The evaluation of emission properties and sublimability of the organic compound according to the present embodiment in the characteristics (1) to (3) is described in more detail in the exemplary embodiments described later.

When further having the following characteristics, the organic compound according to the present embodiment can be particularly suitable for use in an organic light-emitting element. Two or more of the following characteristics may be simultaneously satisfied.

(4) The fused rings represented by $R_1$ and $R_2$ have no sp3 carbon

(5) Each of the fused rings represented by $R_1$ and $R_2$ is bonded to the linking group at a substitution position where steric hindrance has less interfering action

These characteristic are described below.

(4) The fused rings represented by $R_1$ and $R_2$ have no sp3 carbon

In the organic compound according to the present embodiment, the fused rings at both ends preferably have no sp3 carbon. When the fused rings represented by $R_1$ and $R_2$ have no sp3 carbon, due to no carbon-carbon bond with low binding energy, bond cleavage is less likely to occur during the operation of the organic light-emitting element. This can improve the element life characteristics of an organic light-emitting element.

More specifically, the fused rings represented by $R_1$ and $R_2$ are preferably each independently selected from triphenylene, phenanthrene, chrysene, dibenzofuran, dibenzothiophene, and azatriphenylene.

(5) Each of the fused rings represented by $R_1$ and $R_2$ is bonded to the linking group at a substitution position where steric hindrance has less interfering action

In the organic compound according to the present embodiment, the fused rings represented by $R_1$ and $R_2$ are preferably bonded to the linking group at a substitution position where steric hindrance has less interfering action. This is because the fused ring and the linking group bonded to each other at a substitution position where steric hindrance has less interference can have a shorter bond length therebetween. A shorter bond length can result in less cleavage and a stable bond. This can result in less bond cleavage during the operation of the organic light-emitting element and therefore result in the organic light-emitting element with improved element life characteristics.

Table 5 shows comparison results of the bond length between the exemplary compound A4 and the exemplary compound A3.

[Table 5]

| | Structure | Maximum bond length |
|---|---|---|
| Exemplary compound A4 | 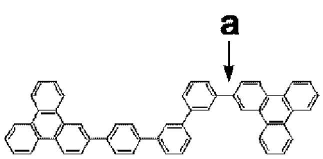 | 1.485 |
| Exemplary compound A3 | 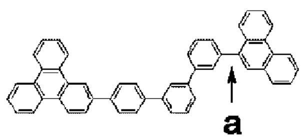 | 1.492 |

In Table 5, "a" denotes the bond with the maximum bond length in each compound. The maximum bond length is 1.485 angstroms in the exemplary compound A4 and 1.492 angstroms in the exemplary compound A3. This is because the steric hindrance of a hydrogen atom at a peri position of the phenanthrene ring increased the bond length between the phenanthrene ring and the linking group. The bond "a" with the maximum bond length is more stable in the exemplary compound A4 than in the exemplary compound A3. Thus, the exemplary compound A4, rather than the exemplary compound A3, can improve the element life characteristics of an organic light-emitting element.

Specific examples of the organic compound according to the present embodiment are described below. However, the present invention is not limited to these.

[Chem. 15]

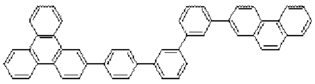

A1

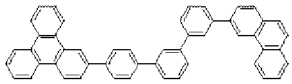

A2

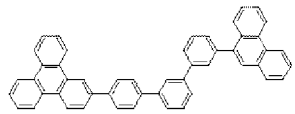

A3

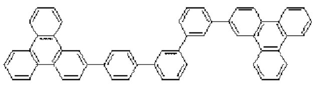

A4

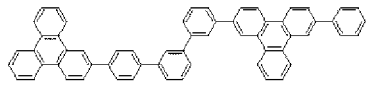

A5

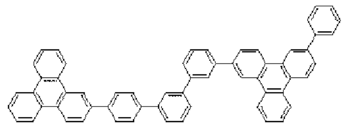

A6

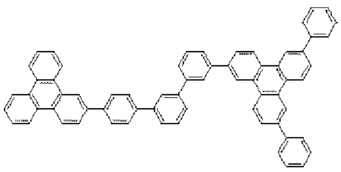

A7

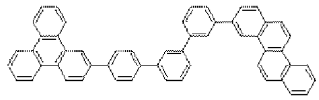

A8

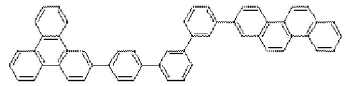

A9

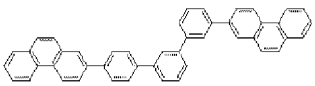

A10

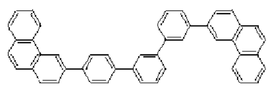

A11

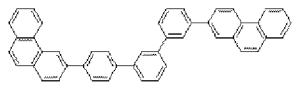

A12

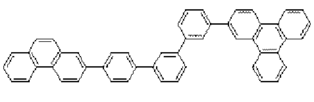

A13

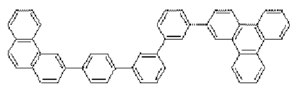

A14

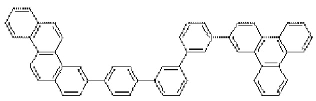

A15

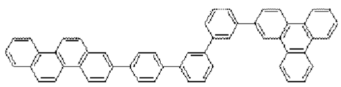

A16

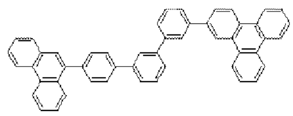

A17

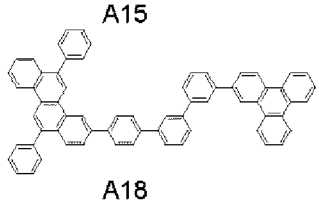

A18

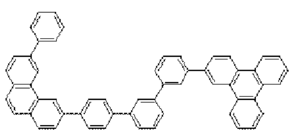

A19

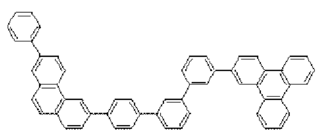

A20

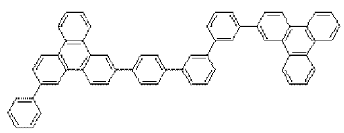

A21

[Chem. 16]

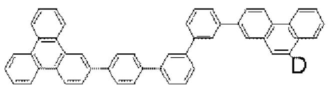

A22

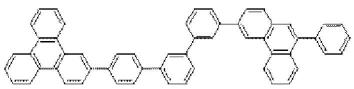

A23

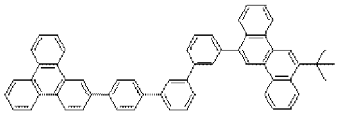

A24

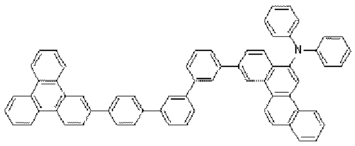

A25

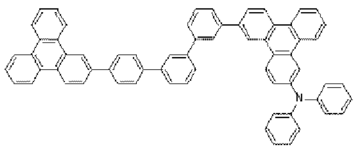

A26

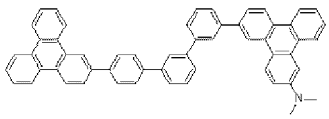

A27

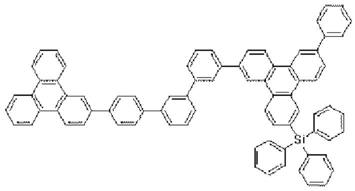

A28

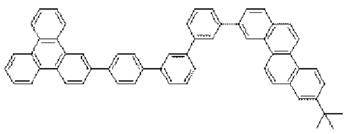

A29

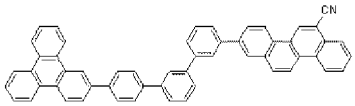

A30

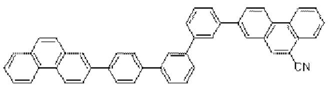

A31

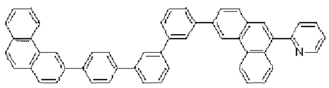

A32

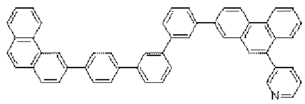

A33

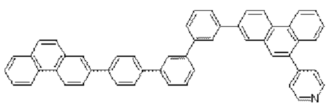

A34

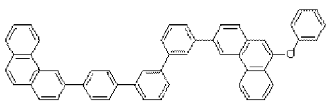

A35

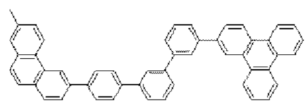

A36

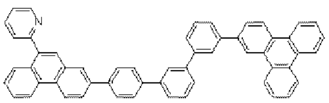

A37

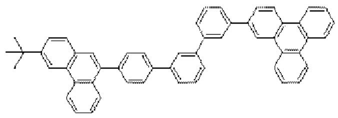

A38

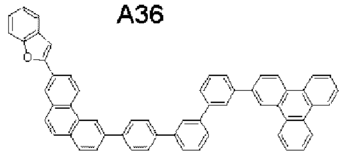

A39

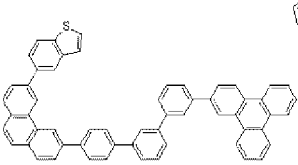

A40

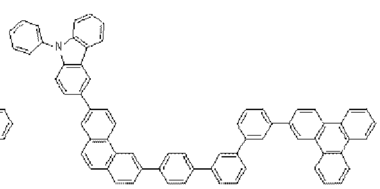

A41

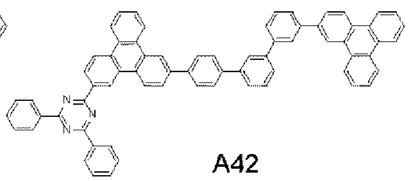

A42

[Chem. 17]

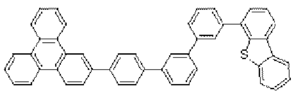 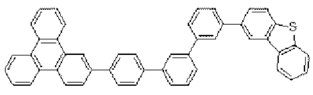 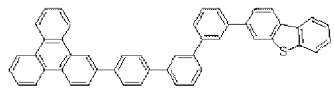

B1 B2 B3

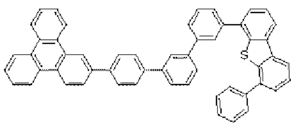 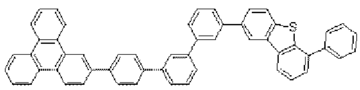 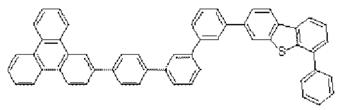

B4 B5 B6

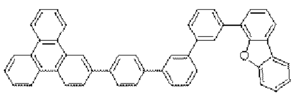 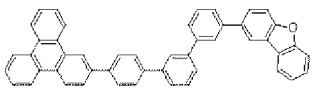 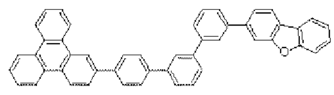

B7 B8 B9

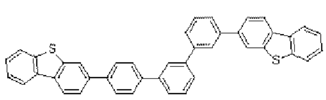 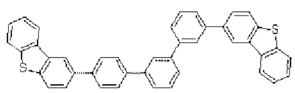 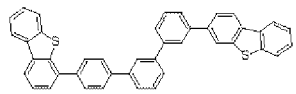

B10 B11 B12

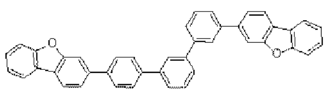 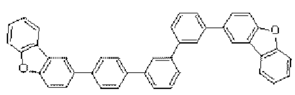 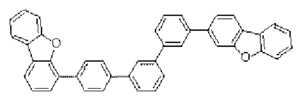

B13 B14 B15

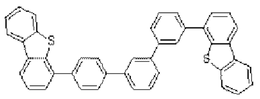 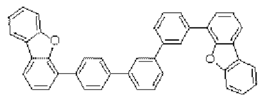 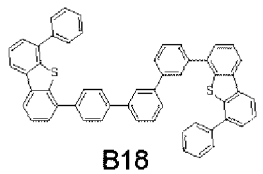

B16 B17 B18

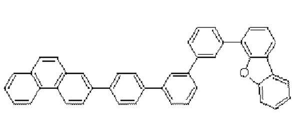 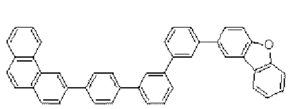 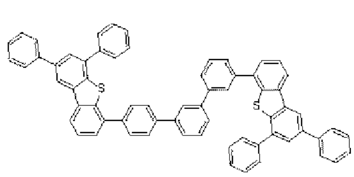

B19 B20 B21

[Chem. 18]

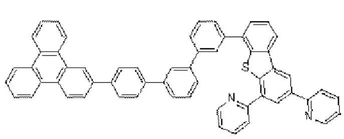
B22

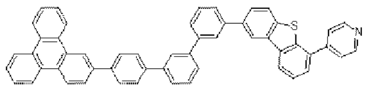
B23

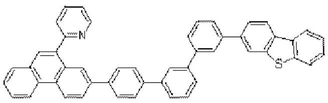
B24

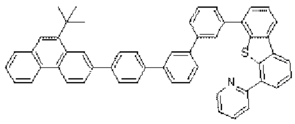
B25

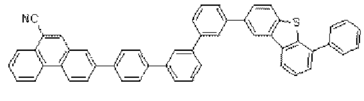
B26

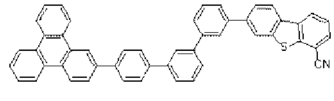
B27

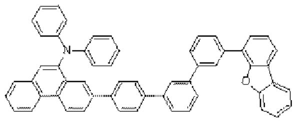
B28

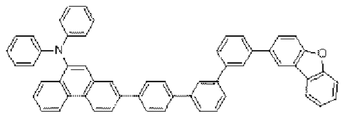
B29

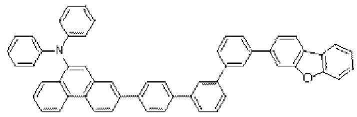
B30

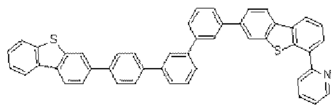
B31

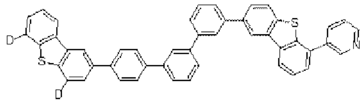
B32

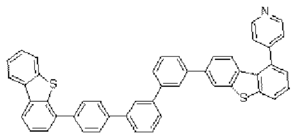
B33

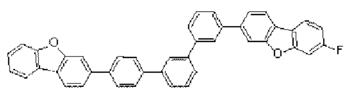
B34

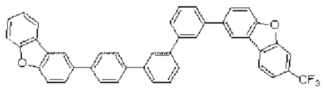
B35

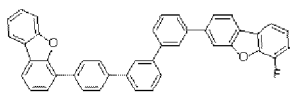
B36

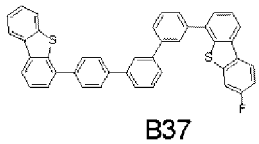
B37

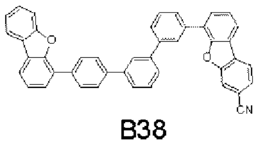
B38

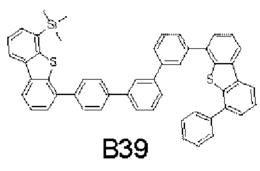
B39

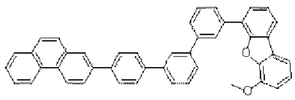
B40

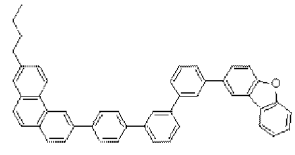
B41

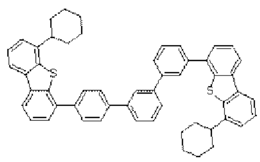
B42

[Chem. 19]

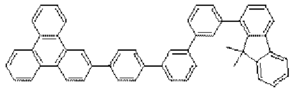

C1

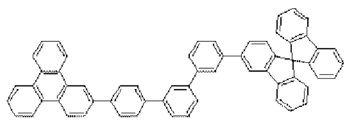

C2

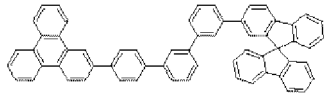

C3

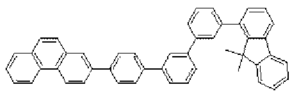

C4

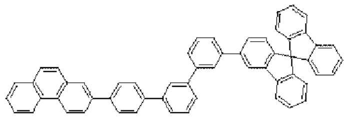

C5

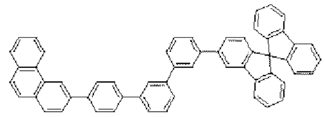

C6

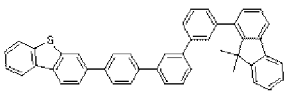

C7

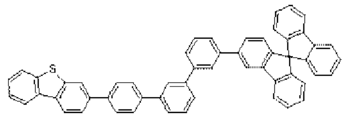

C8

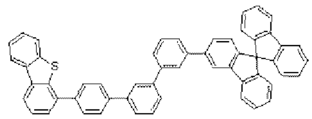

C9

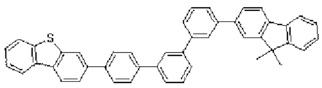

C10

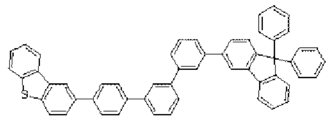

C11

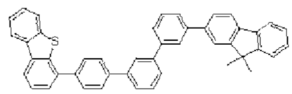

C12

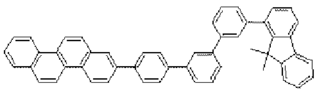

C13

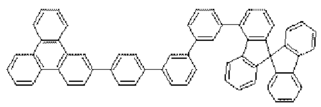

C14

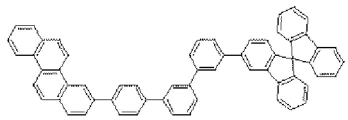

C15

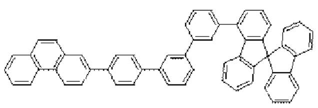

C16

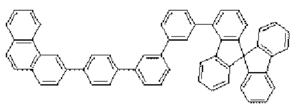

C17

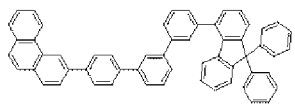

C18

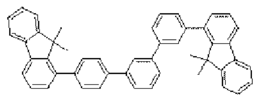

C19

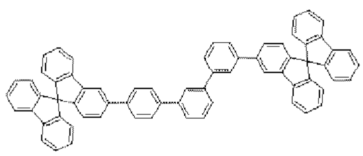

C20

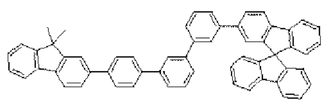

C21

[Chem. 20]

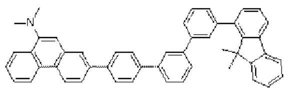

C22

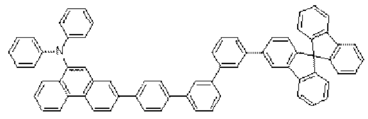

C23

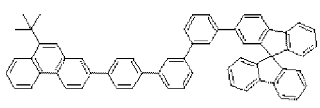

C24

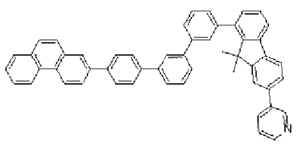

C25

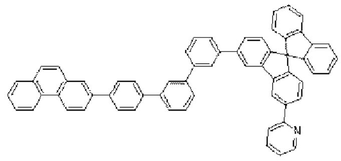

C26

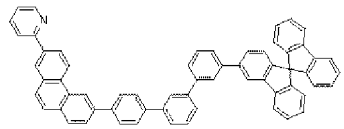

C27

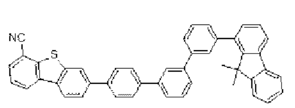

C28

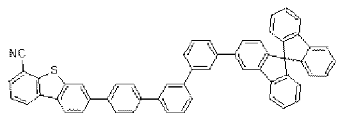

C29

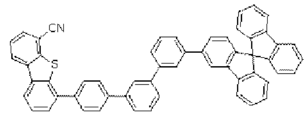

C30

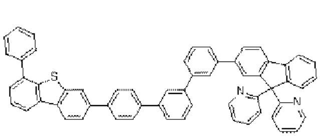

C31

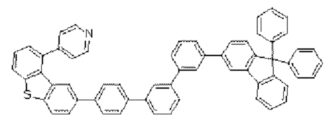

C32

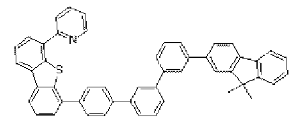

C33

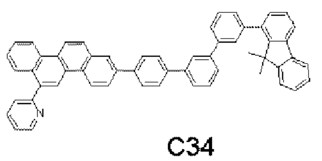

C34

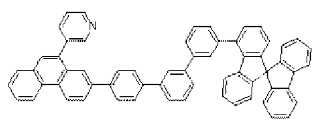

C35

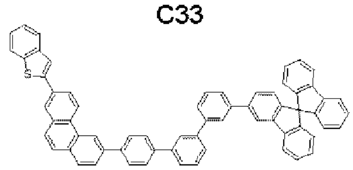

C36

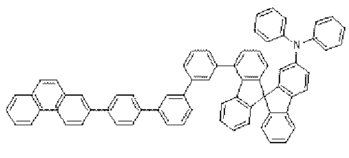

C37

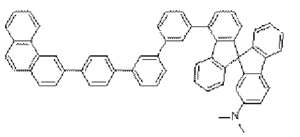

C38

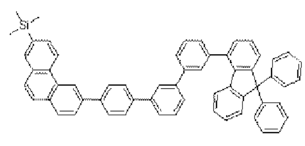

C39

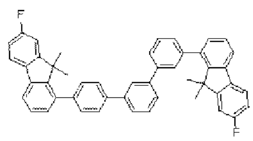

C40

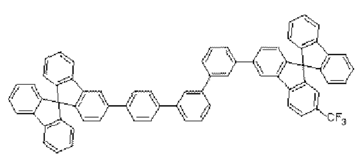

C41

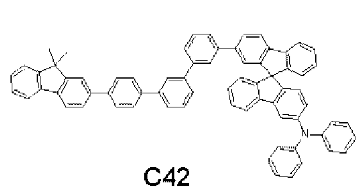

C42

[Chem. 21]

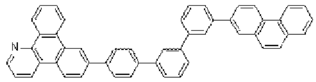

D1

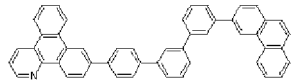

D2

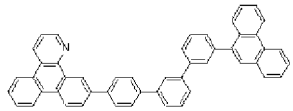

D3

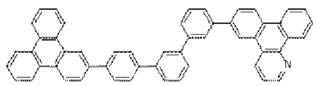

D4

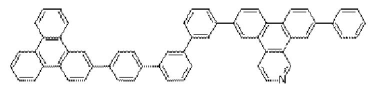

D5

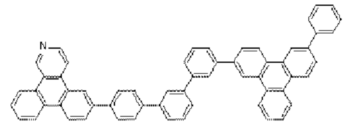

D6

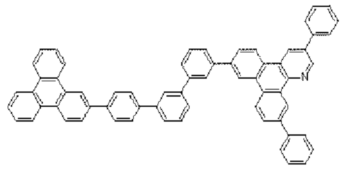

DA7

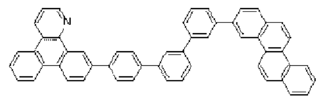

D8

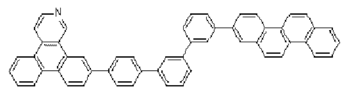

D9

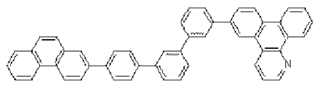

D10

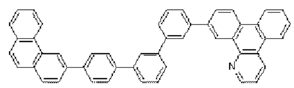

D11

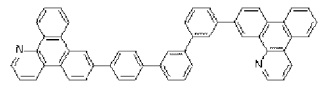

D12

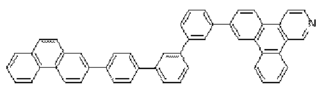

D13

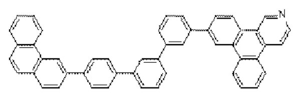

D14

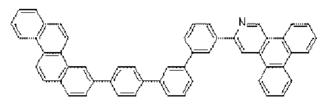

D15

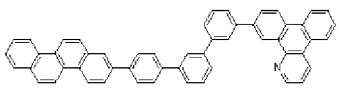

D16

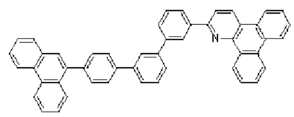

D17

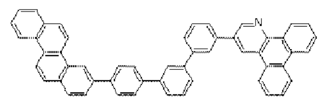

D18

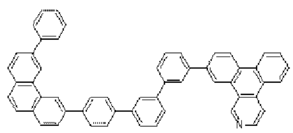

D19

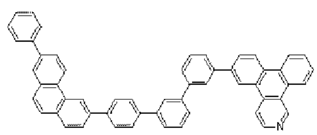

D20

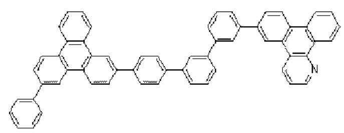

D21

[Chem. 22]

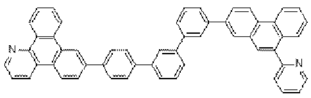 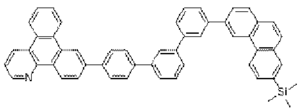 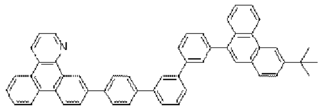

D22 D23 D24

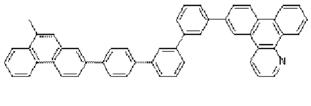 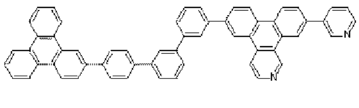 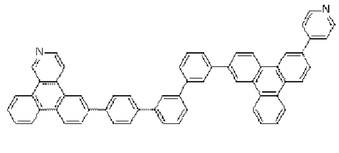

D25 D26 D27

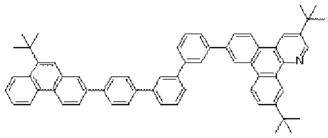 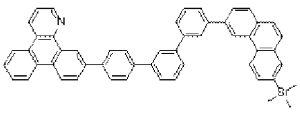 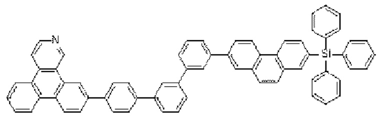

D28 D29 D30

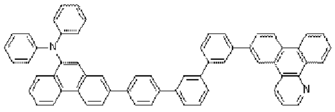 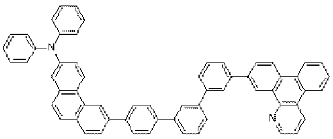 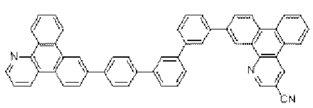

D31 D32 D33

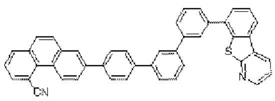 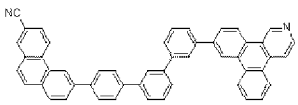 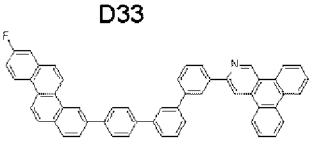

D34 D35 D36

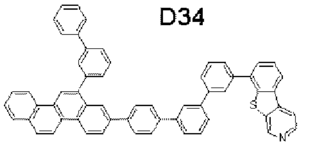 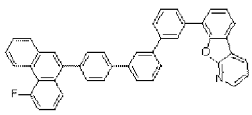 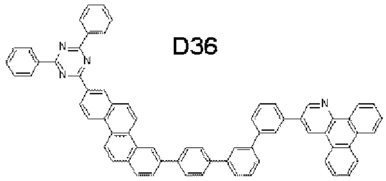

D37 D38 D39

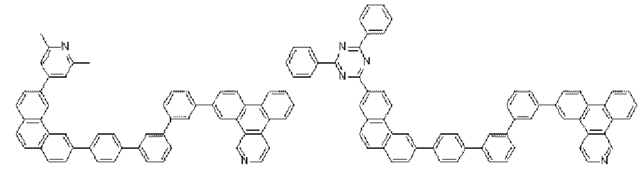 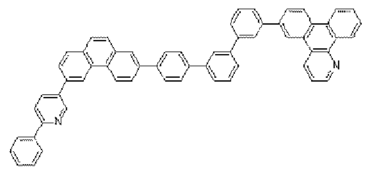

D40 D41 D42

[Chem. 23]

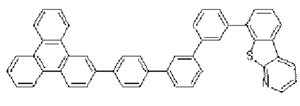

D43

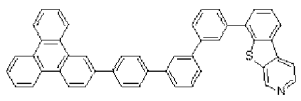

D44

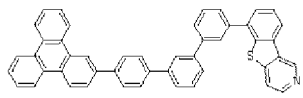

D45

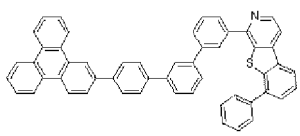

D46

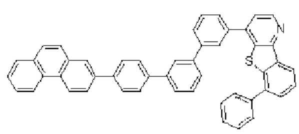

D47

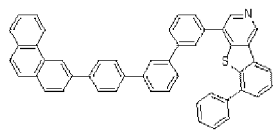

D48

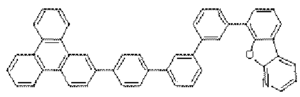

D49

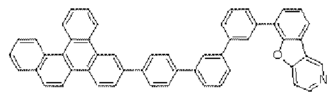

D50

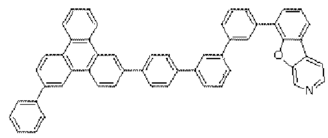

D51

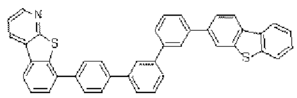

D52

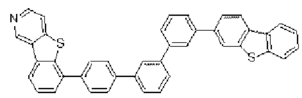

D53

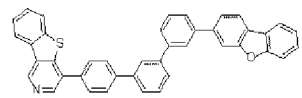

D54

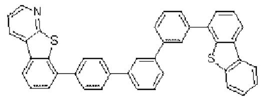

D55

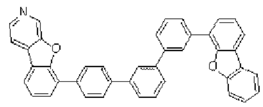

D56

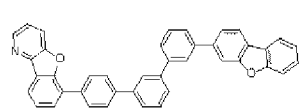

D57

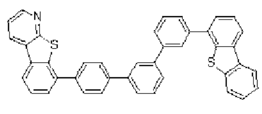

D58

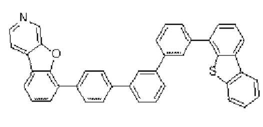

D59

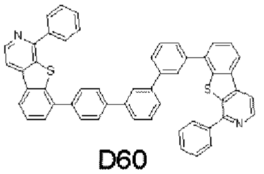

D60

Among these exemplary compounds, the exemplary compounds belonging to Group A (A1 to A42) are compounds in which the skeleton of the fused rings represented by $R_1$ and $R_2$ is an aromatic hydrocarbon and is composed of sp2 carbons.

These compounds are particularly stable among the compounds represented by the general formula [1] because the skeleton of the fused rings is composed of an sp2 hybrid orbital. In particular, the fused rings represented by $R_1$ and $R_2$ are preferably a triphenylene ring or a phenanthrene ring due to high $T_1$. From the perspective of binding stability, it is more preferable that at least one of the fused rings represented by $R_1$ and $R_2$ is a triphenylene ring and is bonded to the linking group at position 2 of the triphenylene ring. Alternatively, from the perspective of binding stability, it is more preferable that at least one of the fused rings represented by $R_1$ and $R_2$ is a phenanthrene ring and is bonded to the linking group at position 2 or 3 of the phenanthrene ring.

Among the exemplary compounds, the exemplary compounds belonging to Group B (B1 to B42) are compounds with a dibenzofuran ring or a dibenzothiophene ring in the fused rings represented by $R_1$ and $R_2$.

These compounds contain an oxygen atom or a sulfur atom in the fused rings represented by $R_1$ and $R_2$.

Abundant lone pairs in these atoms can enhance charge transport ability and make it particularly easy to adjust the carrier balance of the compounds. From the perspective of binding stability, at least one of the fused rings represented by $R_1$ and $R_2$ is preferably bonded to the linking group at position 2, 3, or 4 of the dibenzofuran ring or the dibenzothiophene ring.

Among the exemplary compounds, the exemplary compounds belonging to Group C (C1 to C42) are compounds with a fluorene ring in the fused rings represented by $R_1$ and $R_2$.

These compounds further have a substituent at position 9 of fluorene. Thus, the substituent in the direction perpendicular to the in-plane direction of the fluorene ring can particularly suppress overlapping of fused rings. Thus, the compounds have particularly high sublimability.

Among the exemplary compounds, the exemplary compounds belonging to Group D (D1 to D60) are compounds with an azine ring in the fused rings represented by $R_1$ and $R_2$.

These compounds contain a N atom in a fused ring, and lone pairs and high electronegativity of the N atom can enhance charge transport ability and make it particularly easy to adjust the carrier balance of the compounds.

<<Organic Light-Emitting Element>>

Next, an organic light-emitting element according to the present embodiment is described.

A specific element structure of the organic light-emitting element according to the present embodiment may be a multilayer element structure including an electrode layer and an organic compound layer shown in the following (a) to (f) sequentially stacked on a substrate. More specifically, the organic light-emitting element according to the present embodiment includes at least a pair of electrodes, a positive electrode and a negative electrode, and an organic compound layer between the electrodes. In any of the element structures, the organic compound layer always includes a light-emitting layer containing a light-emitting material.

(a) positive electrode/light-emitting layer/negative electrode
(b) positive electrode/hole transport layer/light-emitting layer/electron transport layer/negative electrode
(c) positive electrode/hole transport layer/light-emitting layer/electron transport layer/electron injection layer/negative electrode
(d) positive electrode/hole injection layer/hole transport layer/light-emitting layer/electron transport layer/negative electrode
(e) positive electrode/hole injection layer/hole transport layer/light-emitting layer/electron transport layer/electron injection layer/negative electrode
(f) positive electrode/hole transport layer/electron-blocking layer/light-emitting layer/hole-blocking layer/electron transport layer/negative electrode

These element structure examples are only very basic element structures, and the element structure of an organic light-emitting element according to the present invention is not limited to these element structures. For example, an insulating layer, an adhesive layer, or an interference layer may be provided at an interface between an electrode and an organic compound layer. An electron transport layer or a hole transport layer may have a multilayered structure having two layers with different ionization potentials. A light-emitting layer may have a multilayered structure having two layers each containing a different light-emitting material. Thus, a first light-emitting layer for emitting first light and a second light-emitting layer for emitting second light may be provided between a positive electrode and a negative electrode. An organic light-emitting element for emitting white light can be produced in which the white light is composed of first light and second light of different colors. In addition to such structures, various other layer structures can be employed.

In the present embodiment, the mode (element form) of extracting light from a light-emitting layer may be a bottom emission mode of extracting light from an electrode on the substrate side or a top emission mode of extracting light from the side opposite to the substrate side. The mode may also be a double-sided extraction mode of extracting light from the substrate side and from the side opposite to the substrate side.

Among the element structures shown in (a) to (f), the structure (f) is preferred due to the presence of both an electron-blocking layer (electron-stopping layer) and a hole-blocking layer (hole-stopping layer). Thus, the electron-blocking layer and the hole-blocking layer in (f) can securely confine carriers of both holes and electrons in the light-emitting layer. Thus, the organic light-emitting element has no carrier leakage and high light emission efficiency.

The organic light-emitting element according to the present embodiment contains an organic compound represented by the general formula [1] in an organic compound layer. The organic light-emitting element according to the present embodiment preferably contains an organic compound represented by the general formula [1] in the light-emitting layer. However, the present invention is not limited thereto, and it can be used as a constituent material of an organic compound layer other than the light-emitting layer constituting the organic light-emitting element according to the present

embodiment. More specifically, it may be used as a constituent material of an electron transport layer, an electron injection layer, an electron-blocking layer, a hole transport layer, a hole injection layer, a hole-blocking layer, or the like.

In the organic light-emitting element according to the present embodiment, when the light-emitting layer contains an organic compound represented by the general formula [1], the light-emitting layer may be a layer composed of the organic compound represented by the general formula [1] and a second compound, which is another compound. For a light-emitting layer composed of an organic compound represented by the general formula [1] and another compound, the organic compound according to the present embodiment may be used as a host (also referred to as a host material) or an assist material of the light-emitting layer.

The host is a compound with the highest mass ratio among the compounds constituting the light-emitting layer. A guest is a compound that has a lower mass ratio than the host among the compounds constituting the light-emitting layer and that is a principal light-emitting compound. The assist material is a compound that has a lower mass ratio than the host among the compounds constituting the light-emitting layer and that assists the guest in emitting light. The assist material is also referred to as a second host. When the host is a first compound and the guest is a second compound, the assist can be referred to as a third compound.

The host is preferably a material with a higher LUMO than the guest (a material with a LUMO closer to the vacuum level). This allows electrons supplied to the host of the light-emitting layer to be efficiently delivered to the guest and improves light emission efficiency. Furthermore, when an assist material is used in addition to the host and the guest, the host is preferably a material with a higher LUMO than the assist material (a material with a LUMO closer to the vacuum level). This allows electrons supplied to the host of the light-emitting layer to be efficiently delivered to the assist material, and the assist material can play a role in exciton recombination. This enables efficient energy transfer to the guest.

The energy (singlet energy) of the excited singlet state ($S_1$) of the host is denoted by $S_{h1}$, the energy (triplet energy) of the excited triplet state ($T_1$) is denoted by $T_{h1}$, the energy of $S_1$ of the guest is denoted by $S_{g1}$, and the energy of $T_1$ of the guest is denoted by $T_{g1}$. Then, $S_{h1} > S_{g1}$ is preferably satisfied. $T_{h1} > T_{g1}$ is also preferably satisfied. Furthermore, the energy $S_{a1}$ of $S_1$ and the energy $T_{a1}$ of $T_1$ of the assist material preferably satisfies $S_{a1} > S_{g1}$, more preferably $T_{a1} > T_{g1}$. Furthermore, $S_{h1} > S_{a1} > S_{g1}$ is still more preferably satisfied, and $T_{h1} > T_{a1} > T_{g1}$ is still more preferably satisfied.

The present inventors conducted various studies and found that an organic light-emitting element with high light emission efficiency and durability can be produced when an organic compound represented by the general formula [1] is used as a host or an assist in a light-emitting layer, particularly as an assist in the light-emitting layer.

The organic compound according to the present embodiment is more preferably used in a light-emitting layer in an organic light-emitting element under the following conditions. Two or more of the following conditions may be simultaneously satisfied.

(6) The light-emitting layer contains an organic compound represented by the general formula [1] at a concentration of 30% by mass or more and 95% by mass or less of the entire light-emitting layer.

(7) The light-emitting layer contains an organic compound represented by the general formula [1] and a phosphorescent material with a tricyclic or higher polycyclic fused ring in a ligand thereof.

Each of the conditions is described below.

(6) The light-emitting layer contains an organic compound represented by the general formula [1] at a concentration of 30% by mass or more and 99% by mass or less of the entire light-emitting layer.

When the organic compound according to the present embodiment is used in the light-emitting layer, the concentration of the organic compound according to the present embodiment is preferably 30% by mass or more and 99% by mass or less of the entire light-emitting layer. Due to its high amorphousness, the organic compound according to the present embodiment is a material suitable for a host material for a light-emitting layer. The concentration of the organic compound according to the present embodiment is preferably 50% by mass or more and 99% by mass or less, more preferably 70% by mass or more and 99% by mass or less, of the entire light-emitting layer. The organic compound according to the present embodiment is a compound with high amorphousness and difficult to crystallize and is therefore has a function with good element life characteristics even when the concentration is 99% by mass of the entire light-emitting layer.

From the perspective of improving the film properties of a light-emitting layer, the organic compound according to the present embodiment may also be used as an assist material. When used as an assist material, the organic compound according to the present embodiment can be used at a concentration of 30% by mass or more and 50% by mass or less of the entire light-emitting layer.

(7) The light-emitting layer contains an organic compound represented by the general formula [1] and a phosphorescent material with a tricyclic or higher polycyclic fused ring in a ligand thereof.

The organic compound according to the present embodiment is a compound with a tricyclic or higher polycyclic

fused ring at both ends. Thus, a phosphorescent material (guest material) used with the organic compound according to the present embodiment in a light-emitting layer preferably has a structure including a ligand with extended n-conjugation. More specifically, the phosphorescent material used with the organic compound according to the present embodiment preferably has a tricyclic or higher polycyclic fused ring in the structure of a ligand thereof.

This is because, when the guest material has a structure with high flatness as in the host material, moieties with high flatness in the guest material and the host material can approach each other through interaction. More specifically, a flat moiety of the host material easily approaches a ligand of an organometallic complex (the guest material). This can be expected to decrease the intermolecular distance between the host material and the guest material.

It is known that triplet energy utilized in a phosphorescent element is transferred by the Dexter mechanism. In the energy transfer by the Dexter mechanism, energy is transferred by contact between molecules. More specifically, the intermolecular distance between a host material and a guest material is shortened for efficient energy transfer from the host material to the guest material.

As described above, the use of an organometallic complex with high flatness and with a ligand structure including a tricyclic or higher polycyclic fused ring as a guest material decreases the intermolecular distance between the guest material and a host material, which is an organic compound represented by the general formula [1]. This is likely to cause energy transfer from the host material to the guest material by the Dexter mechanism. Consequently, an organic light-emitting element with high light emission efficiency can be provided.

A tricyclic or higher polycyclic fused ring structure with high flatness in a ligand is preferably a triphenylene structure, a phenanthrene structure, a fluorene structure, a benzofluorene structure, a dibenzofuran structure, or a dibenzothiophene structure. Using an organometallic complex with at least one of these structures in a ligand also as a light-emitting material, the organic compound according to the present embodiment can provide a light-emitting element with higher efficiency.

Specific examples of the organometallic complex according to the present embodiment are described below. However, the present invention is not limited to these. In the following structural formulae, in the case where two bonds between a bidentate ligand and an Ir atom are represented by a solid line instead of an arrow, one of the bonds may be a covalent bond, and the other bond may be a coordinate bond.

[Chem. 24]

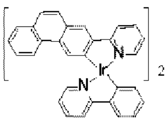
AA1
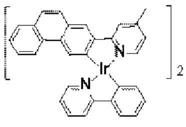
AA2
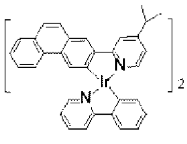
AA3
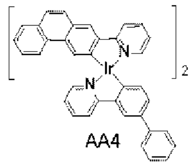
AA4
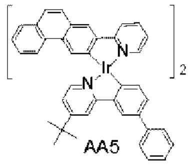
AA5

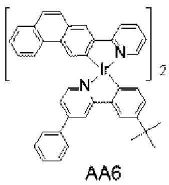
AA6
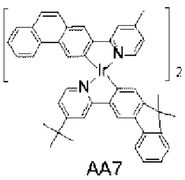
AA7
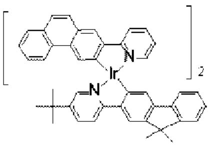
AA8
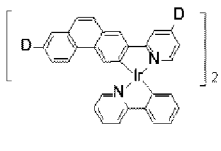
AA9
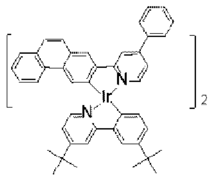
AA10

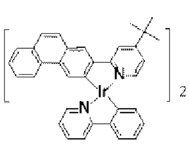
AA11
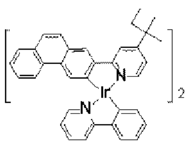
A12
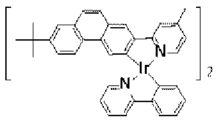
AA13
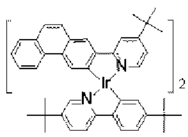
AA14
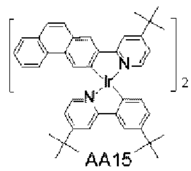
AA15

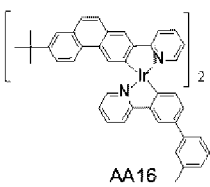
AA16
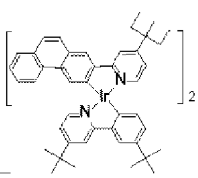
AA17
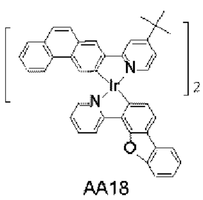
AA18
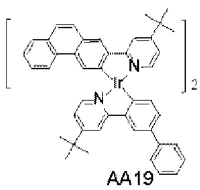
AA19
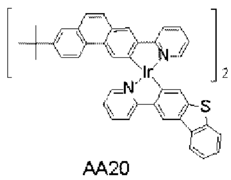
AA20

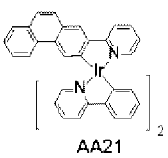
AA21
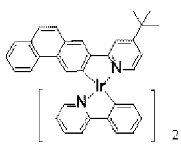
AA22
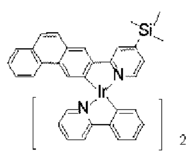
AA23
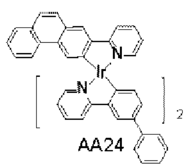
AA24
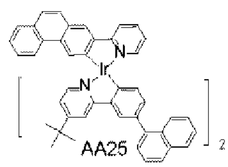
AA25

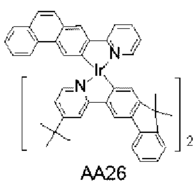
AA26
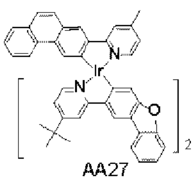
AA27
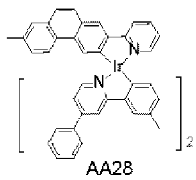
AA28
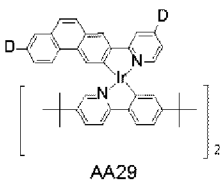
AA29
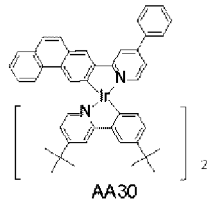
AA30

[Chem. 25]

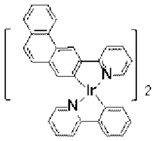
BB1

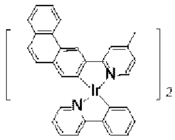
BB2

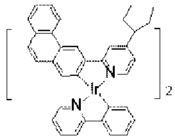
BB3

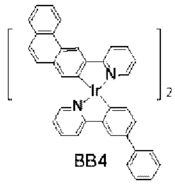
BB4

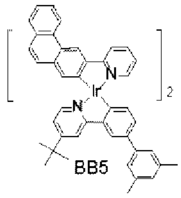
BB5

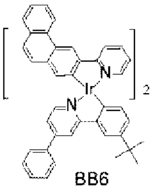
BB6

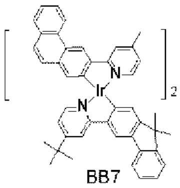
BB7

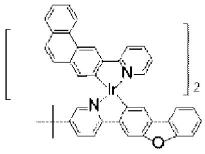
BB8

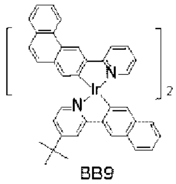
BB9

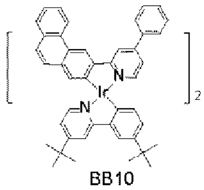
BB10

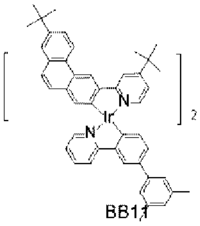
BB11

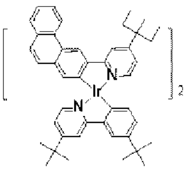
BB12

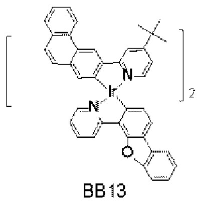
BB13

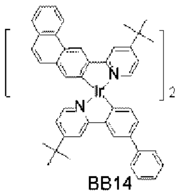
BB14

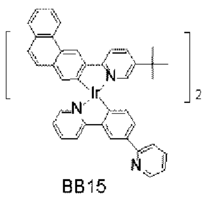
BB15

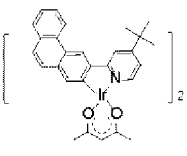
BB16

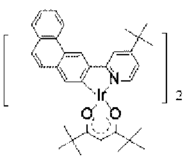
BB17

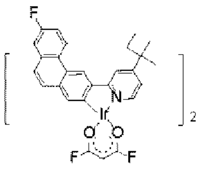
BB18

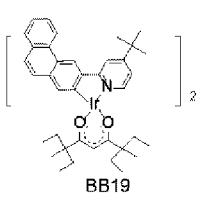
BB19

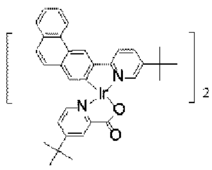
BB20

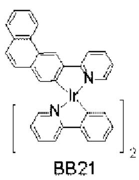
BB21

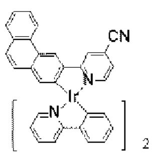
BB22

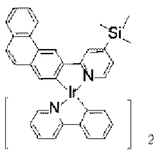
BB23

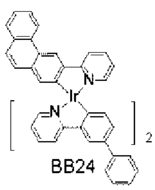
BB24

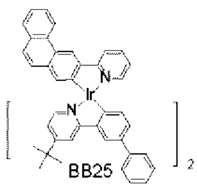
BB25

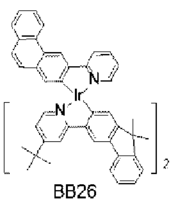
BB26

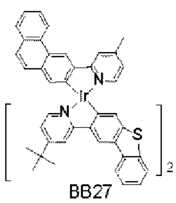
BB27

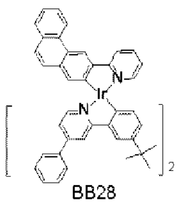
BB28

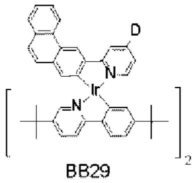
BB29

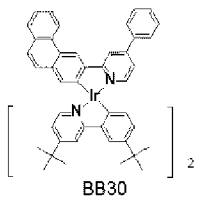
BB30

[Chem. 26]

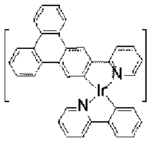
CC1
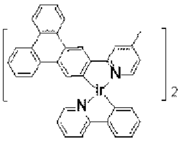
CC2
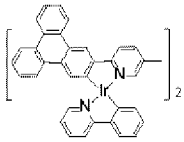
CC3
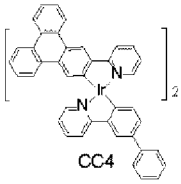
CC4
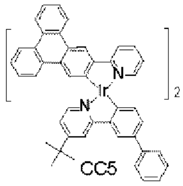
CC5

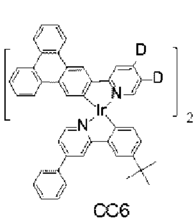
CC6
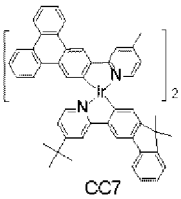
CC7
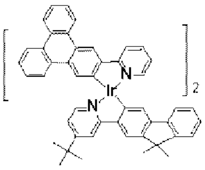
CC8
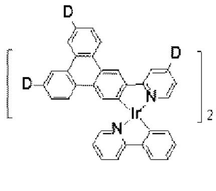
CC9
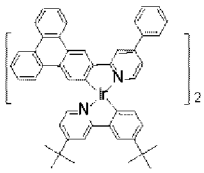
CC10

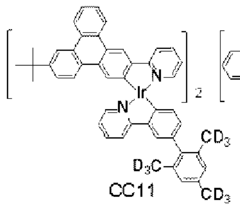
CC11
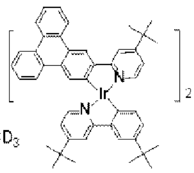
CC12
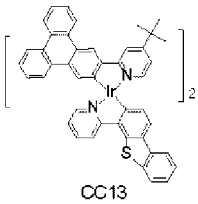
CC13
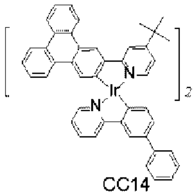
CC14
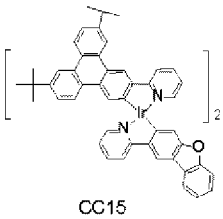
CC15

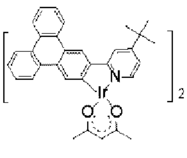
CC16
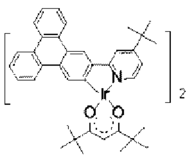
CC17
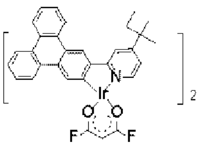
CC18
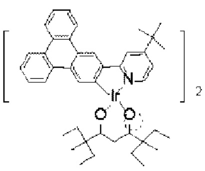
CC19
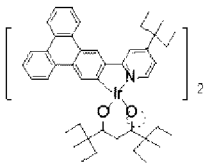
CC20

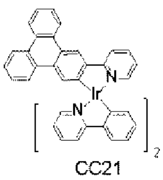
CC21
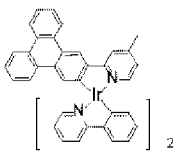
CC22
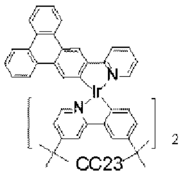
CC23
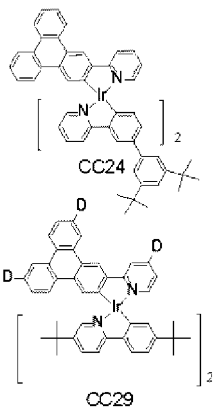
CC24
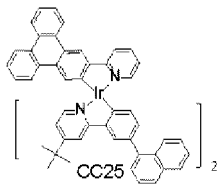
CC25

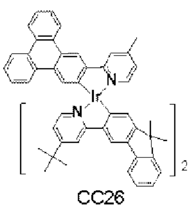
CC26
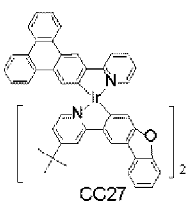
CC27
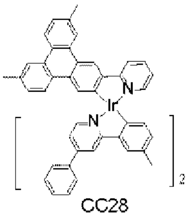
CC28
CC29
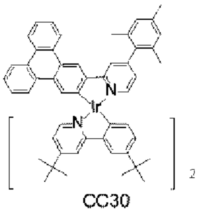
CC30

[Chem. 27]

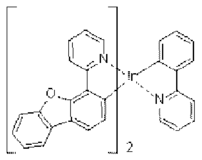
DD1

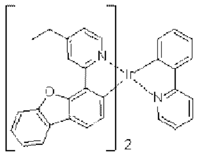
DD2

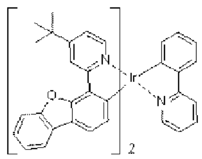
DD3

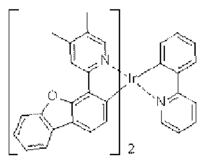
DD4

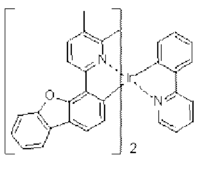
DD5

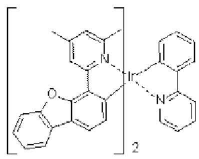
DD6

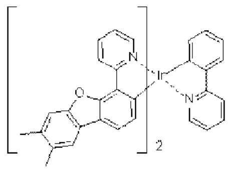
DD7

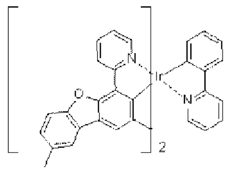
DD8

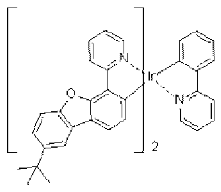
DD9

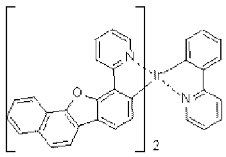
DD10

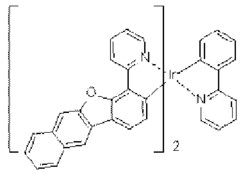
DD11

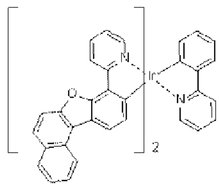
DD12

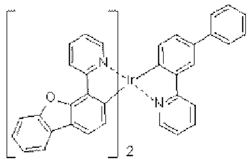
DD13

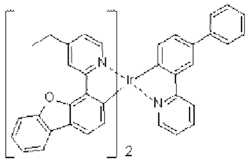
DD14

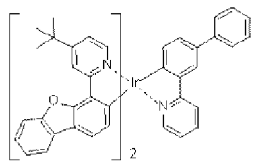
DD15

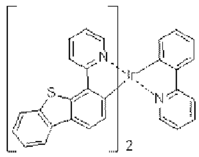
DD16

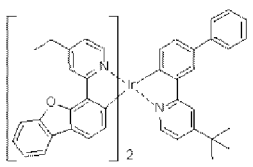
DD17

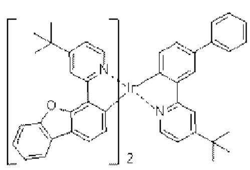
DD18

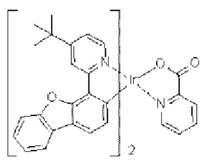
DD19

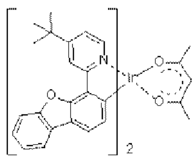
DD20

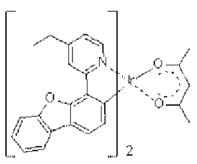
DD21

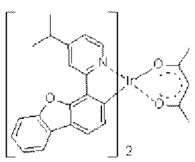
DD22

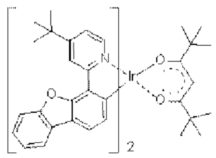
DD23

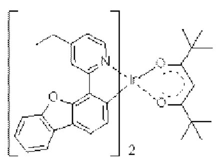
DD24

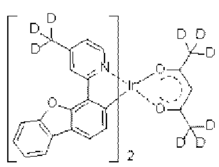
DD25

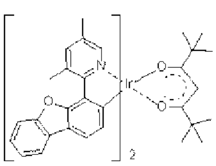
DD26

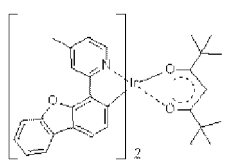
DD27

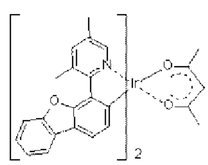
DD28

[Chem. 28]

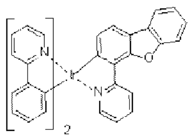
DD29

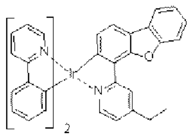
DD30

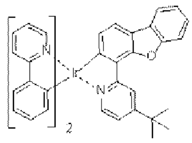
DD31

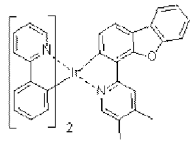
DD32

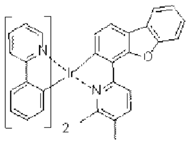
DD33

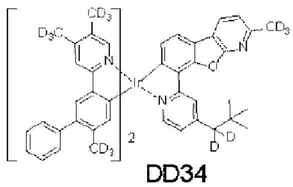
DD34

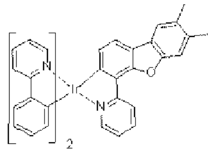
DD35

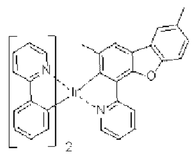
DD36

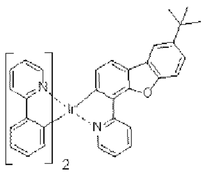
DD37

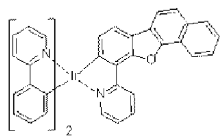
DD38

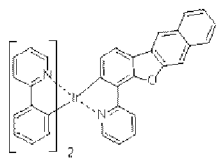
DD39

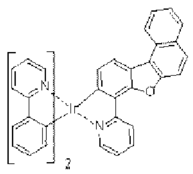
DD40

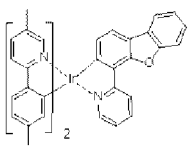
DD41

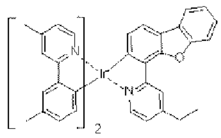
DD42

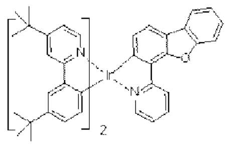
DD43

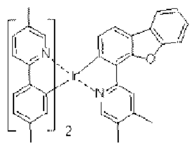
DD44

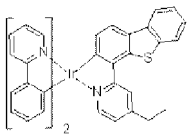
DD45

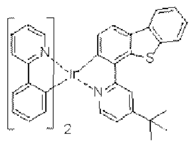
DD46

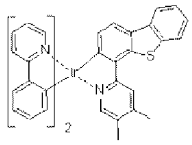
DD47

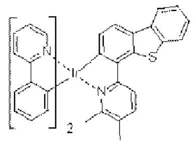
DD48

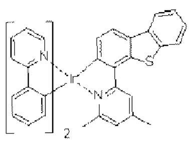
DD49

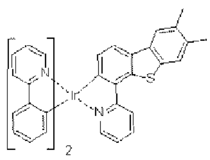
DD50

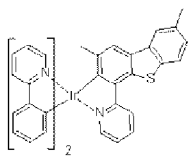
DD51

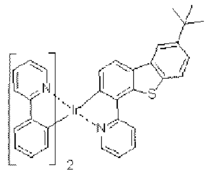
DD52

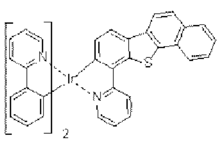
DD53

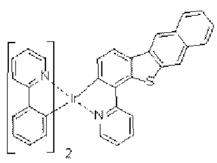
DD54

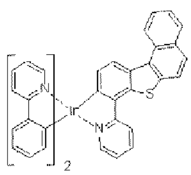
DD55

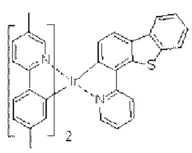
DD56

[Chem. 29]

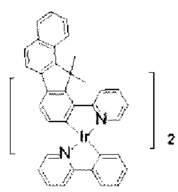
EE1

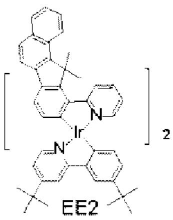
EE2

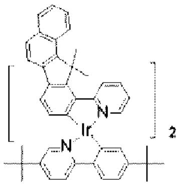
EE3

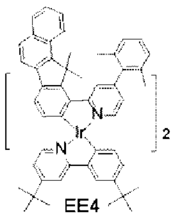
EE4

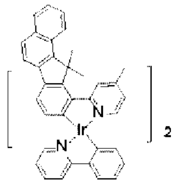
EE5

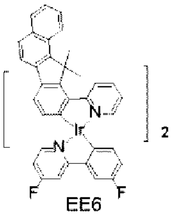
EE6

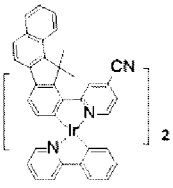
EE7

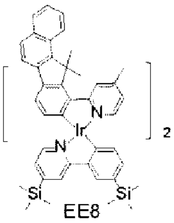
EE8

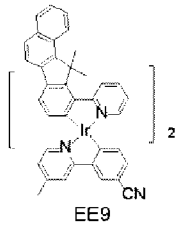
EE9

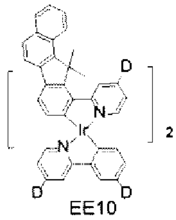
EE10

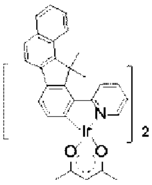
EE11

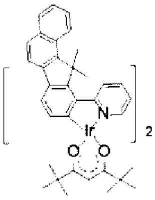
EE12

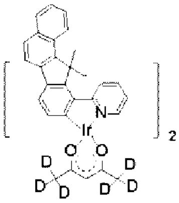
EE13

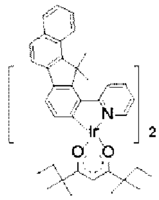
EE14

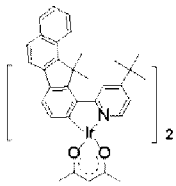
EE15

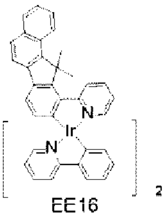
EE16

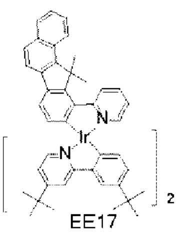
EE17

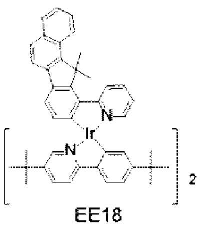
EE18

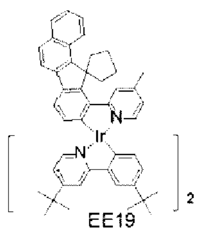
EE19

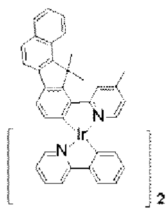
EE20

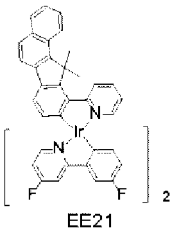
EE21

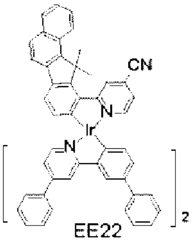
EE22

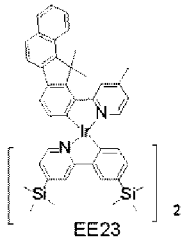
EE23

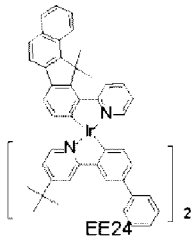
EE24

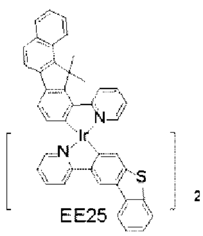
EE25

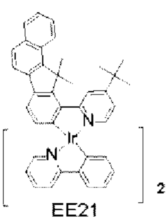
EE21

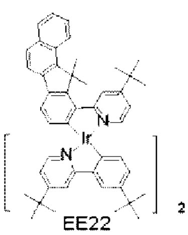
EE22

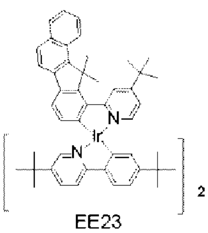
EE23

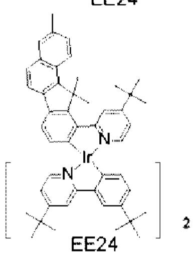
EE24

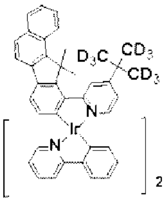
EE25

[Chem. 30]

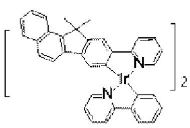
FF1
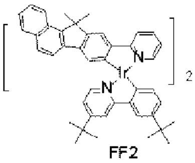
FF2
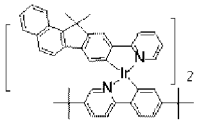
FF3
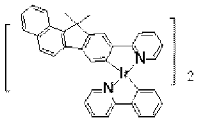
FF4
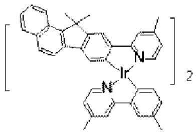
FF5
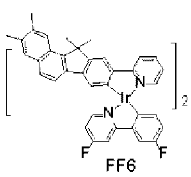
FF6
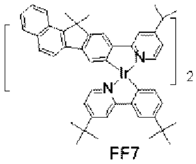
FF7
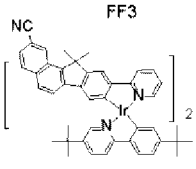
FF8
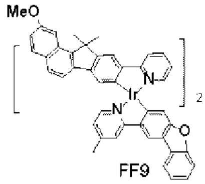
FF9
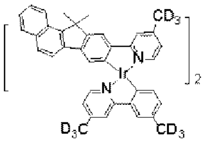
FF10
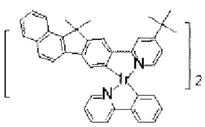
FF11
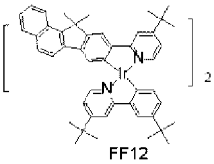
FF12
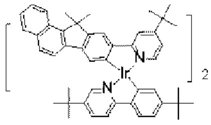
FF13
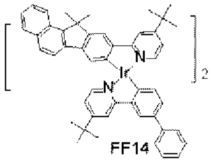
FF14
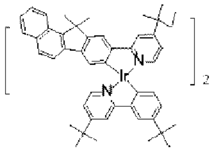
FF15
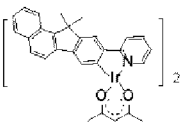
FF16
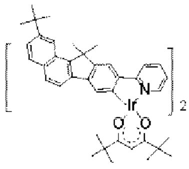
FF17
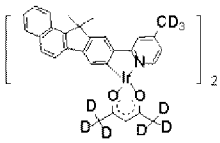
FF18
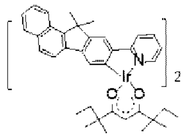
FF19
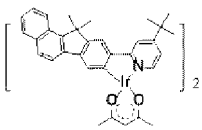
FF20
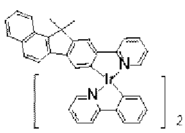
FF21
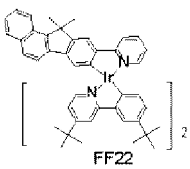
FF22
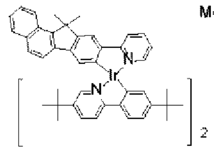
FF23
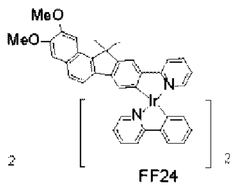
FF24
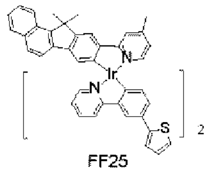
FF25
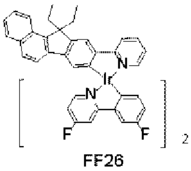
FF26
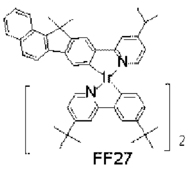
FF27
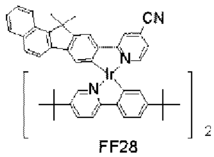
FF28
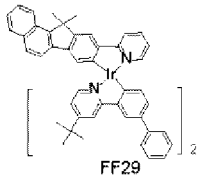
FF29
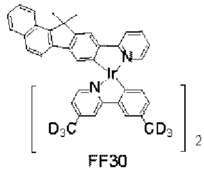
FF30
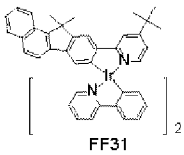
FF31
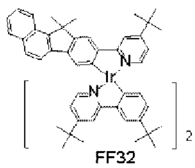
FF32
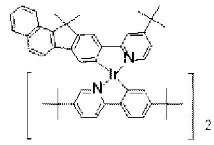
FF33
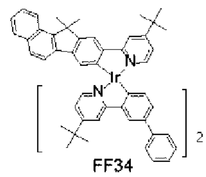
FF34
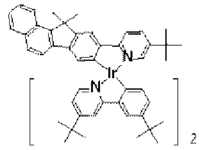
FF35

[Chem. 31]

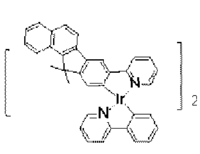
GG1
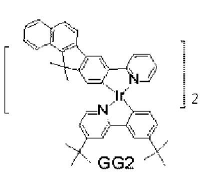
GG2
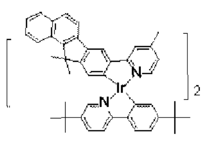
GG3
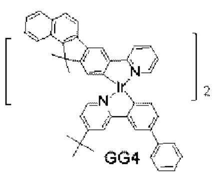
GG4
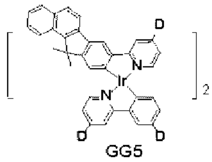
GG5

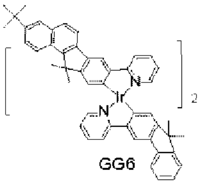
GG6
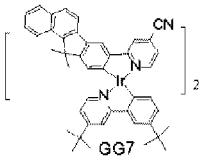
GG7
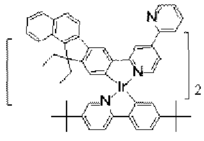
GG8
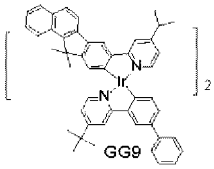
GG9
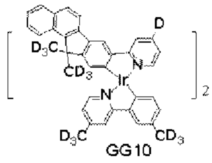
GG10

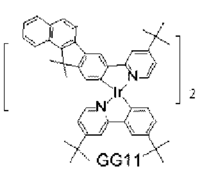
GG11
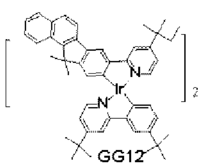
GG12
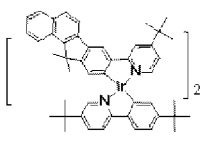
GG13
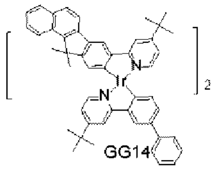
GG14
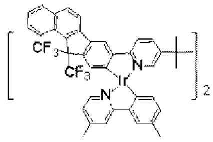
GG15

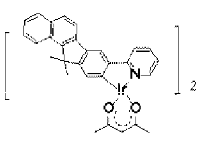
GG16
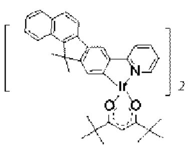
GG17
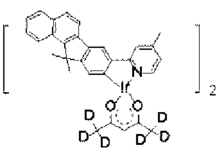
GG18
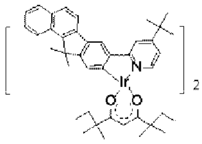
GG19
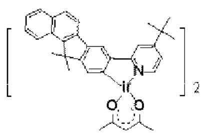
GG20

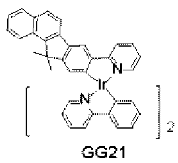
GG21
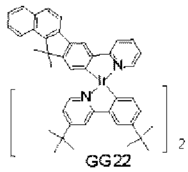
GG22
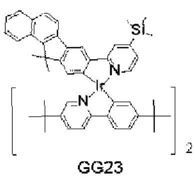
GG23
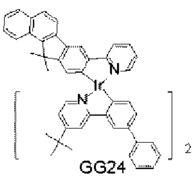
GG24
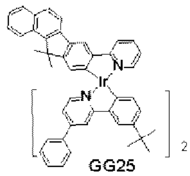
GG25

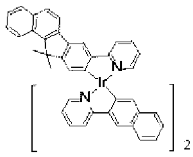
GG26
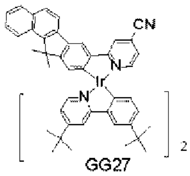
GG27
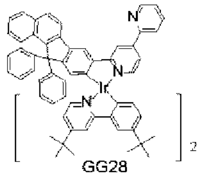
GG28
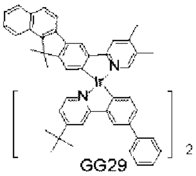
GG29
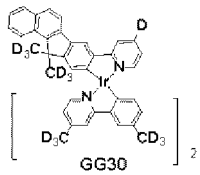
GG30

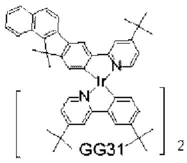
GG31
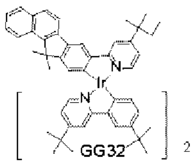
GG32
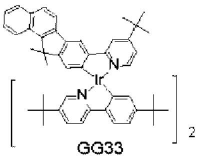
GG33
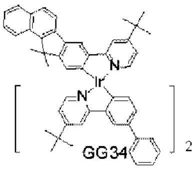
GG34
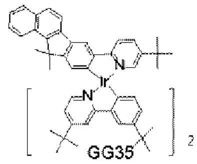
GG35

[Chem. 32]

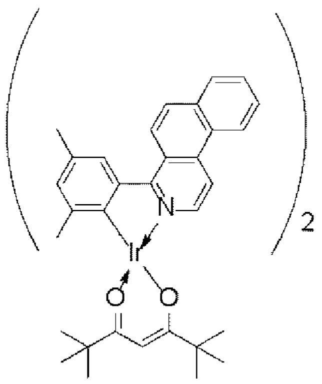

HH1

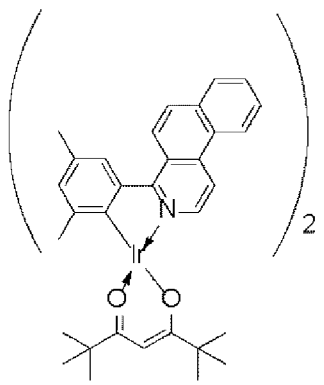

HH2

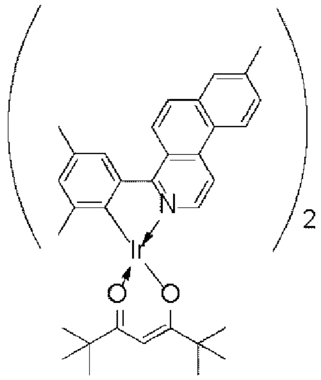

HH3

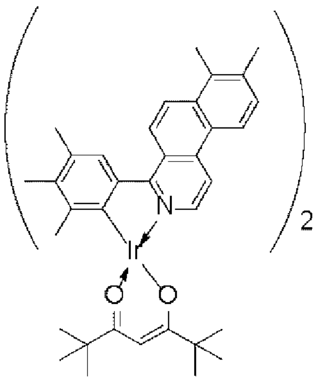

HH4

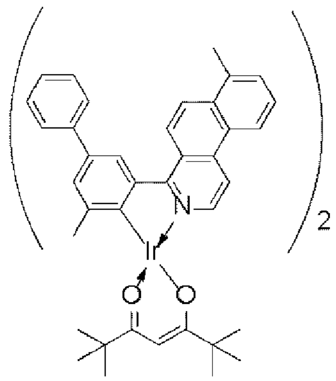

HH5

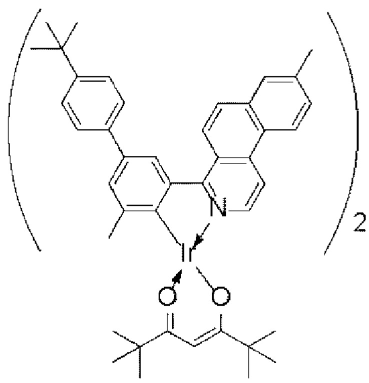

HH6

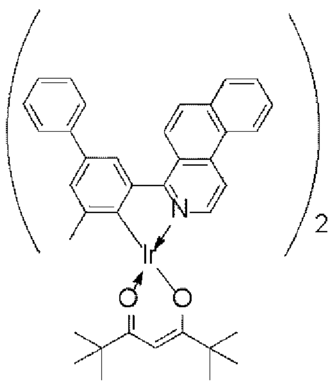

HH7

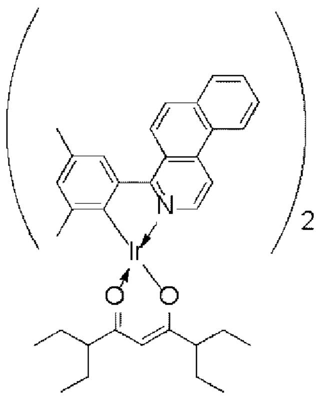

HH8

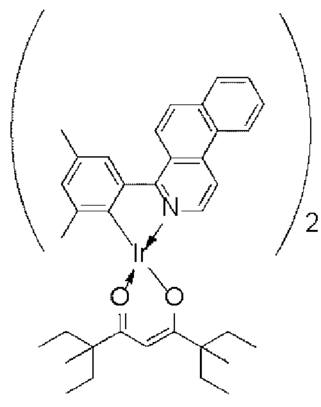

HH9

[Chem. 33]

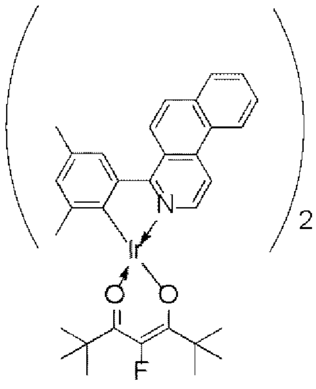

HH10

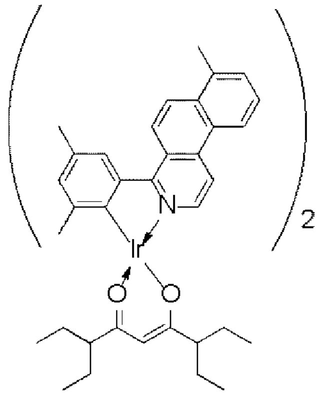

HH11

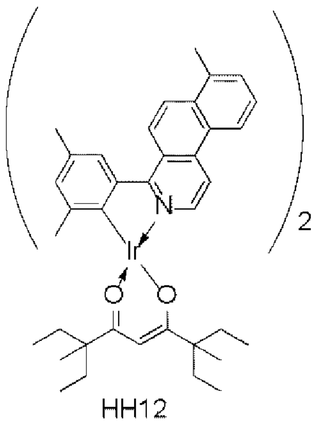

HH12

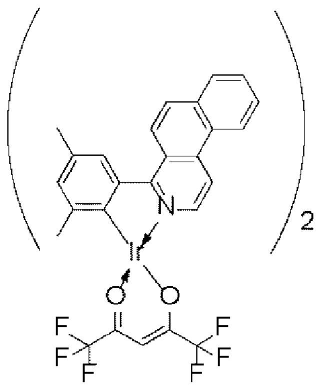

HH13

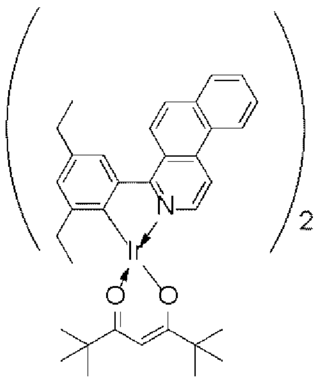

HH14

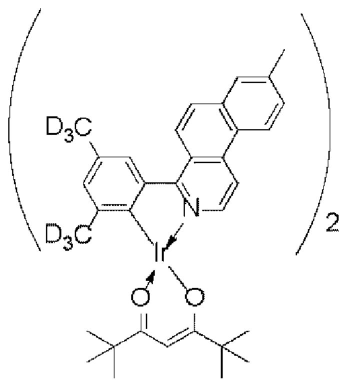

HH15

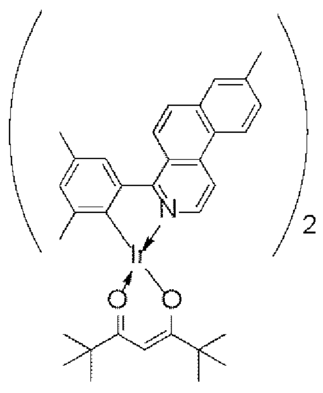

HH16

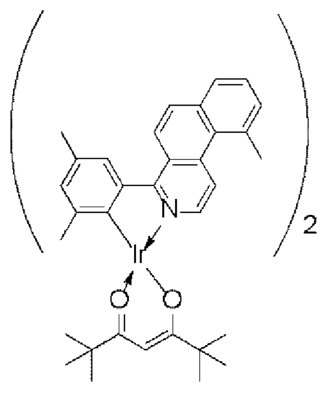

HH17

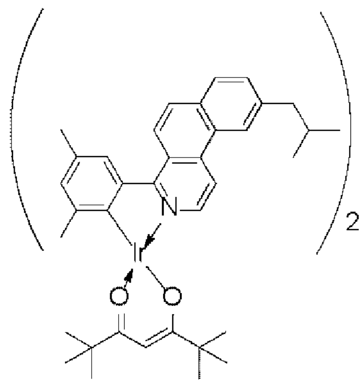

HH18

[Chem. 34]

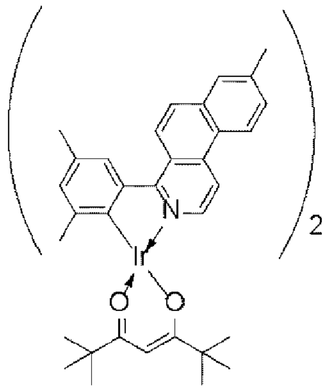

HH19

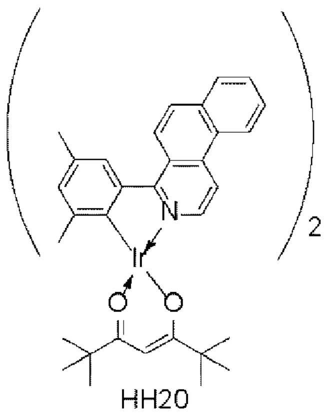

HH20

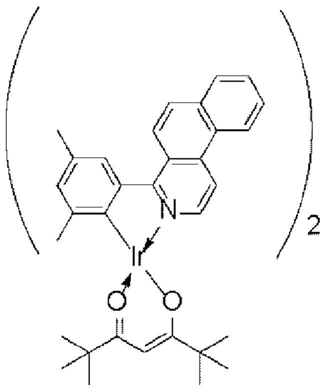

HH21

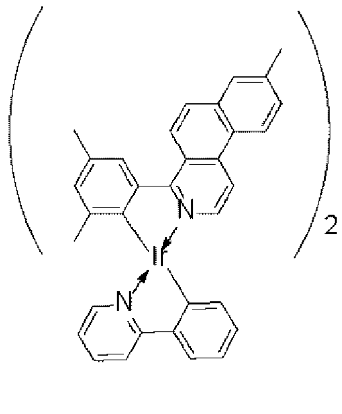

HH22

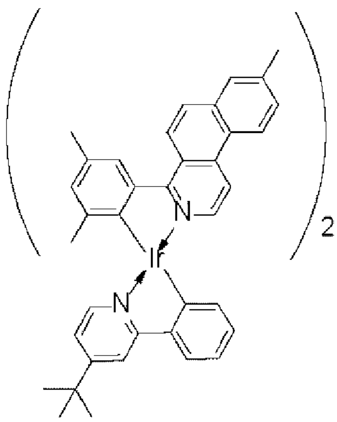

HH23

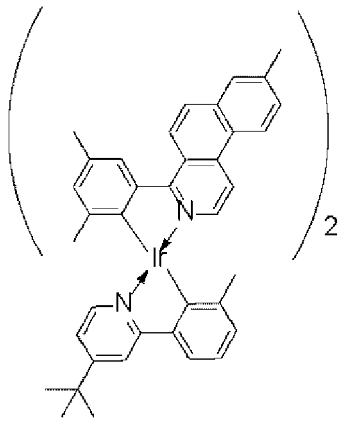

HH24

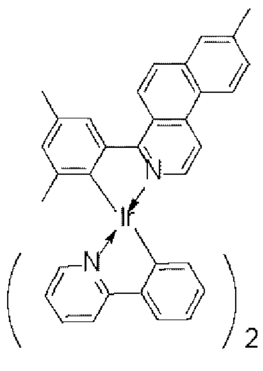

HH25

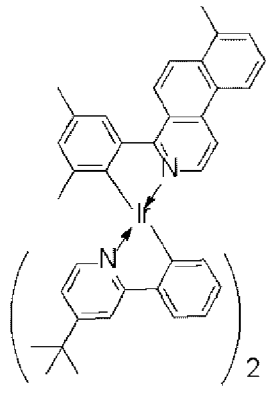

HH26

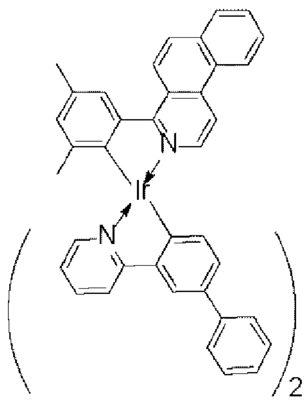

HH27

[Chem. 35]

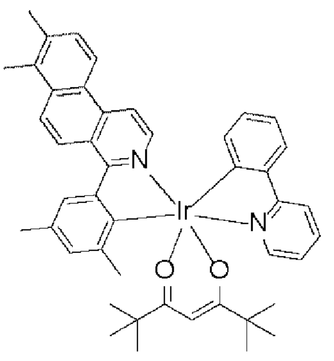

HH28

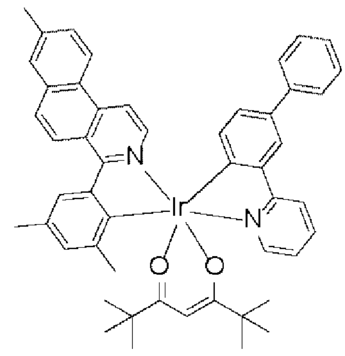

HH29

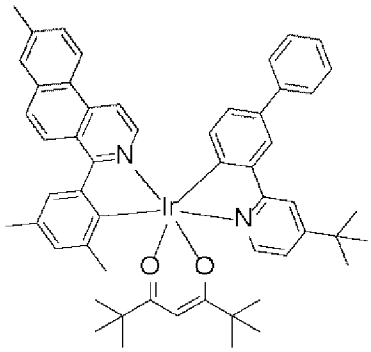

HH30

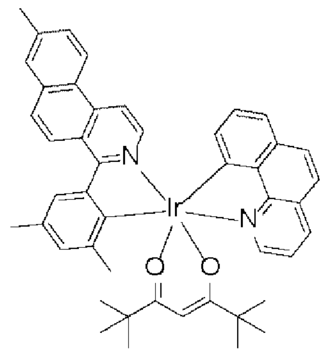

HH31

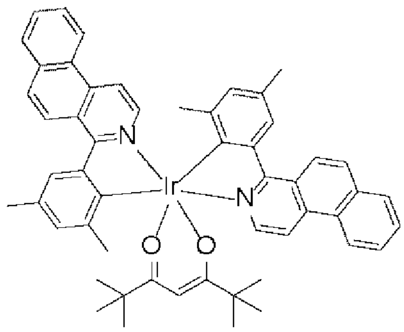

HH32

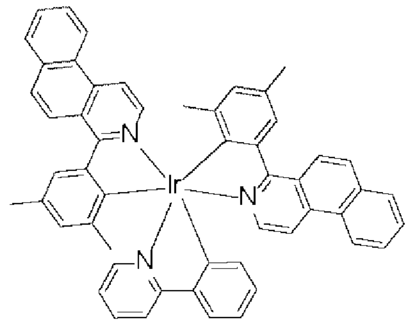

HH33

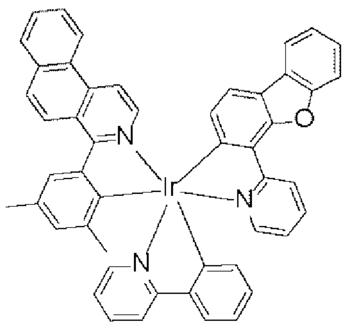

HH34

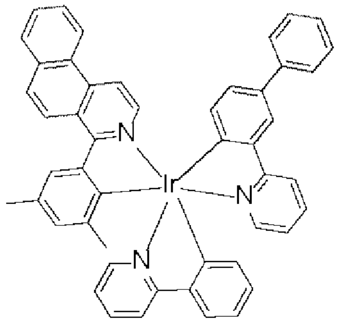

HH35

[Chem. 36]

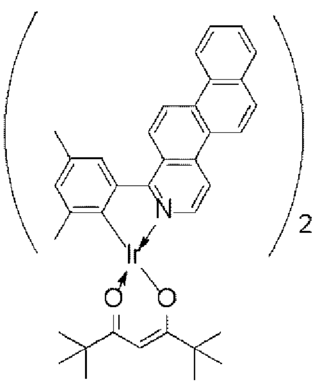

II1

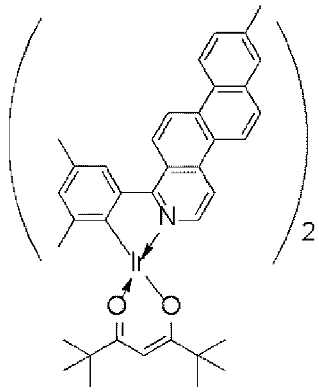

II2

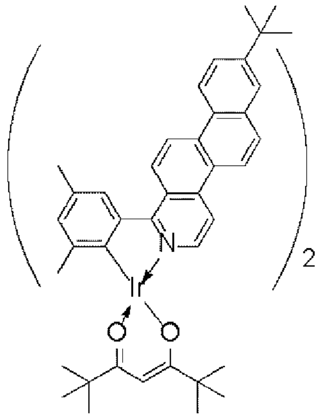

II3

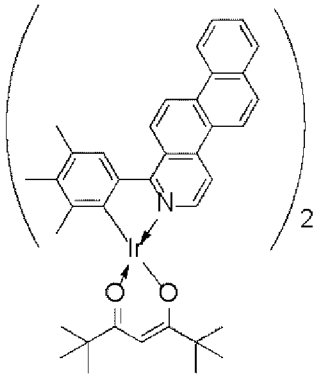

II4

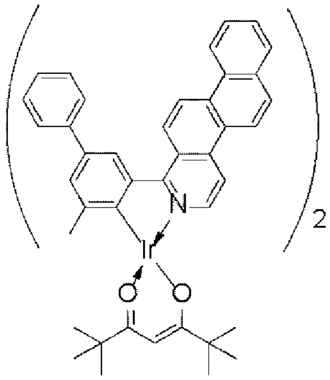

II5

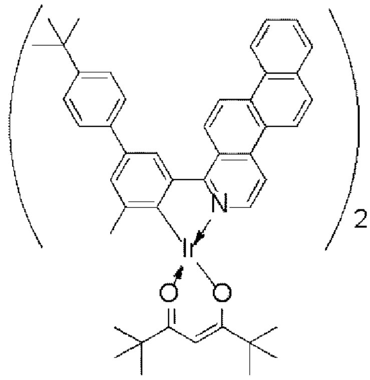

II6

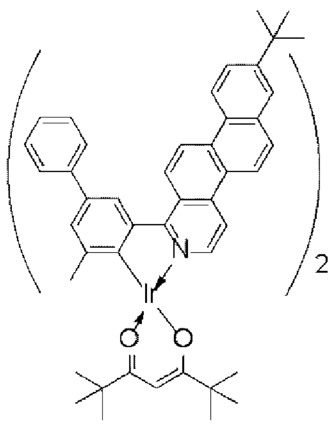

II7

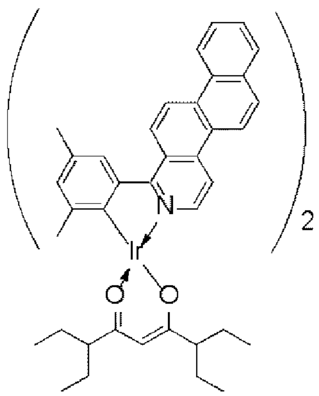

II8

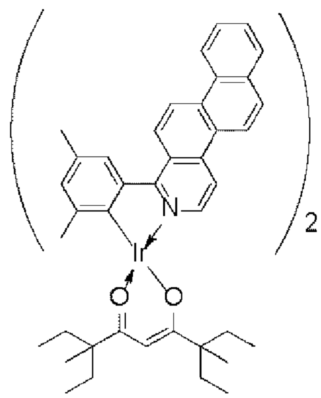

II9

[Chem. 37]

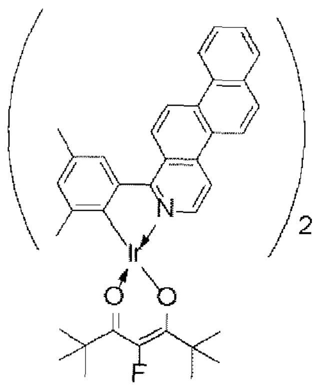

II10

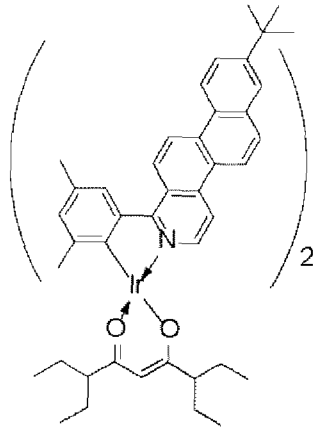

II11

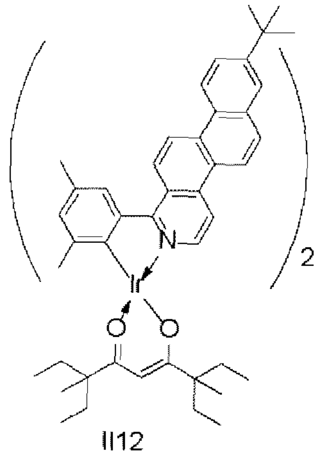

II12

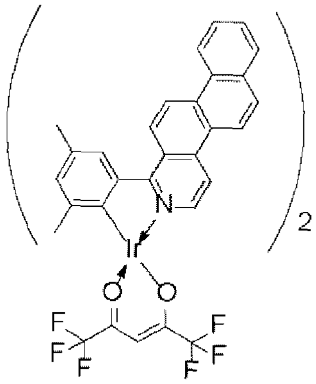

II13

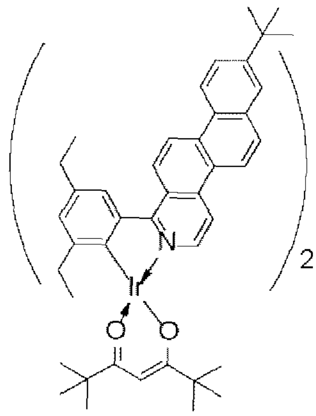

II14

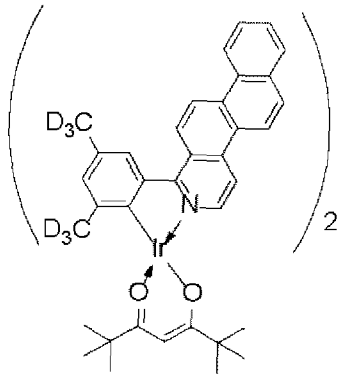

II15

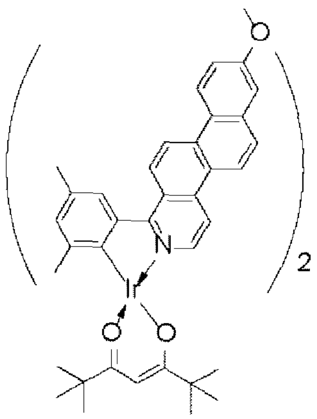

II16

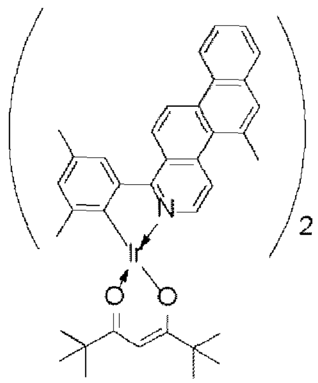

II17

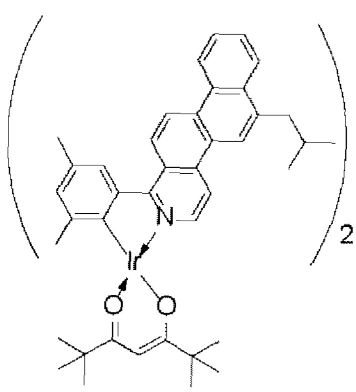

II18

[Chem. 38]

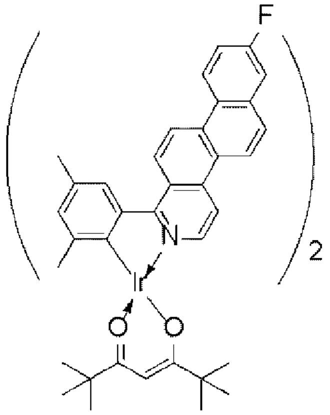

II19

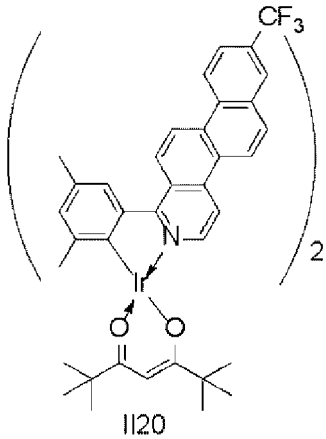

II20

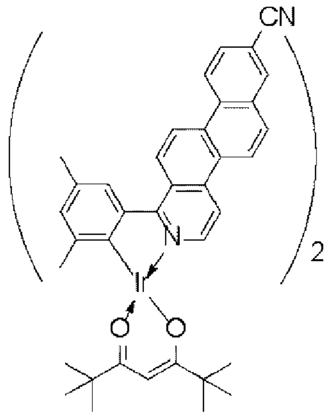

II21

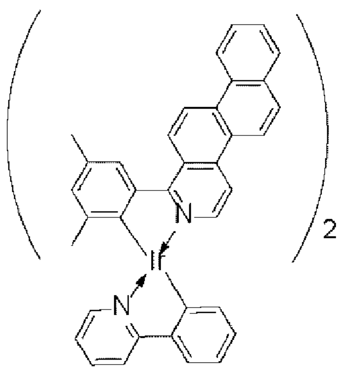

II22

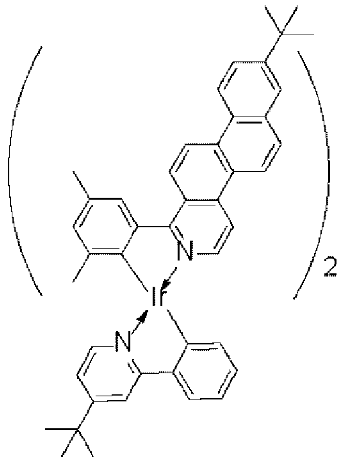

II23

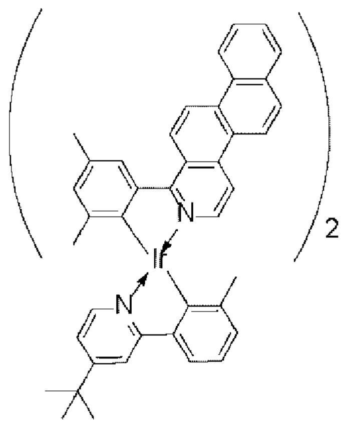

II24

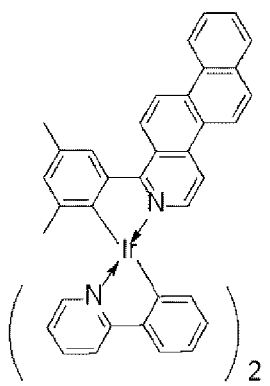

II25

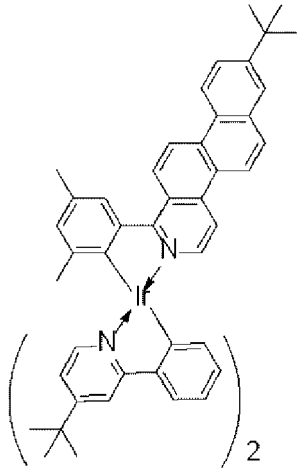

II26

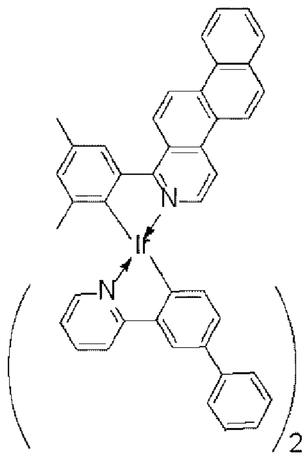

II27

[Chem. 39]

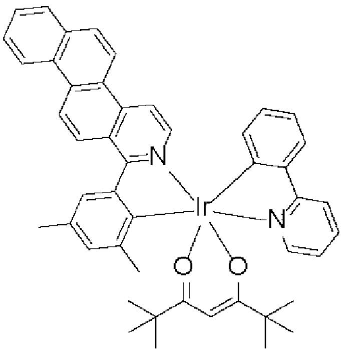

II28

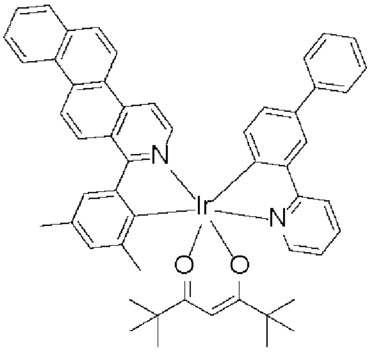

II29

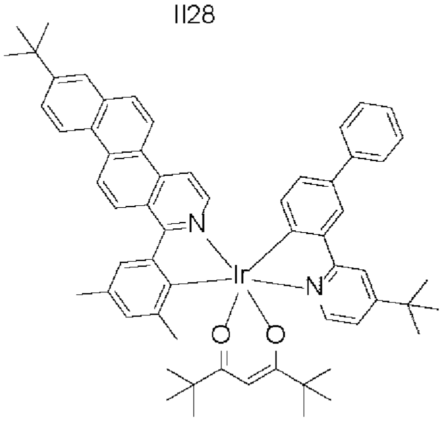

II30

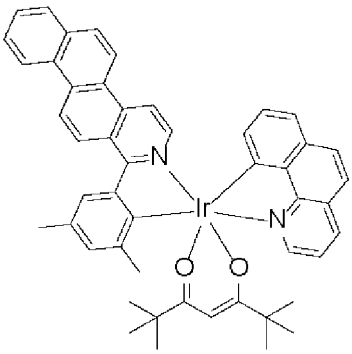

II31

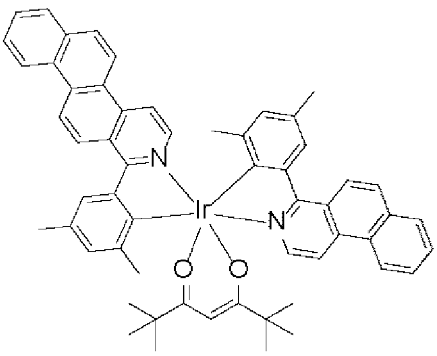

II32

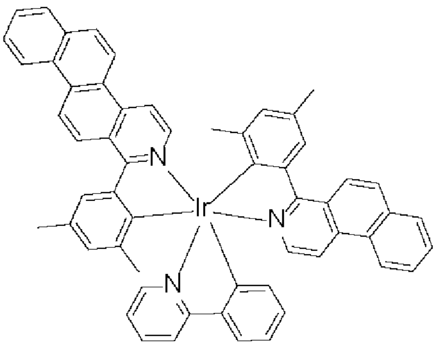

II33

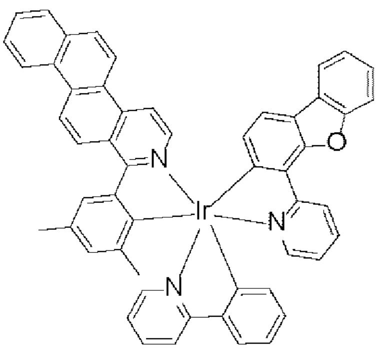

II34

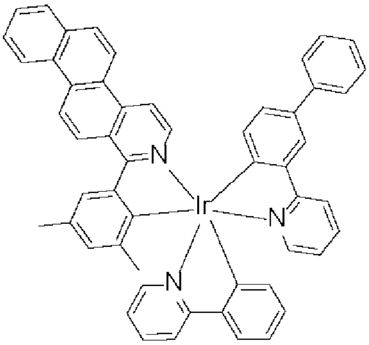

II35

Among these organometallic complexes, the exemplary compounds belonging to the groups AA (AA1 to AA30) and BB (BB1 to BB30) are compounds with at least a phenanthrene ring in a ligand of the Ir complex. Thus, the fused rings are composed of an sp2 hybrid orbital, and the compounds are therefore particularly stable.

Among these organometallic complexes, the exemplary compounds belonging to the group CC (CC1 to CC30)

are compounds with at least a triphenylene ring in a ligand of the Ir complex. Thus, the fused rings are composed of an sp2 hybrid orbital, and the compounds are therefore particularly stable.

Among these organometallic complexes, the exemplary compounds belonging to the group DD (DD1 to DD56) are compounds with at least a dibenzofuran ring or a dibenzothiophene ring in a ligand of the Ir complex. Thus, these compounds contain an oxygen atom or a sulfur atom in a fused ring. Abundant lone pairs in these atoms can enhance charge transport ability and make it particularly easy to adjust the carrier balance of the compounds.

Among these organometallic complexes, the exemplary compounds belonging to the groups EE (EE1 to EE25), FF (FF1 to FF35), and GG (GG1 to GG35) are compounds with at least a benzofluorene ring in a ligand of the Ir complex. Thus, these compounds further have a substituent at position 9 of fluorene. Thus, the substituent in the direction perpendicular to the in-plane direction of the fluorene ring can particularly suppress overlapping of fused rings. Thus, the compounds have particularly high sublimability.

Among these organometallic complexes, the exemplary compounds belonging to the group HH (HH1 to HH35) are compounds with at least a benzoisoquinoline ring in a ligand of the Ir complex. Thus, these compounds contain a N atom in a fused ring, and lone pairs in the atom and high electronegativity can enhance charge transport ability and make it particularly easy to adjust the carrier balance of the compounds.

Among these organometallic complexes, the exemplary compounds belonging to the group II (II1 to II35) are compounds with at least a naphthoisoquinoline ring in a ligand of the Ir complex. Thus, these compounds contain a N atom in a fused ring, and lone pairs in the atom and high electronegativity can enhance charge transport ability and make it particularly easy to adjust the carrier balance of the compounds.

(Other Compounds)

Examples of other compounds that can be used for the organic light-emitting element according to the present embodiment are described below.

A hole injection/transport material suitably used for the hole injection layer or the hole transport layer is preferably a material with high hole mobility that can facilitate hole injection from the positive electrode and that can transport injected holes to the light-emitting layer. Furthermore, a material with a high glass transition temperature is preferred to reduce degradation of film quality, such as crystallization, in an organic light-emitting element. Examples of a low-molecular-weight or high-molecular-weight material with hole injection/transport ability include, but are not limited to, a triarylamine derivative, an aryl carbazole derivative, a phenylenediamine derivative, a stilbene derivative, a phthalocyanine derivative, a porphyrin derivative, polyvinylcarbazole, polythiophene, and another electrically conductive polymer. Furthermore, the hole injection/transport material can also be suitable for use in an electron-blocking layer.

Specific examples of a compound that can be used as a hole injection/transport material include, but are not limited to, the following.

[Chem. 40]

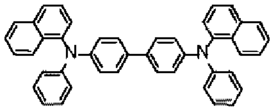
HT1

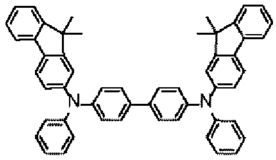
HT2

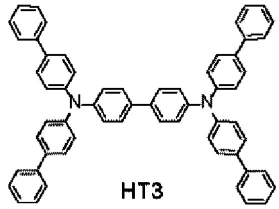
HT3

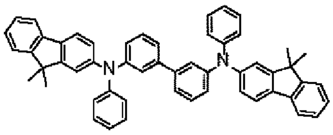
HT4

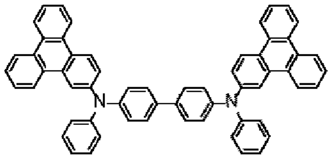
HT5

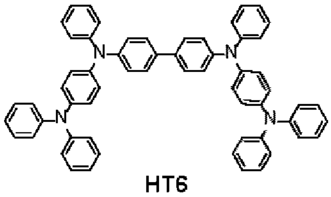
HT6

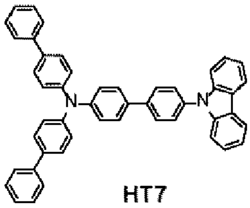
HT7

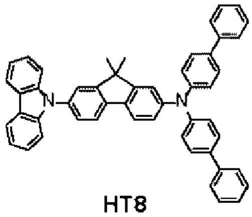
HT8

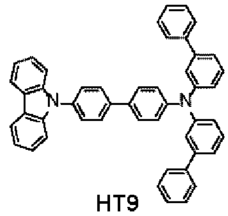
HT9

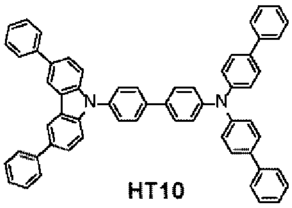
HT10

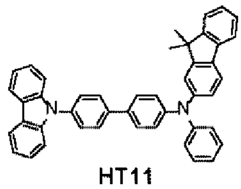
HT11

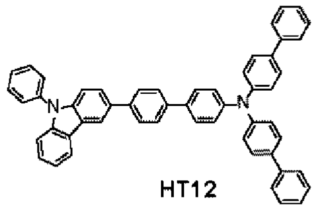
HT12

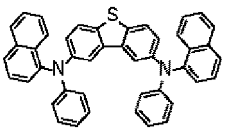
HT13

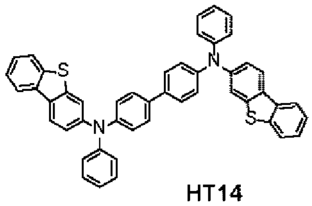
HT14

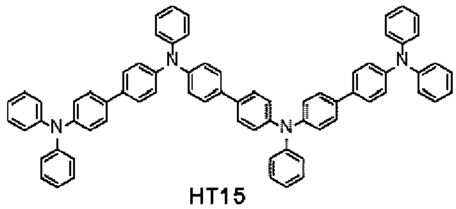
HT15

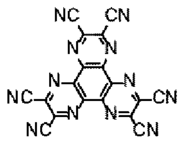
HT16

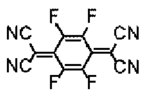
HT17

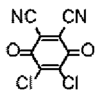
HT18

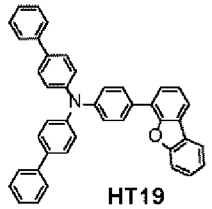
HT19

A light-emitting material mainly related to the light-emitting function may be, in addition to an organic compound represented by the general formula [1], a fused-ring compound (for example, a fluorene derivative, a naphthalene derivative, a pyrene derivative, a perylene derivative, a tetracene derivative, an anthracene derivative, rubrene, or the like), a quinacridone derivative, a coumarin derivative, a stilbene derivative, an organoaluminum complex, such as tris(8-quinolinolato)aluminum, an iridium complex, a platinum complex, a rhenium complex, a copper complex, an europium complex, a ruthenium complex, or a polymer derivative, such as a poly(phenylene vinylene) derivative, a polyfluorene derivative, or a polyphenylene derivative.

Specific examples of a compound that can be used as a light-emitting material include, but are not limited to, the following.

[Chem. 41]

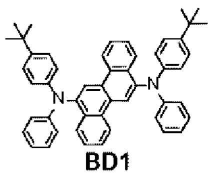
BD1
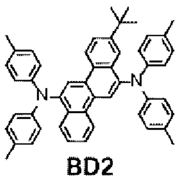
BD2
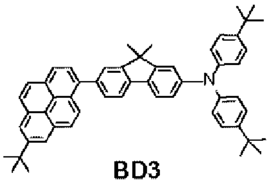
BD3
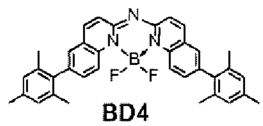
BD4
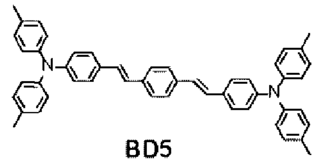
BD5
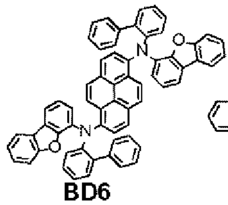
BD6
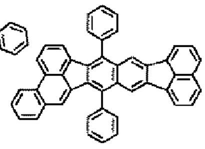
BD7
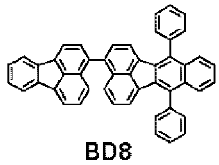
BD8
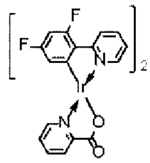
BD9
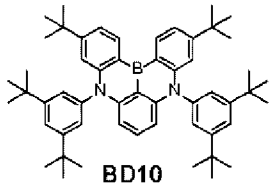
BD10
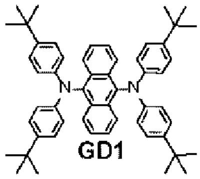
GD1
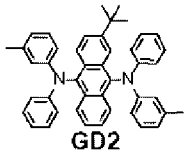
GD2
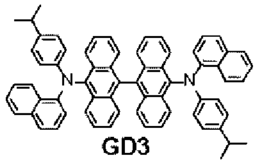
GD3
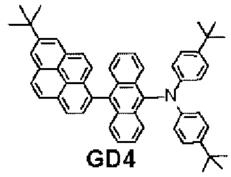
GD4
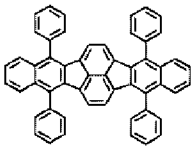
GD5
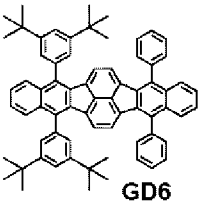
GD6
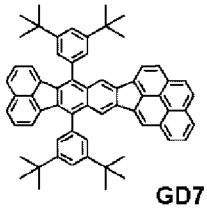
GD7
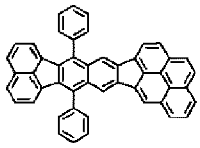
GD8
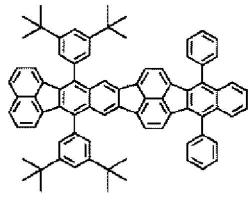
GD9
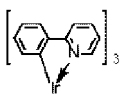
GD10
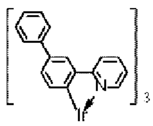
GD11
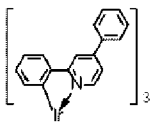
GD12
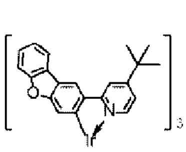
GD13
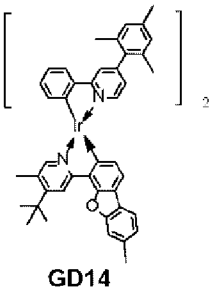
GD14
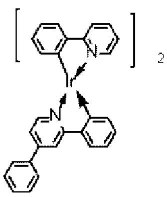
GD15

[Chem. 42]

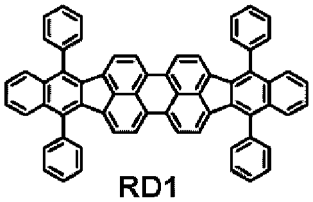
RD1

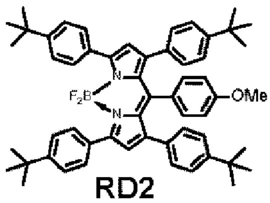
RD2

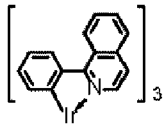
RD3

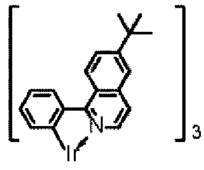
RD4

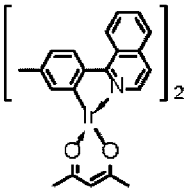
RD5

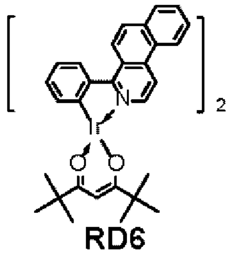
RD6

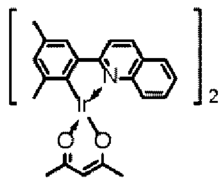
RD7

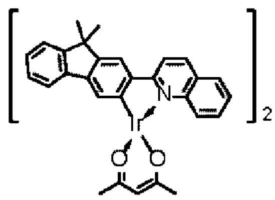
RD8

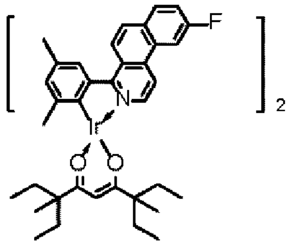
RD9

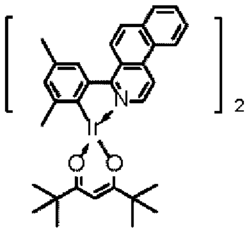
RD10

A light-emitting layer host or a light-emitting assist material in the light-emitting layer may be, in addition to the materials of the exemplary compounds A to D, an aromatic hydrocarbon compound or a derivative thereof, a carbazole derivative, a dibenzofuran derivative, a dibenzothiophene derivative, an organoaluminum complex, such as tris(8-quinolinolato)aluminum, an organoberyllium complex, or the like.

In particular, the assist material is preferably a material with a carbazole structure in a skeleton thereof, a material with an azine ring in a skeleton thereof, or a material with a xanthone structure in a skeleton thereof. This is because these materials have high electron-donating ability and electron-withdrawing ability, and the HOMO and LUMO can be easily adjusted.

An organic compound represented by the general formula [1] has a structure with a tricyclic or higher polycyclic fused ring bonded to both ends of a linking group composed of a phenylene chain and therefore has a band gap widened to some extent. In particular, a material with the skeleton that can adjust the HOMO or LUMO level is preferred as an assist material. Such an assist material in combination with an organic compound represented by the general formula [1] can achieve a good carrier balance.

Specific examples of a compound used as a host or an assist material contained in the light-emitting layer together with a compound represented by the general formula [1] or contained in another light-emitting layer are described below. As a matter of course, the present invention is not limited thereto.

Among the following specific examples, materials with a carbazole skeleton that is preferred for an assist material are EM32 to EM38. Materials with an azine ring that is preferred for an assist material in a skeleton are EM35, EM36, EM37, EM38, EM39, and EM40. Materials with xanthone that is preferred for an assist material in a skeleton are EM28 and EM30.

[Chem. 43]

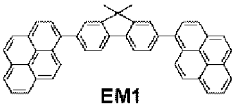
EM1
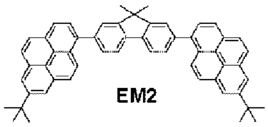
EM2
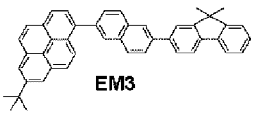
EM3
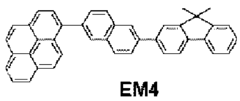
EM4
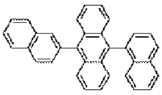
EM5
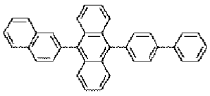
EM6
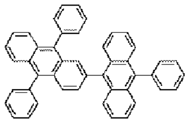
EM7
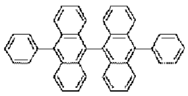
EM8
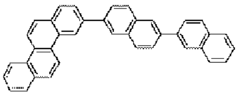
EM9
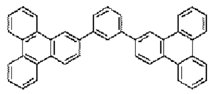
EM10
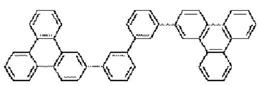
EM11
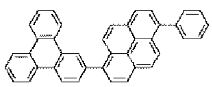
EM12
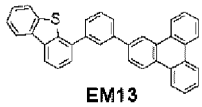
EM13
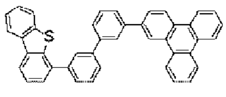
EM14
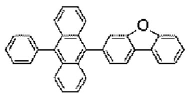
EM15
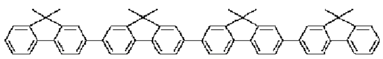
EM16
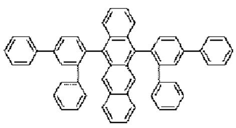
EM17
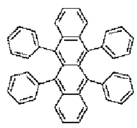
EM18
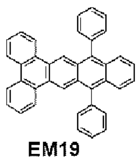
EM19
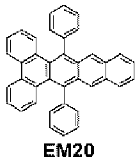
EM20
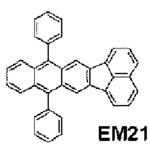
EM21
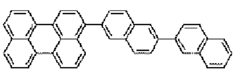
EM22
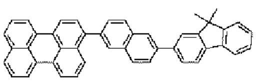
EM23
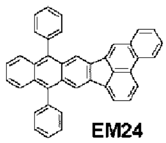
EM24
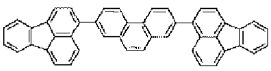
EM25
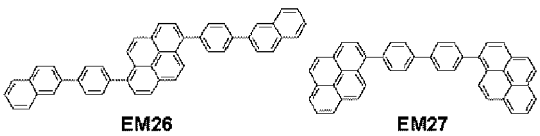
EM26
EM27
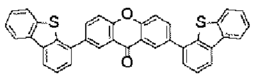
EM28
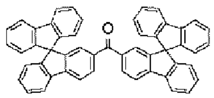
EM29
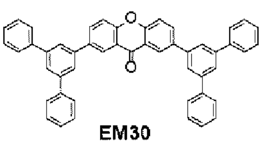
EM30
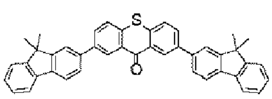
EM31
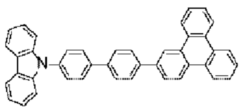
EM32
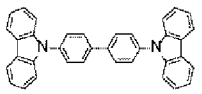
EM33
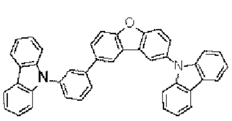
EM34
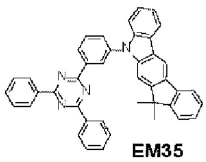
EM35
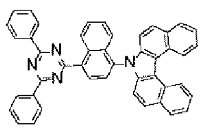
EM36
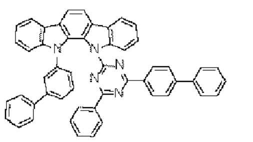
EM37
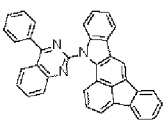
EM38
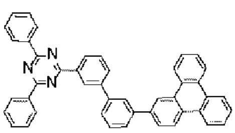
EM39
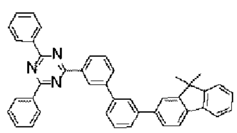
EM40

An electron transport material can be selected from materials that can transport an electron injected from the negative electrode to the light-emitting layer and is selected in consideration of the balance with the hole mobility of a hole transport material and the like. A material with electron transport ability may be an oxadiazole derivative, an oxazole derivative, a pyrazine derivative, a triazole derivative, a triazine derivative, a quinoline derivative, a quinoxaline derivative, a phenanthroline derivative, an organoaluminum complex, and a fused-ring compound (for example, a fluorene derivative,

a naphthalene derivative, a chrysene derivative, an anthracene derivative, or the like). Furthermore, the electron transport material is also suitable for use in a hole-blocking layer.

Specific examples of a compound that can be used as an electron transport material include, but are not limited to, the following.

[Chem. 44]

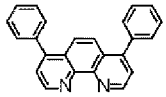
ET1
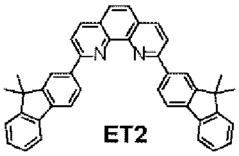
ET2
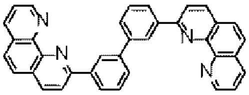
ET3
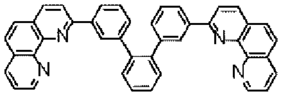
ET4
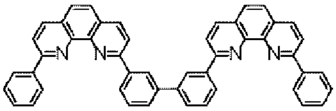
ET5
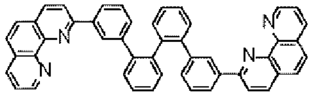
ET6
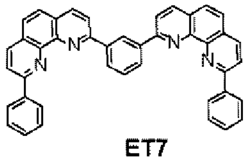
ET7
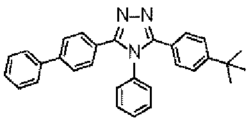
ET8
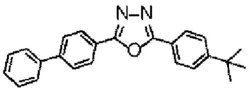
ET9
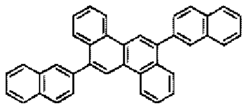
ET10
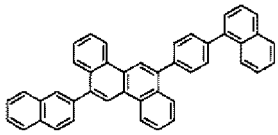
ET11
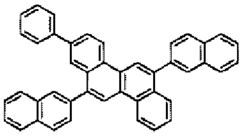
ET12
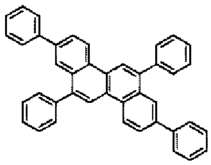
ET13
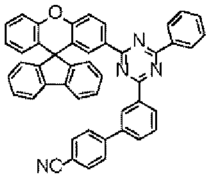
ET14
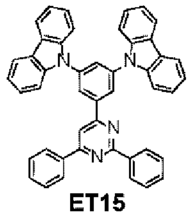
ET15
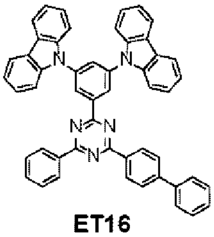
ET16
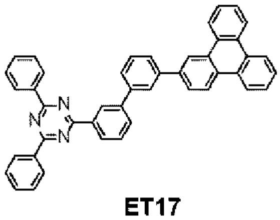
ET17
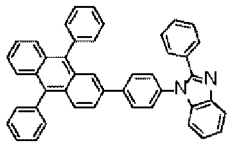
ET18
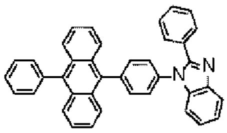
ET19
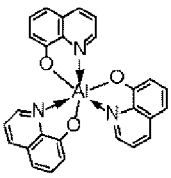
ET20
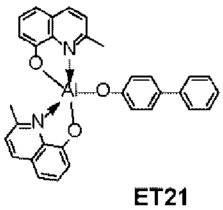
ET21
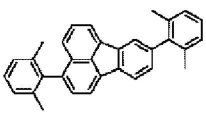
ET22
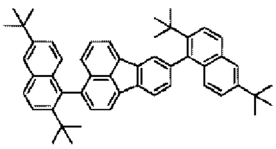
ET23
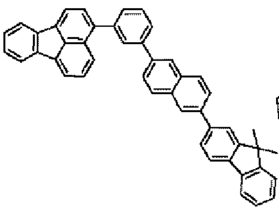
ET24
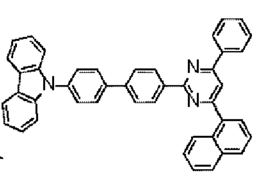
ET25
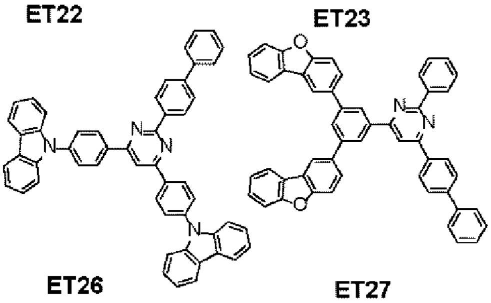
ET26
ET27

Constituents other than the organic compound layers constituting the organic light-emitting element according to the present embodiment are described below. The organic light-emitting element may include a first electrode, an organic compound layer, and a second electrode on a substrate. One of the first electrode and the second electrode is a positive electrode, and the other is a negative electrode. A protective layer, a color filter, or the like may be provided on the second electrode. When a color filter is provided, a planarization layer may be provided between the color filter and a protective layer. The planarization layer may be composed of an acrylic resin or the like.

The substrate may be formed of quartz, glass, silicon, resin, metal, or the like. The substrate may have a switching element, such as a transistor, and wiring, on which an insulating layer may be provided. The insulating layer may be formed of any material, provided that the insulating layer can have a contact hole to ensure electrical connection between the positive electrode and wiring and can be insulated from unconnected wiring. For example, the insulating layer may be formed of a resin, such as polyimide, silicon oxide, or silicon nitride.

A constituent material of the positive electrode preferably has as large a work function as possible. Examples thereof include a metal element, such as gold, platinum, silver, copper, nickel, palladium, cobalt, selenium, vanadium, or tungsten, a mixture thereof, an alloy thereof, and a metal oxide, such as tin oxide, zinc oxide, indium oxide, indium tin oxide (ITO), or indium zinc oxide. An electrically conductive polymer, such as polyaniline, polypyrrole, or polythiophene, may also be used. These electrode materials may be used alone or in combination. The positive electrode may be composed of a single layer or a plurality of layers. When used as a reflective electrode, for example, chromium, aluminum, silver, titanium, tungsten, molybdenum, an alloy thereof, or a laminate thereof can be used. When used as a transparent electrode, an oxide transparent electroconductive layer, such as indium tin oxide (ITO) or indium zinc oxide, can be used. However, the present disclosure is not limited thereto. The positive electrode may be formed by photolithography.

On the other hand, a constituent material of the negative electrode is preferably a material with a small work function. For example, an alkali metal, such as lithium, an alkaline-earth metal, such as calcium, a metal element, such as aluminum, titanium, manganese, silver, lead, or chromium, or a mixture thereof may be used. An alloy of these metal elements may also be used. For example, magnesium-silver, aluminum-lithium, aluminum-magnesium, silver-copper, or zinc-silver may be used. A metal oxide, such as indium tin oxide (ITO), may also be used. These electrode materials may be used alone or in combination. The negative electrode may be composed of a single layer or a plurality of layers. In particular, silver is preferably used, and a silver alloy is more preferably used to reduce the aggregation of silver. As long as the aggregation of silver can be reduced, the alloy may have any ratio. For example, it may be 1:1.

The negative electrode may be, but is not limited to, an oxide electroconductive layer, such as ITO, for a top emission element or a reflective electrode, such as aluminum (Al), for a bottom emission element. The negative electrode may be formed by any method. More preferably, a direct-current or alternating-current sputtering method can achieve good film coverage and easily decrease resistance.

A protective layer may be provided after the negative electrode is formed. For example, a glass sheet with a moisture absorbent may be attached to the negative electrode to decrease the amount of water or the like entering the organic compound layer and to reduce the occurrence of display defects. In another embodiment, a passivation film of silicon nitride or the like may be provided on the negative electrode to decrease the amount of water or the like entering the organic compound layer. For example, after the negative electrode is formed, the negative electrode is transferred to another chamber without breaking the vacuum, and a silicon nitride film with a thickness of 2 um may be formed as a protective layer by a chemical vapor deposition (CVD) method. The film formation by the CVD method may be followed by the formation of a protective layer by an atomic layer deposition (ALD) method.

Furthermore, each pixel may be provided with a color filter. For example, a color filter that matches the size of the pixel may be provided on another substrate and may be bonded to the substrate of the organic light-emitting element, or a color filter may be patterned by photolithography on the protective layer formed of silicon oxide or the like.

An organic compound layer (a hole injection layer, a hole transport layer, an electron-blocking layer, a light-emitting layer, a hole-blocking layer, an electron transport layer, an electron injection layer, etc.) constituting the organic light-emitting element according to the present embodiment is formed by the following method. That is, an organic compound layer may be formed by a dry process, such as a vacuum deposit method, an ionized deposition method, sputtering, or plasma. Instead of the dry process, a wet process may also be employed in which a layer is formed by a known coating method (for example, spin coating, dipping, a casting method, an LB method, an ink jet method, or the like) using an appropriate solvent. A layer formed by a vacuum deposition method, a solution coating method, or the like undergoes little crystallization or the like and has high temporal stability. When a film is formed by a coating method, the film may also be formed in combination with an appropriate binder resin. The binder resin may be, but is not limited to, a polyvinylcarbazole resin, a polycarbonate resin, a polyester resin, an ABS resin, an acrylic resin, a polyimide resin, a phenolic resin, an epoxy resin, a silicon resin, or a urea resin. These binder resins may be used alone or in combination as a homopolymer or a copolymer. If necessary, an additive agent, such as a known plasticizer, oxidation inhibitor, and/or ultraviolet absorbent, may also be used.

<<Apparatus Including Organic Light-Emitting Element>>

The organic light-emitting element according to the present embodiment can be used as a constituent of a display apparatus or a lighting apparatus. Other applications include an exposure light source for an electrophotographic image-forming apparatus, a backlight for a liquid crystal display, and a light-emitting apparatus with a color filter in a white light source.

The display apparatus may be an image-information-processing apparatus that includes an image input unit for inputting image information from an area CCD, a linear CCD, a memory card, or the like, includes an information processing unit for processing the input information, and displays an input image on a display unit. The display apparatus may have a plurality of pixels, and at least one of the pixels may include the organic light-emitting element according to the present embodiment and a transistor coupled to the organic light-emitting element. The substrate may be a semiconductor substrate formed of silicon or the like, and the transistor may be a MOSFET formed on the substrate.

A display unit of an imaging apparatus or an ink jet printer may have a touch panel function. A driving system of the touch panel function may be, but is not limited to, an infrared radiation system, an electrostatic capacitance system, a resistive film system, or an electromagnetic induction system. The display apparatus may be used for a display unit of a multifunction printer.

Next, a display apparatus according to the present embodiment is described below with reference to the accompanying drawings.

Figs. 1A and 1B are schematic cross-sectional views of an example of a display apparatus that includes an organic light-emitting element and a transistor coupled to the organic light-emitting element. The transistor is an example of an active element. The transistor may be a thin-film transistor (TFT).

Fig. 1A illustrates an example of a pixel serving as a constructional element of the display apparatus according to the present embodiment. The pixel has subpixels 10. The subpixels are 10R, 10G, and 10B with different emission colors. The emission colors may be distinguished by the wavelength of light emitted from the light-emitting layer, or light emitted from each subpixel may be selectively transmitted or color-converted with a color filter or the like. Each subpixel has, on an interlayer insulating layer 1, a reflective electrode 2 as a first electrode, an insulating layer 3 covering the ends of the reflective electrode 2, organic compound layers 4 covering the first electrode and the insulating layer, a transparent electrode 5, a protective layer 6, and a color filter 7.

A transistor and/or a capacitor element may be provided under or inside the interlayer insulating layer 1. The transistor may be electrically connected to the first electrode via a contact hole (not shown) or the like.

The insulating layer 3 is also referred to as a bank or a pixel separation film. The insulating layer 3 covers the ends of the first electrode and surrounds the first electrode. A portion of the first electrode not covered with the insulating layer is in contact with the organic compound layers 4 and serves as a light-emitting region.

The organic compound layers 4 include a hole injection layer 41, a hole transport layer 42, a first light-emitting layer 43, a second light-emitting layer 44, and an electron transport layer 45.

The second electrode 5 may be a transparent electrode, a reflective electrode, or a semitransparent electrode.

The protective layer 6 reduces the penetration of moisture into the organic compound layers. The protective layer is illustrated as a single layer but may be a plurality of layers. The protective layer 6 may include an inorganic compound layer and an organic compound layer.

The color filter 7 is divided into 7R, 7G, and 7B according to the color. The color filter may be formed on a planarization film (not shown). Furthermore, a resin protective layer (not shown) may be provided on the color filter. The color filter may be formed on the protective layer 6. Alternatively, the color filter 7 may be bonded after being provided on an opposite substrate, such as a glass substrate.

A display apparatus 100 illustrated in Fig. 1B includes an organic light-emitting element 26 and a TFT 18, which is an example of a transistor. The display apparatus includes a substrate 11 made of glass, silicon, or the like and an insulating layer 12 on the substrate 11. An active element, such as the TFT 18, and a gate electrode 13, a gate-insulating film 14, and a semiconductor layer 15 of the active element are disposed on the insulating layer 12.

The TFT 18 includes a semiconductor layer 15, a drain electrode 16, and a source electrode 17. The TFT 18 is covered with an insulating film 19. A positive electrode 21 constituting the organic light-emitting element 26 is connected to the source electrode 17 via a contact hole 20.

The method for electrically connecting the electrodes (the positive electrode 21 and a negative electrode 23) of the organic light-emitting element 26 to the electrodes (the source electrode 17 and the drain electrode 16) of the TFT is not limited to the embodiment illustrated in Fig. 1B. More specifically, it is only necessary to electrically connect either the positive electrode 21 or the negative electrode 23 to either the source electrode 17 or the drain electrode 16 of the TFT 18.

Although an organic compound layer 22 is a single layer in the display apparatus 100 illustrated in Fig. 1B, the organic compound layer 22 may be composed of a plurality of layers. The negative electrode 23 is covered with a first protective layer 25 and a second protective layer 24 for reducing degradation of the organic light-emitting element.

Although the display apparatus 100 illustrated in Fig. 1B includes a transistor as a switching element, another switching element, such as a MIM element, may be used instead.

The transistor used in the display apparatus 100 in Fig. 1B is not limited to a thin-film transistor including an active layer on an insulating surface of a substrate and may also be a transistor including a single crystal silicon wafer. The active layer may be single-crystal silicon, non-single-crystal silicon, such as amorphous silicon or microcrystalline silicon, or a non-single-crystal oxide semiconductor, such as indium zinc oxide or indium gallium zinc oxide. The thin-film transistor is also referred to as a TFT element.

The transistor in the display apparatus 100 illustrated in Fig. 1B may be formed within a substrate, such as a Si substrate. The phrase "formed within a substrate" means that the substrate, such as a Si substrate, itself is processed to form the transistor. Thus, the transistor within the substrate can be considered that the substrate and the transistor are integrally formed.

In the organic light-emitting element according to the present embodiment, the luminous brightness is controlled with the TFT, which is an example of a switching element. The organic light-emitting element can be provided in a plurality of planes to display an image at each luminous brightness. The switching element according to the present embodiment is not limited to the TFT and may be a transistor formed of low-temperature polysilicon or an active-matrix driver formed on a substrate, such as a Si substrate. The phrase "on a substrate" may also be referred to as "within a substrate". Whether a transistor is provided within a substrate or a TFT is used depends on the size of a display unit. For example, for an approximately 0.5-inch display unit, an organic light-emitting element is preferably provided on a Si substrate.

Fig. 2 is a schematic view of an example of a display apparatus according to the present embodiment. A display apparatus 1000 may include a touch panel 1003, a display panel 1005, a frame 1006, a circuit substrate 1007, and a battery 1008 between an upper cover 1001 and a lower cover 1009. The touch panel 1003 and the display panel 1005 are coupled to flexible print circuits FPC 1002 and 1004, respectively. Transistors are printed on the circuit substrate 1007. The battery 1008 is not necessarily provided when the display apparatus is not a mobile device, or may be provided at another position even when the display apparatus is a mobile device.

The display apparatus according to the present embodiment may be used for a display unit of an imaging apparatus that includes an optical unit with a plurality of lenses and an imaging element for receiving light passing through the optical unit. The imaging apparatus may include a display unit for displaying information acquired by the imaging element. The display unit may be a display unit exposed outside from the imaging apparatus or a display unit located in a finder. The imaging apparatus may be a digital camera or a digital video camera. The imaging apparatus may also be referred to as a photoelectric conversion apparatus.

Fig. 3A is a schematic view of an example of an imaging apparatus according to the present embodiment. An imaging apparatus 1100 may include a viewfinder 1101, a rear display 1102, an operating unit 1103, and a housing 1104. The viewfinder 1101 may include the display apparatus according to the present embodiment. In such a case, the display apparatus may display environmental information, imaging instructions, and the like as well as an image to be captured. The environmental information may include the intensity of external light, the direction of external light, the travel speed of the photographic subject, the possibility that the photographic subject is shielded by a shielding material, and the like.

Because the appropriate timing for imaging is a short time, it is better to display information as early as possible. Thus, a display apparatus including the organic light-emitting element according to the present embodiment is preferably used. This is because the organic light-emitting element has a high response speed. A display apparatus including the organic light-emitting element can be more suitably used than these apparatuses and liquid crystal displays that require a high display speed.

The imaging apparatus 1100 includes an optical unit (not shown). The optical unit has a plurality of lenses and focuses an image on an imaging element in the housing 1104. The focus of the lenses can be adjusted by adjusting their relative positions. This operation can also be automatically performed.

The display apparatus according to the present embodiment may include color filters of red, green, and blue colors. In the color filters, the red, green, and blue colors may be arranged in a delta arrangement.

The display apparatus according to the present embodiment may be used for a display unit of electronic equipment, such as a mobile terminal. Such a display apparatus may have both a display function and an operation function. Examples of the mobile terminal include mobile phones, such as smartphones, tablets, and head-mounted displays.

Fig. 3B is a schematic view of an example of electronic equipment according to the present embodiment. Electronic equipment 1200 includes a display unit 1201, an operating unit 1202, and a housing 1203. The housing 1203 may include a circuit, a printed circuit board including the circuit, a battery, and a communication unit. The operating unit 1202 may be a button or a touch panel response unit. The operating unit may be a biometric recognition unit that recognizes a fingerprint and releases the lock. Electronic equipment with a communication unit may also be referred to as communication equipment.

Figs. 4A and 4B are schematic views of an example of a display apparatus according to the present embodiment. Fig. 4A illustrates a display apparatus, such as a television monitor or a PC monitor. A display apparatus 1300 includes a frame 1301 and a display unit 1302. A light-emitting apparatus according to the present embodiment may be used for the display unit 1302. The display apparatus 1300 includes a base 1303 for supporting the frame 1301 and the display unit 1302. The base 1303 is not limited to the structure illustrated in Fig. 4A. The lower side of the frame 1301 may also serve as the base. The frame 1301 and the display unit 1302 may be bent so that the display surface of the display unit 1302 is curved. The radius of curvature thereof may be 5000 mm or more and 6000 mm or less.

Fig. 4B is a schematic view of another example of the display apparatus according to the present embodiment. A display apparatus 1310 in Fig. 4B is configured to be foldable and is a so-called foldable display apparatus. The display apparatus 1310 includes a first display unit 1311, a second display unit 1312, a housing 1313, and a folding point 1314. The first display unit 1311 and the second display unit 1312 may include the light-emitting apparatus according to the present embodiment. The first display unit 1311 and the second display unit 1312 may be a single display apparatus without a joint. The first display unit 1311 and the second display unit 1312 can be divided by the folding point. The first display unit 1311 and the second display unit 1312 may display different images or one image.

Fig. 5A is a schematic view of an example of a lighting apparatus according to the present embodiment. A lighting apparatus 1400 may include a housing 1401, a light source 1402, a circuit substrate 1403, an optical film 1404 that transmits light emitted by the light source 1402, and a light-diffusing unit 1405. The light source 1402 may include the organic light-emitting element according to the present embodiment. The optical filter may be a filter for improving the color rendering properties of the light source. The light-diffusing unit can effectively diffuse light from the light source and widely spread light as in illumination. The optical filter and the light-diffusing unit may be provided on the light output side of the lighting apparatus. If necessary, a cover may be provided on the outermost side.

For example, the lighting apparatus is an interior lighting apparatus. The lighting apparatus may emit white light, neutral white light, or light of any color from blue to red. The lighting apparatus may have a light control circuit for controlling such light or a color control circuit for controlling emission color. The lighting apparatus may include the organic light-emitting element according to the present embodiment and a power supply circuit coupled thereto. The power supply circuit is a circuit that converts an AC voltage to a DC voltage. White has a color temperature of 4200 K, and neutral white has a color temperature of 5000 K. The lighting apparatus may have a color filter.

The lighting apparatus according to the present embodiment may include a heat dissipation unit. The heat dissipation unit releases heat from the apparatus to the outside and may be a metal or liquid silicon with a high specific heat.

Fig. 5B is a schematic view of an automobile as an example of a moving body according to the present embodiment. The automobile has a taillight as an example of a lamp. An automobile 1500 may have a taillight 1501, which comes on when a brake operation or the like is performed.

The taillight 1501 may include the organic light-emitting element according to the present embodiment. The taillight 1501 may have a protective member for protecting an organic EL element. The protective member may be formed of any transparent material with moderately high strength and is preferably formed of polycarbonate or the like. The polycarbonate may be mixed with a furan dicarboxylic acid derivative, an acrylonitrile derivative, or the like.

The automobile 1500 may have a body 1503 and a window 1502 on the body 1503. The window 1502 may be a transparent display as long as it is not a window for checking the front and rear of the automobile. The transparent display may include the organic light-emitting element according to the present embodiment. In such a case, constituent materials, such as electrodes, of the organic light-emitting element are transparent materials.

The moving body according to the present embodiment may be a ship, an aircraft, a drone, or the like. The moving body may include a body and a lamp provided on the body. The lamp may emit light to indicate the position of the body. The lamp includes the organic light-emitting element according to the present embodiment.

Application examples of the display apparatus according to one of the embodiments are described below with reference to Figs. 6A and 6B. The display apparatus can be applied to a system that can be worn as a wearable device, such as smart glasses, a head-mounted display (HMD), or smart contact lenses. An imaging and displaying apparatus used in such an application example includes an imaging apparatus that can photoelectrically convert visible light and a display apparatus that can emit visible light.

Fig. 6A illustrates glasses 1600 (smart glasses) according to one application example. An imaging apparatus 1602, such as a complementary metal-oxide semiconductor (CMOS) sensor or a single-photon avalanche photodiode (SPAD), is provided on the front side of a lens 1601 of the glasses 1600. The display apparatus according to one of the embodiments is provided on the back side of the lens 1601.

The glasses 1600 further include a controller 1603. The controller 1603 functions as a power supply for supplying power to the imaging apparatus 1602 and the display apparatus according to one of the embodiments. The controller 1603 controls the operation of the imaging apparatus 1602 and the display apparatus. The lens 1601 has an optical system for focusing light on the imaging apparatus 1602.

Fig. 6B illustrates glasses 1610 (smart glasses) according to one application example. The glasses 1610 have a controller 1612, which includes an imaging apparatus corresponding to the imaging apparatus 1602 and a display

apparatus. A lens 1611 includes an optical system for projecting light from the imaging apparatus of the controller 1612 and the display apparatus, and an image is projected on the lens 1611. The controller 1612 functions as a power supply for supplying power to the imaging apparatus and the display apparatus and controls the operation of the imaging apparatus and the display apparatus. The controller may include a line-of-sight detection unit for detecting the line of sight of the wearer. Infrared radiation may be used to detect the line of sight. An infrared radiation unit emits infrared light to an eyeball of a user who is gazing at a display image. Reflected infrared light from the eyeball is detected by an imaging unit including a light-receiving element to capture an image of the eyeball. A reduction unit for reducing light from the infrared radiation unit to a display unit in a plan view is provided to reduce degradation in image quality.

The line of sight of the user for the display image is detected from the image of the eyeball captured by infrared imaging. Any known technique can be applied to line-of-sight detection using the captured image of the eyeball. For example, it is possible to use a line-of-sight detection method based on a Purkinje image obtained by the reflection of irradiation light by the cornea.

More specifically, a line-of-sight detection process based on a pupil-corneal reflection method is performed. The line of sight of the user is detected by calculating a line-of-sight vector representing the direction (rotation angle) of an eyeball on the basis of an image of a pupil and a Purkinje image included in a captured image of the eyeball using the pupil-corneal reflection method.

A display apparatus according to an embodiment of the present invention may include an imaging apparatus including a light-receiving element and may control a display image on the basis of line-of-sight information of a user from the imaging apparatus.

More specifically, on the basis of the line-of-sight information, the display apparatus determines a first visibility region at which the user gazes and a second visibility region other than the first visibility region. The first visibility region and the second visibility region may be determined by the controller of the display apparatus or may be received from an external controller. In the display region of the display apparatus, the first visibility region may be controlled to have higher display resolution than the second visibility region. In other words, the second visibility region may have lower resolution than the first visibility region.

The display region has a first display region and a second display region different from the first display region, and the priority of the first display region and the second display region depends on the line-of-sight information. The first visibility region and the second visibility region may be determined by the controller of the display apparatus or may be received from an external controller. A region with a higher priority may be controlled to have higher resolution than another region. In other words, a region with a lower priority may have lower resolution.

The first visibility region or a region with a higher priority may be determined by artificial intelligence (AI). The AI may be a model configured to estimate the angle of the line of sight and the distance to a target ahead of the line of sight from an image of an eyeball using the image of the eyeball and the direction in which the eyeball actually viewed in the image as teaching data. The AI program may be stored in the display apparatus, the imaging apparatus, or an external device. The AI program stored in an external device is transmitted to the display apparatus via communication.

For display control based on visual recognition detection, the present disclosure can be applied to smart glasses further having an imaging apparatus for imaging the outside. Smart glasses can display captured external information in real time.

Fig. 7 is a schematic view of an example of an image-forming apparatus according to the present embodiment. An image-forming apparatus 40 is an electrophotographic image-forming apparatus and includes a photosensitive member 27, an exposure light source 28, a charging unit 30, a developing unit 31, a transfer unit 32, a conveying roller 33, and a fixing unit 35. The exposure light source 28 emits light 29, and an electrostatic latent image is formed on the surface of the photosensitive member 27. The exposure light source 28 includes the organic light-emitting element according to the present embodiment. The developing unit 31 contains toner and the like. The charging unit 30 electrifies the photosensitive member 27. The transfer unit 32 transfers a developed image onto a recording medium 34. The conveying roller 33 conveys the recording medium 34. The recording medium 34 is paper, for example. The fixing unit 35 fixes an image on the recording medium 34.

Figs. 8A and 8B are schematic views of the exposure light source 28 in which a plurality of light-emitting portions 36 are arranged on a long substrate. An arrow 37 indicates a longitudinal direction in which the organic light-emitting elements are arranged. This column direction is the same as the direction of the rotation axis of the photosensitive member 27. This direction can also be referred to as the major-axis direction of the photosensitive member 27. In Fig. 8A, the light-emitting portions 36 are arranged in the major-axis direction of the photosensitive member 27. In Fig. 8B, unlike Fig. 8A, the light-emitting portions 36 are alternately arranged in the column direction in the first and second columns. The first and second columns are arranged at different positions in the row direction. In the first column, the light-emitting portions 36 are arranged at intervals. In the second column, the light-emitting portions 36 are arranged at positions corresponding to the spaces between the light-emitting portions 36 of the first column. Thus, the light-emitting portions 36 are also arranged at intervals in the row direction. The arrangement in Fig. 8B can also be referred to as a grid-like pattern, a staggered pattern, or a checkered pattern, for example.

As described above, an apparatus including the organic light-emitting element according to the present embodiment can be used to stably display a high-quality image for extended periods.

Exemplary Embodiments

The present invention is described below with exemplary embodiments. The present invention is not limited to these.

[Exemplary Embodiment 1 (Synthesis of Exemplary Compound A2)]

[Chem. 45]

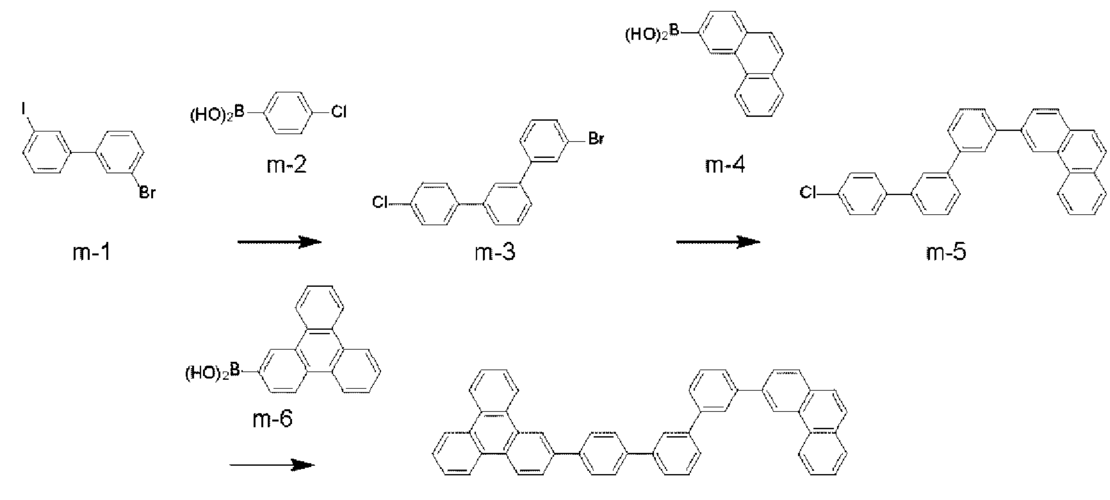
m-1
m-2
m-3
m-4
m-5
m-6
A2

(1) Synthesis of Compound m-3

A 200-ml recovery flask was charged with the following reagent(s) and solvent(s).

Compound m-1: 4.0 g (11.1 mmol)
Compound m-2: 1.7 g (11.1 mmol)
$Pd(PPh_3)_4$: 0.13 g
Toluene: 40 ml
Ethanol: 20 ml
2M-sodium carbonate aqueous solution: 20 ml

The reaction solution was then heated and stirred under reflux in a nitrogen stream for 6 hours. After completion of the reaction, water was added to the product for separation. The product was dissolved in chloroform, was purified by column chromatography (chloroform:heptane), and was recrystallized in toluene/heptane. Thus, 3.0 g (yield: 78%) of a compound m-3 as a white solid was produced.

(2) Synthesis of Compound m-5

A 200-ml recovery flask was charged with the following reagent(s) and solvent(s).

Compound m-3: 2.5 g (7.3 mmol)
Compound m-4: 1.8 g (8.0 mmol)
$Pd(PPh_3)_4$: 0.08 g
Toluene: 25 ml
Ethanol: 13 ml
2M-sodium carbonate aqueous solution: 20 ml

The reaction solution was then heated and stirred under reflux in a nitrogen stream for 6 hours. After completion of the reaction, water was added to the product for separation. The product was dissolved in chloroform, was purified by column chromatography (chloroform:heptane), and was recrystallized in toluene/heptane. Thus, 4.0 g (yield: 82%) of a compound m-5 as a white solid was produced.

(3) Synthesis of Compound A2

A 200-ml recovery flask was charged with the following reagent(s) and solvent(s).

Compound m-5: 2.0 g (4.5 mmol)
Compound m-6: 1.4 g (5.0 mmol)
$Pd(dba)_2$: 0.52 g
SPhos: 0.74 g
Potassium phosphate: 2.88 g
Toluene: 100 ml
$H_2O$: 10 ml

The reaction solution was then heated and stirred under reflux in a nitrogen stream for 6 hours. After completion of the reaction, water was added to the product for separation. The product was dissolved in chloroform, was purified by column chromatography (chloroform:heptane), and was recrystallized in toluene/heptane. Thus, 5.2 g (yield: 74%) of an exemplary compound A2 as a white solid was produced.

The exemplary compound A25 was subjected to mass spectrometry with MALDI-TOF-MS (Autoflex LRF manufactured by Bruker).

[MALDI-TOF-MS]

Measured value: $m/z = 632$ Calculated value: $C_{90}H_{32} = 632$

[Exemplary Embodiments 2 to 20 (Synthesis of Exemplary Compounds)]

As shown in Tables 6 and 7, exemplary compounds of Exemplary Embodiments 2 to 20 were synthesized in the same manner as in Exemplary Embodiment 1 except that the raw materials m-4 and m-6 of Exemplary Embodiment 1 were changed. Actual values m/z measured by mass spectrometry in the same manner as in Exemplary Embodiment 1 are also shown.

[Table 6]

| Exemplary embodiment | Exemplary compound | Raw material m-4 | Raw material m-6 | m/z |
|---|---|---|---|---|
| 2 | A3 | 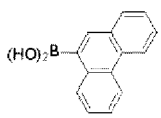 | 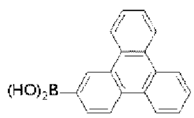 | 632 |
| 3 | A4 | 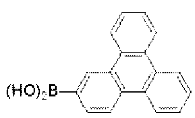 | 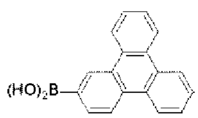 | 683 |
| 4 | A9 | 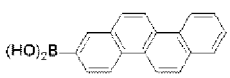 | 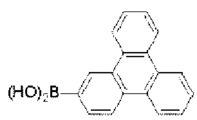 | 683 |

(continued)

| Exemplary embodiment | Exemplary compound | Raw material m-4 | Raw material m-6 | m/z |
|---|---|---|---|---|
| 5 | A11 | 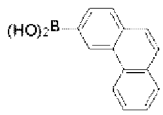 | 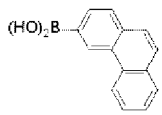 | 583 |
| 6 | A12 | 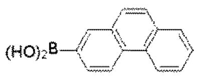 | 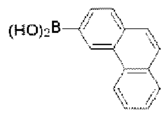 | 583 |
| 7 | A15 | 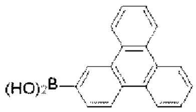 | 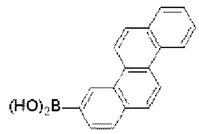 | 583 |
| 8 | A37 | 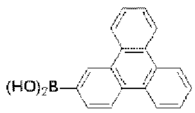 | 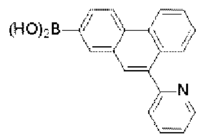 | 710 |
| 9 | A38 | 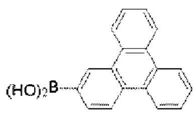 | 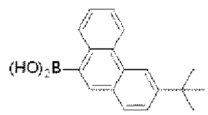 | 689 |
| 10 | B11 | 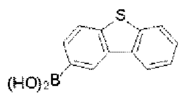 | 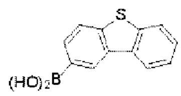 | 595 |
| 11 | B15 | 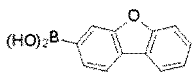 | 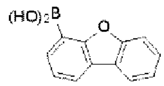 | 563 |

[Table 7]

| Exemplary embodiment | Exemplary compound | Raw material m-4 | Raw material m-6 | m/z |
|---|---|---|---|---|
| 12 | B18 | 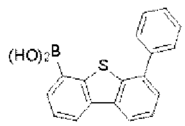 | 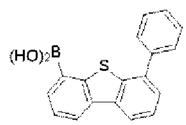 | 747 |
| 13 | B27 | 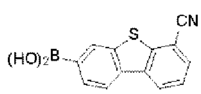 | 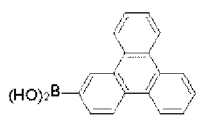 | 664 |
| 14 | C1 | 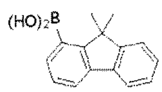 | 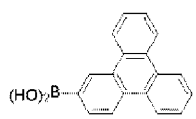 | 649 |
| 15 | C10 | 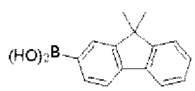 | 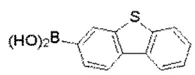 | 605 |

(continued)

| Exemplary embodiment | Exemplary compound | Raw material m-4 | Raw material m-6 | m/z |
|---|---|---|---|---|
| 16 | C16 | 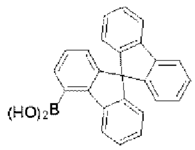 | 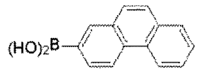 | 721 |
| 17 | C33 | 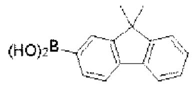 | 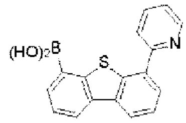 | 682 |
| 18 | D1 | 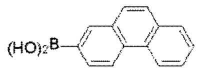 | 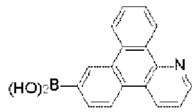 | 634 |
| 19 | D2 | 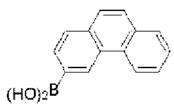 | 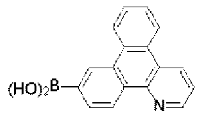 | 634 |
| 20 | D12 | 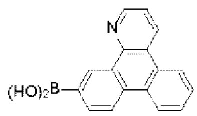 | 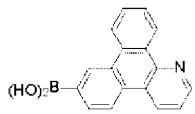 | 685 |

[Comparative Examples 1 and 2 (Synthesis of the Comparative Compounds)]

As shown in Table 8, comparative compounds of Comparative Examples 1 and 2 were synthesized in the same manner as in Exemplary Embodiment 1 except that the raw materials m-1, m-2, m-4, and m-6 of Exemplary Embodiment 1 were changed. Actual values m/z measured by mass spectrometry in the same manner as in Exemplary Embodiment 1 are also shown.

[Table 8]

| Comparative example | Comparative compound | Raw material m-1 | Raw material m-2 | Raw material m-4 | Raw material m-6 | m/z |
|---|---|---|---|---|---|---|
| 1 | 1-A | 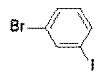 | 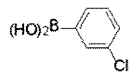 | 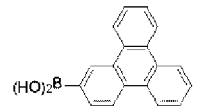 | 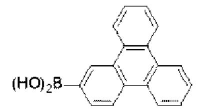 | 607 |
| 2 | 1-B | 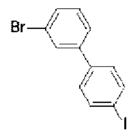 | 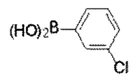 | 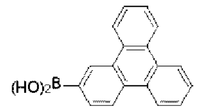 | 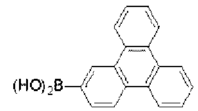 | 683 |

[Exemplary Embodiment 21]

An organic light-emitting element of a bottom emission type was produced. The organic light-emitting element included a positive electrode, a hole injection layer, a hole transport layer, an electron-blocking layer, a light-emitting layer, a hole-blocking layer, an electron transport layer, an electron injection layer, and a negative electrode sequentially formed on a substrate.

First, an ITO film was formed on a glass substrate and was subjected to desired patterning to form an ITO electrode (positive electrode). The ITO electrode had a thickness of 100 nm. The substrate on which the ITO electrode was formed was used as an ITO substrate in the following process. Vacuum evaporation was then performed by resistance heating in a vacuum chamber at $1.33 \times 10^{-4}$ Pa to continuously form an organic compound layer and an electrode layer shown in Table 9 on the ITO substrate. The counter electrode (a metal electrode layer, a negative electrode) had an electrode area of 3 $mm^2$.

[Table 9]

| | Raw material | | | Film thickness (nm) |
|---|---|---|---|---|
| Negative electrode | A1 | | | 100 |
| Electron injection layer (EIL) | LiF | | | 1 |
| Electron transport layer (ETL) | ET2 | | | 20 |
| Hole-blocking layer (HBL) | ET11 | | | 20 |
| Light-emitting layer (EML) | Host | A4 | Weight ratio A4:AA2 =90:10 | 20 |
| | Guest | AA2 | | |
| Electron-blocking layer (HBL) | HT19 | | | 15 |
| Hole transport layer (HTL) | HT3 | | | 30 |
| Hole injection layer (HIL) | HT16 | | | 5 |

The characteristics of the element were measured and evaluated. The light-emitting element had a maximum emission wavelength of 522 nm and a maximum external quantum efficiency (E.Q.E.) of 13%. A continuous operation test was performed at a current density of 100 mA/$cm^2$ to measure the time (LT95) when the luminance degradation rate reached 5%. The time (LT95) at which the luminance decay rate of Comparative Example 1 reached 5% is 1.0. The ratio of the time (LT95) at which the luminance decay rate in the present exemplary embodiment reached 5% was 1.4.

In the present exemplary embodiment, with respect to measuring apparatuses, more specifically, the current-voltage characteristics were measured with a microammeter 4140B manufactured by Hewlett-Packard Co., and the luminous brightness was measured with BM7 manufactured by Topcon Corporation.

[Exemplary Embodiments 22 to 41 and Comparative Examples 3 and 4]

Organic light-emitting elements were produced in the same manner as in Exemplary Embodiment 21 except that the materials forming each layer were appropriately changed to the compounds shown in Table 10. A layer not shown in Table 10 had the same structure as in Exemplary Embodiment 21. Characteristics of the elements were measured and evaluated in the same manner as in Exemplary Embodiment 21. Table 10 shows the measurement results together with the results of Exemplary Embodiment 21.

[Table 10]

| | HIL | HTL | EBL | EML | | HBL | ETL | E.Q.E [%] | Luminance decay rate |
|---|---|---|---|---|---|---|---|---|---|
| | | | | Host | Guest | | | | |
| Exemplary embodiment 22 | HT16 | HT3 | HT19 | A1 | AA26 | ET12 | ET15 | 13 | 1.5 |
| Exemplary embodiment 23 | HT16 | HT2 | HT15 | A4 | AA27 | ET12 | ET2 | 14 | 1.4 |

(continued)

| | HIL | HTL | EBL | EML | | HBL | ETL | E.Q.E [%] | Luminance decay rate |
|---|---|---|---|---|---|---|---|---|---|
| | | | | Host | Guest | | | | |
| Exemplary embodiment 24 | HT16 | HT2 | HT15 | A4 | AA30 | ET11 | ET2 | 13 | 1.4 |
| Exemplary embodiment 25 | HT16 | HT3 | HT19 | A3 | CC17 | ET11 | ET2 | 13 | 1.1 |
| Exemplary embodiment 26 | HT16 | HT3 | HT19 | A14 | GD10 | ET11 | ET2 | 10 | 1.1 |
| Exemplary embodiment 27 | HT16 | HT3 | HT19 | A15 | HH1 | ET11 | ET15 | 12 | 1.4 |
| Exemplary embodiment 28 | HT16 | HT3 | HT19 | B1 | BB24 | ET12 | ET2 | 13 | 1.5 |
| Exemplary embodiment 29 | HT16 | HT2 | HT15 | B2 | BB25 | ET12 | ET15 | 13 | 1.4 |
| Exemplary embodiment 30 | HT16 | HT3 | HT19 | B3 | GD10 | ET12 | ET15 | 11 | 1.1 |
| Exemplary embodiment 31 | HT16 | HT2 | HT15 | B9 | DD5 | ET11 | ET2 | 13 | 1.4 |
| Exemplary embodiment 32 | HT16 | HT3 | HT19 | B12 | DD31 | ET12 | ET15 | 14 | 1.3 |
| Exemplary embodiment 33 | HT16 | HT2 | HT15 | C1 | DD27 | ET12 | ET2 | 13 | 1.2 |
| Exemplary embodiment 34 | HT16 | HT2 | HT15 | C2 | EE1 | ET11 | ET2 | 14 | 1.2 |
| Exemplary embodiment 35 | HT16 | HT3 | HT19 | C3 | EE2 | ET12 | ET15 | 15 | 1.2 |
| Exemplary embodiment 36 | HT16 | HT3 | HT19 | C14 | FF29 | ET12 | ET15 | 14 | 1.2 |
| Exemplary embodiment 37 | HT16 | HT3 | HT19 | C15 | H21 | ET11 | ET15 | 13 | 1.2 |
| Exemplary embodiment 38 | HT16 | HT3 | HT19 | D1 | FF1 | ET12 | ET15 | 12 | 1.2 |
| Exemplary embodiment 39 | HT16 | HT3 | HT19 | D2 | GG4 | ET11 | ET15 | 13 | 1.2 |
| Exemplary embodiment 40 | HT16 | HT3 | HT19 | D18 | HH27 | ET12 | ET2 | 13 | 1.2 |
| Exemplary embodiment 41 | HT16 | HT2 | HT15 | D21 | II2 | ET12 | ET15 | 13 | 1.2 |
| Comparative example 3 | HT16 | HT3 | HT19 | Comparative compound 1-A | GD10 | ET11 | ET2 | 9 | 1.0 |
| Comparative example 4 | HT16 | HT3 | HT19 | Comparative compound 1-B | GD10 | ET11 | ET2 | 8 | 0.9 |

Table 10 shows that Comparative Examples 3 and 4 had a maximum external quantum efficiency (E.Q.E.) of 8% and 9%, respectively, and Exemplary Embodiments 21 to 41 had a maximum external quantum efficiency in the range of 10% to 15%. Thus, the organic light-emitting elements of Exemplary Embodiments 21 to 41 had higher light

emission efficiency. This is probably because each compound contained as a host in the light-emitting layer of the organic light-emitting elements of Exemplary Embodiments 21 to 41 has a lower ΔST than the organic compounds (the comparative compounds 1-A and 1-B) contained as a host in the light-emitting layer of the organic light-emitting elements of Comparative Examples 3 and 4. More specifically, the hosts of Exemplary Embodiments 21 to 41 have a structure with two fused rings linked at the positions 4 and 3" of the linking group 1,1':3',1"-terphenyl and therefore have a lower $S_1$ energy level while maintaining a high $T_1$ energy level. Thus, it is thought that the hosts of Exemplary Embodiments 21 to 41 and the hosts of Comparative Examples 3 and 4 have almost the same $T_1$ energy level and therefore provide almost the same quantum efficiency. On the other hand, the hosts of Exemplary Embodiments 21 to 41 have a lower $S_1$ energy level than the hosts of Comparative Examples 3 and 4, and Exemplary Embodiments 21 to 41 therefore have a lower drive voltage than Comparative Examples 3 and 4. Consequently, Exemplary Embodiments 21 to 41 had higher maximum external quantum efficiency (E.Q.E.) than Comparative Examples 3 and 4.

Furthermore, the organic light-emitting elements of Exemplary Embodiments 21 to 41 had a longer life than the organic light-emitting elements of Comparative Examples 3 and 4. This is probably because each compound contained as a host in the light-emitting layer of the organic light-emitting elements of Exemplary Embodiments 21 to 41 has higher amorphousness and sublimability than the organic compounds (the comparative compounds 1-A and 1-B) contained as a host in the light-emitting layer of the organic light-emitting elements of Comparative Examples 3 and 4.

When an organic compound represented by the general formula [1] was used as a host, and when a phosphorescent material with a tricyclic or higher polycyclic fused ring in a ligand thereof was used as a guest, a light-emitting element with particularly high efficiency and a long life could be provided. More specifically, a light-emitting element with particularly high efficiency and a long life could be provided when a phosphorescent element with a structure selected from a triphenylene structure, a phenanthrene structure, a benzofluorene structure, a dibenzofuran structure, a dibenzothiophene structure, a benzoisoquinoline structure, and a naphthoisoquinoline structure was used as a guest.

Thus, it was found that a compound represented by the general formula [1] can be used to provide an element with high efficiency and element durability.

[Exemplary Embodiment 42]

An organic light-emitting element was produced in the same manner as in Exemplary Embodiment 21 except that the organic compound layer and the electrode layer shown in Table 11 were continuously formed.

[Table 11]

| | Raw material | | | Film thickness (nm) |
|---|---|---|---|---|
| Negative electrode | A1 | | | 100 |
| Electron injection layer (EIL) | LiF | | | 1 |
| Electron transport layer (ETL) | ET2 | | | 20 |
| Hole-blocking layer (HBL) | ET11 | | | 20 |
| Light-emitting layer (EML) | Host | A4 | Weight ratio A4:AA22:EM30 =60:10:30 | 20 |
| | Guest | AA22 | | |
| | Assist | EM30 | | |
| Electron-blocking layer (HBL) | HT19 | | | 15 |
| Hole transport layer (HTL) | HT3 | | | 30 |
| Hole injection layer (HIL) | HT16 | | | 5 |

The characteristics of the element were measured and evaluated. The light-emitting element had a green emission color and a maximum external quantum efficiency (E.Q.E.) of 19%.

[Exemplary Embodiments 43 to 67]

Organic light-emitting elements were produced in the same manner as in Exemplary Embodiment 42 except that the materials forming each layer were appropriately changed to the compounds shown in Table 12. A layer not shown in Table 12 had the same structure as in Exemplary Embodiment 42. Characteristics of the elements were measured and evaluated in the same manner as in Exemplary Embodiment 42. Table 12 shows the measurement results.

[Table 12]

| | HIL | HTL | EBL | EML | | | HBL | ETL | E.Q.E [%] |
|---|---|---|---|---|---|---|---|---|---|
| | | | | Host | Guest | Assist | | | |
| Exemplary embodiment 42 | HT16 | HT3 | HT19 | A4 | AA22 | EM30 | ET11 | ET2 | 19 |
| Exemplary embodiment 43 | HT16 | HT3 | HT19 | B19 | BB24 | EM29 | ET26 | ET3 | 17 |
| Exemplary embodiment 44 | HT16 | HT2 | HT15 | B18 | BB21 | EM35 | ET13 | ET2 | 16 |
| Exemplary embodiment 45 | HT16 | HT2 | HT15 | C19 | CC17 | EM37 | ET13 | ET2 | 17 |
| Exemplary embodiment 46 | HT16 | HT3 | HT19 | C18 | DD5 | EM13 | ET16 | ET15 | 15 |
| Exemplary embodiment 47 | HT16 | HT3 | HT19 | A7 | DD31 | EM30 | ET16 | ET15 | 18 |
| Exemplary embodiment 48 | HT16 | HT3 | HT19 | B28 | DD27 | EM28 | ET17 | ET15 | 16 |
| Exemplary embodiment 49 | HT16 | HT3 | HT19 | A21 | EE1 | EM39 | ET13 | ET2 | 15 |
| Exemplary embodiment 50 | HT16 | HT2 | HT15 | A8 | EE2 | GD10 | ET15 | ET3 | 14 |
| Exemplary embodiment 51 | HT16 | HT3 | HT19 | A5 | FF2 | ET15 | ET15 | ET15 | 16 |
| Exemplary embodiment 52 | HT16 | HT2 | HT15 | A4 | FF23 | ET16 | ET2 | ET2 | 17 |
| Exemplary embodiment 53 | HT16 | HT3 | HT19 | A4 | FF1 | EM16 | ET26 | ET3 | 13 |
| Exemplary embodiment 54 | HT16 | HT2 | HT15 | B6 | GG3 | EM16 | ET13 | ET2 | 13 |
| Exemplary embodiment 55 | HT16 | HT2 | HT15 | B1 | HH25 | EM34 | ET13 | ET2 | 14 |
| Exemplary embodiment 56 | HT16 | HT3 | HT19 | C18 | GG21 | EM35 | ET16 | ET15 | 17 |
| Exemplary embodiment 57 | HT16 | HT3 | HT19 | A7 | DD46 | EM37 | ET16 | ET15 | 16 |
| Exemplary embodiment 58 | HT16 | HT2 | HT15 | B4 | DD35 | EM13 | ET15 | ET3 | 14 |
| Exemplary embodiment 59 | HT16 | HT3 | HT19 | B2 | DD31 | EM30 | ET15 | ET15 | 19 |
| Exemplary embodiment 60 | HT16 | HT2 | HT15 | B3 | DD9 | EM28 | ET2 | ET2 | 18 |
| Exemplary embodiment 61 | HT16 | HT3 | HT19 | B9 | DD8 | EM28 | ET26 | ET3 | 18 |
| Exemplary embodiment 62 | HT16 | HT2 | HT15 | B5 | DD3 | EM29 | ET13 | ET2 | 15 |
| Exemplary embodiment 63 | HT16 | HT3 | HT19 | D1 | AA6 | EM30 | ET26 | ET3 | 16 |
| Exemplary embodiment 64 | HT16 | HT2 | HT15 | D15 | HH7 | AA6 | ET13 | ET2 | 17 |
| Exemplary embodiment 65 | HT16 | HT2 | HT15 | C3 | HH25 | EM30 | ET13 | ET2 | 18 |
| Exemplary embodiment 66 | HT16 | HT3 | HT19 | C2 | II2 | GD12 | D60 | ET15 | 16 |
| Exemplary embodiment 67 | HT16 | HT3 | HT19 | B16 | II3 | GD10 | D54 | ET15 | 16 |

Thus, an organic light-emitting element with high light emission efficiency could be realized by using an organic compound represented by the general formula [1] as a host of a light-emitting layer and further using a material with a carbazole structure, an azine ring, or a xanthone structure as an assist material.

The present invention is not limited to these embodiments, and various changes and modifications may be made therein without departing from the spirit and scope of the present invention. Accordingly, the following claims are attached to make the scope of the present invention public.

This application claims the benefit of Japanese Patent Application No. 2021-184897 filed November 12, 2021, which is hereby incorporated by reference herein in its entirety.

Reference Signs List

1 organic light-emitting element

11 substrate
21 positive electrode
22 organic compound layer
23 negative electrode

## Claims

**1.** An organic compound represented by the following general formula [1].

[Chem. 1]

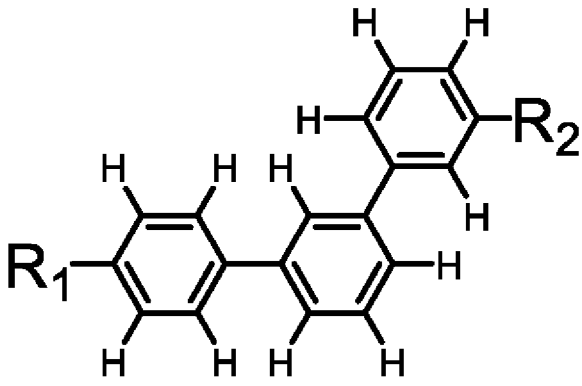

[1]

(In the general formula [1], $R_1$ and $R_2$ are each independently selected from the following substituent A group.
(Substituent A Group)

[Chem. 2]

A: 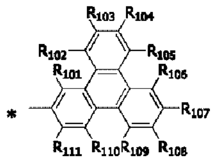 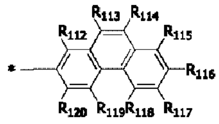

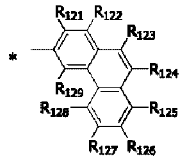 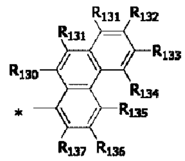 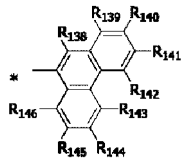 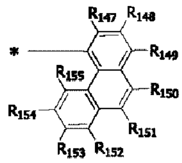

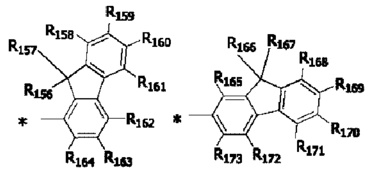 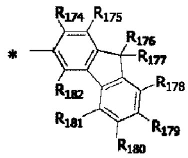 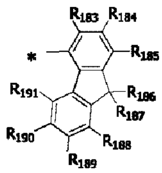

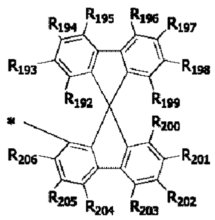 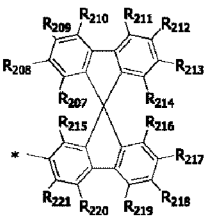 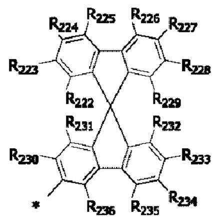 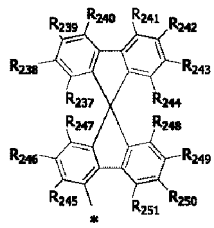

[Chem. 3]

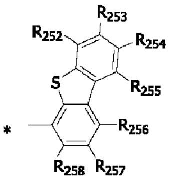
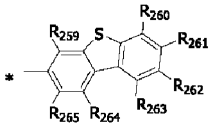
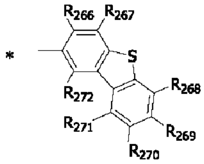
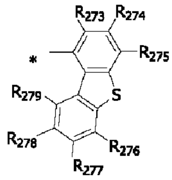

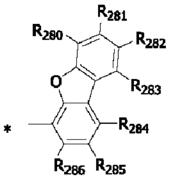
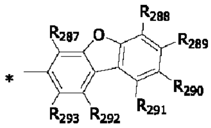
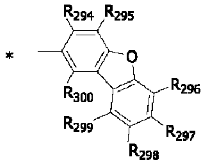
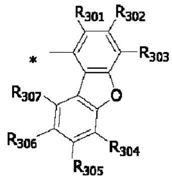

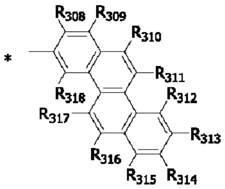
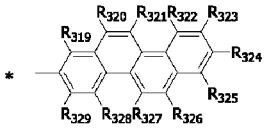
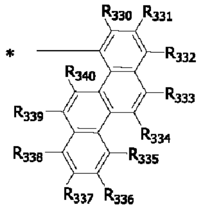
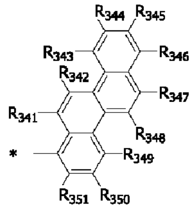

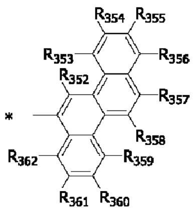

[Chem. 4]

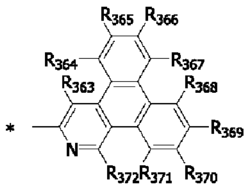
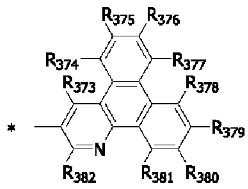
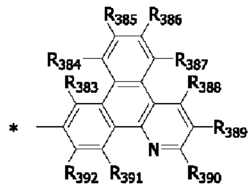

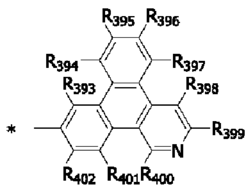
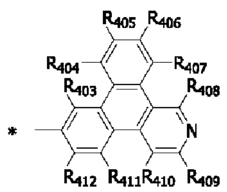
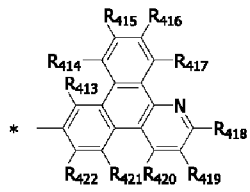

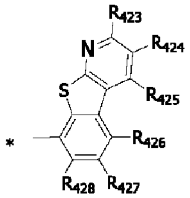
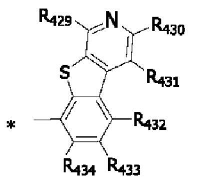
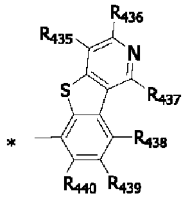
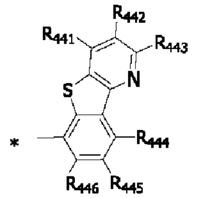

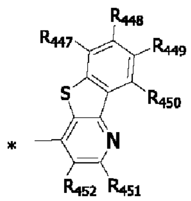
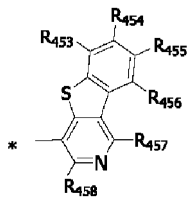
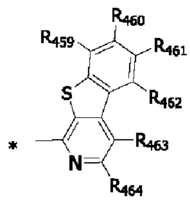

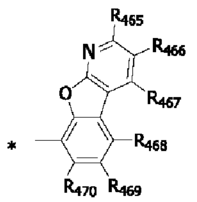
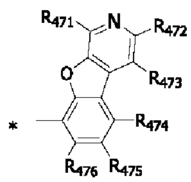
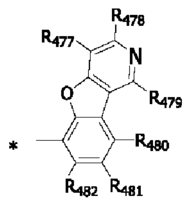
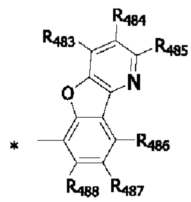

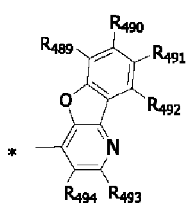
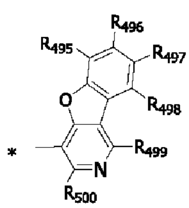
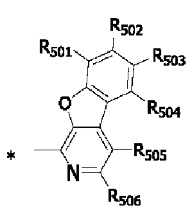

In the substituent A group, $R_{101}$ to $R_{506}$ are each independently selected from a hydrogen atom, a deuterium atom, a halogen atom, a substituted or unsubstituted alkyl group, a substituted or unsubstituted alkoxy group, a substituted or unsubstituted amino group, an aryloxy group, a silyl group, an aromatic hydrocarbon group, a heterocyclic group, and a cyano group.)

**2.** The organic compound according to Claim 1, wherein at least one of $R_1$ and $R_2$ in the general formula [1] is each independently selected from the following substituent B group.
(Substituent B Group)

[Chem. 5]

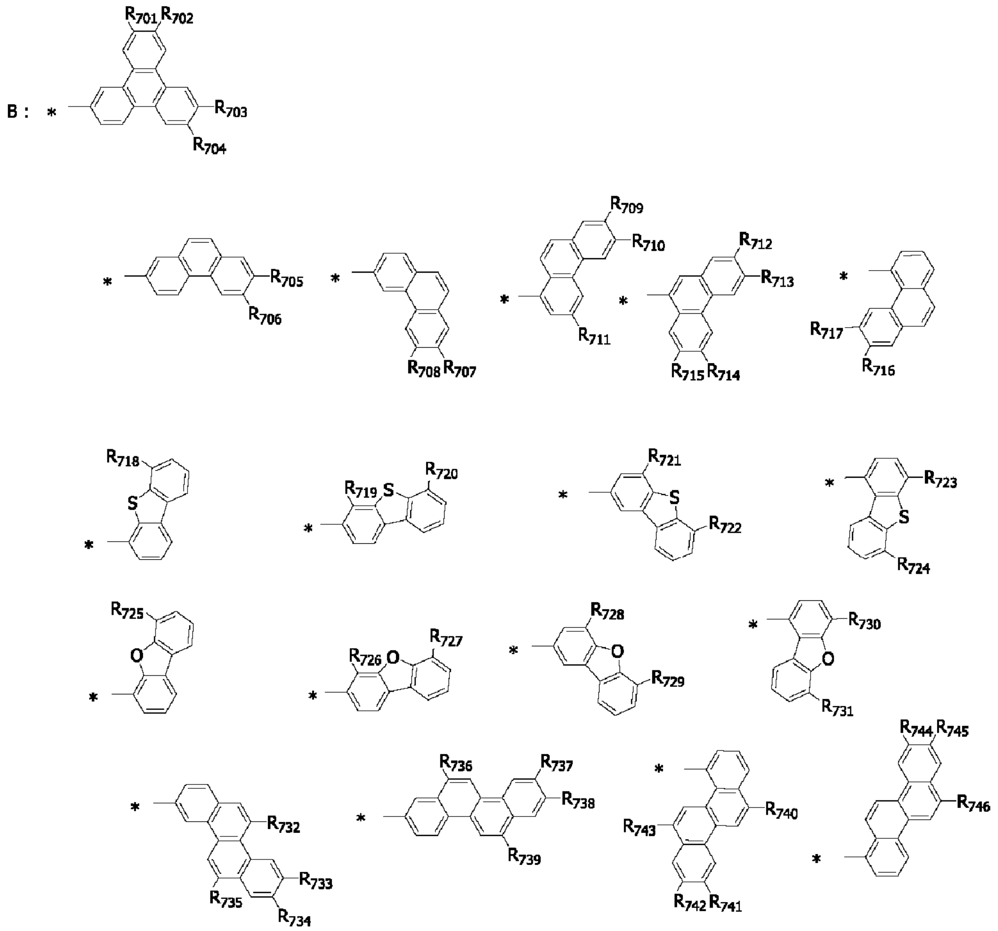

In the substituent B group, $R_{701}$ to $R_{746}$ are each independently selected from a hydrogen atom, a deuterium atom, a halogen atom, a substituted or unsubstituted alkyl group, a substituted or unsubstituted alkoxy group, a substituted or unsubstituted amino group, an aryloxy group, a silyl group, an aromatic hydrocarbon group, a heterocyclic group, and a cyano group.)

**3.** The organic compound according to Claim 1, wherein at least one of $R_1$ and $R_2$ in the general formula [1] is each independently selected from the following substituent C group.
(Substituent C Group)

[Chem. 6]

C : 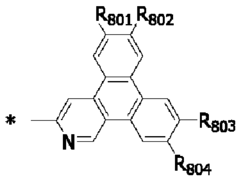 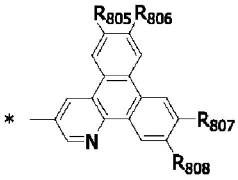 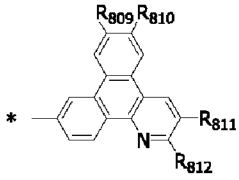

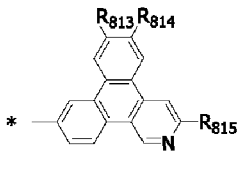 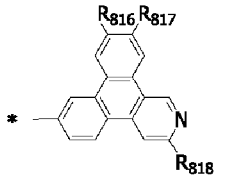 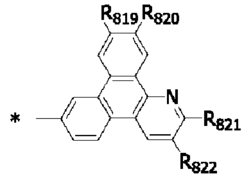

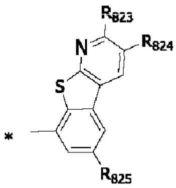 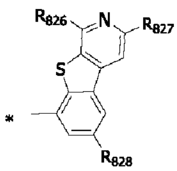 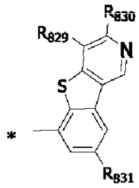 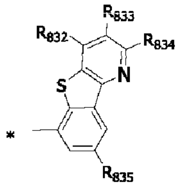

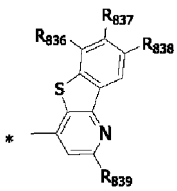 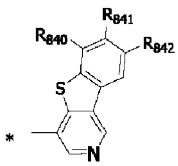 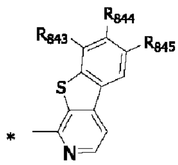

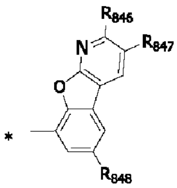 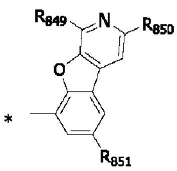 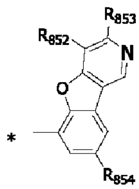 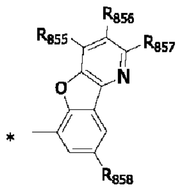

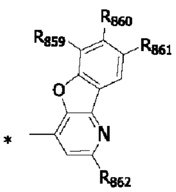 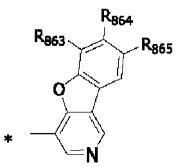 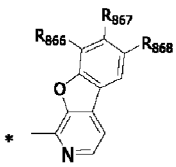

In the substituent C group, $R_{801}$ to $R_{868}$ are each independently selected from a hydrogen atom, a deuterium atom, a halogen atom, a substituted or unsubstituted alkyl group, a substituted or unsubstituted alkoxy group, a substituted or unsubstituted amino group, an aryloxy group, a silyl group, an aromatic hydrocarbon group, a heterocyclic group, and a cyano group.)

**4.** The organic compound according to Claim 1, wherein at least one of $R_1$ and $R_2$ in the general formula [1] is each independently selected from the following substituent D group.
(Substituent D Group)

[Chem. 7]

D : 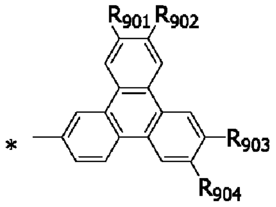 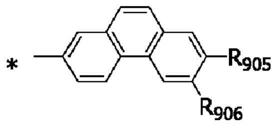 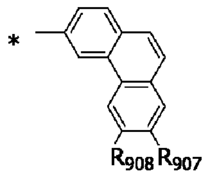

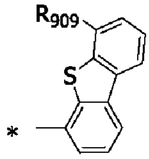 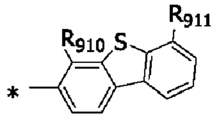 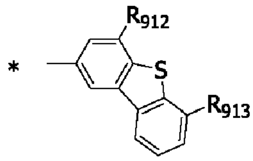

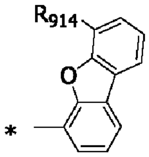 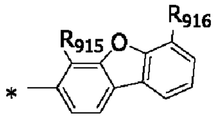 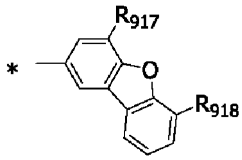

In the substituent D group, $R_{901}$ to $R_{918}$ are each independently selected from a hydrogen atom, a deuterium atom, a halogen atom, a substituted or unsubstituted alkyl group, a substituted or unsubstituted alkoxy group, a substituted or unsubstituted amino group, an aryloxy group, a silyl group, an aromatic hydrocarbon group, a heterocyclic group, and a cyano group.)

**5.** An organic light-emitting element comprising:

a positive electrode and a negative electrode; and
an organic compound layer located between the positive electrode and the negative electrode and having at least a light-emitting layer,
wherein the organic compound layer contains the organic compound according to any one of Claims 1 to 4.

**6.** The organic light-emitting element according to Claim 5, wherein the light-emitting layer contains the organic compound.

**7.** The organic light-emitting element according to Claim 6,

wherein the light-emitting layer further contains a phosphorescent material, and
the organic compound in the light-emitting layer has a concentration of 30% by mass or more and 99% by mass or less.

**8.** The organic light-emitting element according to Claim 7, wherein the phosphorescent material is an organometallic complex and has a tricyclic or higher polycyclic fused ring in a ligand thereof.

**9.** The organic light-emitting element according to Claim 8, wherein the tricyclic or higher polycyclic fused ring has at least one structure selected from a triphenylene structure, a phenanthrene structure, a benzofluorene structure, a dibenzofuran structure, a dibenzothiophene structure, a benzoisoquinoline structure, and a naphthoisoquinoline structure.

**10.** The organic light-emitting element according to any one of Claims 6 to 9, wherein the light-emitting layer further contains a phosphorescent material, and a third compound different from the organic compound and the phosphorescent material.

**11.** The organic light-emitting element according to Claim 10, wherein the third compound has a carbazole skeleton.

**12.** The organic light-emitting element according to Claim 10 or 11, wherein the third compound has an azine ring in a skeleton thereof.

**13.** The organic light-emitting element according to any one of Claims 10 to 12, wherein the third compound has a xanthone structure in a skeleton thereof.

**14.** The organic light-emitting element according to any one of Claims 5 to 13,

wherein the light-emitting layer is a first light-emitting layer,
a second light-emitting layer different from the first light-emitting layer is further provided between the first light-emitting layer and the positive electrode or between the first light-emitting layer and the negative electrode, and the second light-emitting layer emits light of a different color from light emitted by the first light-emitting layer.

**15.** The organic light-emitting element according to Claim 14, which emits white light.

**16.** A display apparatus comprising a plurality of pixels,
wherein at least one of the plurality of pixels includes the organic light-emitting element according to any one of Claims 5 to 15 and an active element coupled to the organic light-emitting element.

**17.** The display apparatus according to Claim 16, further comprising a color filter.

**18.** A photoelectric conversion apparatus comprising:

an optical unit including a plurality of lenses; an imaging element configured to receive light passing through the optical unit; and
a display unit configured to display an image taken by the imaging element,
wherein the display unit includes the organic light-emitting element according to any one of Claims 5 to 15.

**19.** Electronic equipment comprising:

a housing;
a communication unit configured to communicate with the outside; and
a display unit,
wherein the display unit includes the organic light-emitting element according to any one of Claims 5 to 15.

**20.** A lighting apparatus comprising:

a light source; and
a light-diffusing unit or an optical filter,
wherein the light source includes the organic light-emitting element according to any one of Claims 5 to 15.

**21.** A moving body comprising:

a body; and
a lamp provided on the body,
wherein the lamp includes the organic light-emitting element according to any one of Claims 5 to 15.

**22.** An exposure light source for an electrophotographic image-forming apparatus, comprising the organic light-emitting element according to any one of Claims 5 to 15.

FIG. 1A

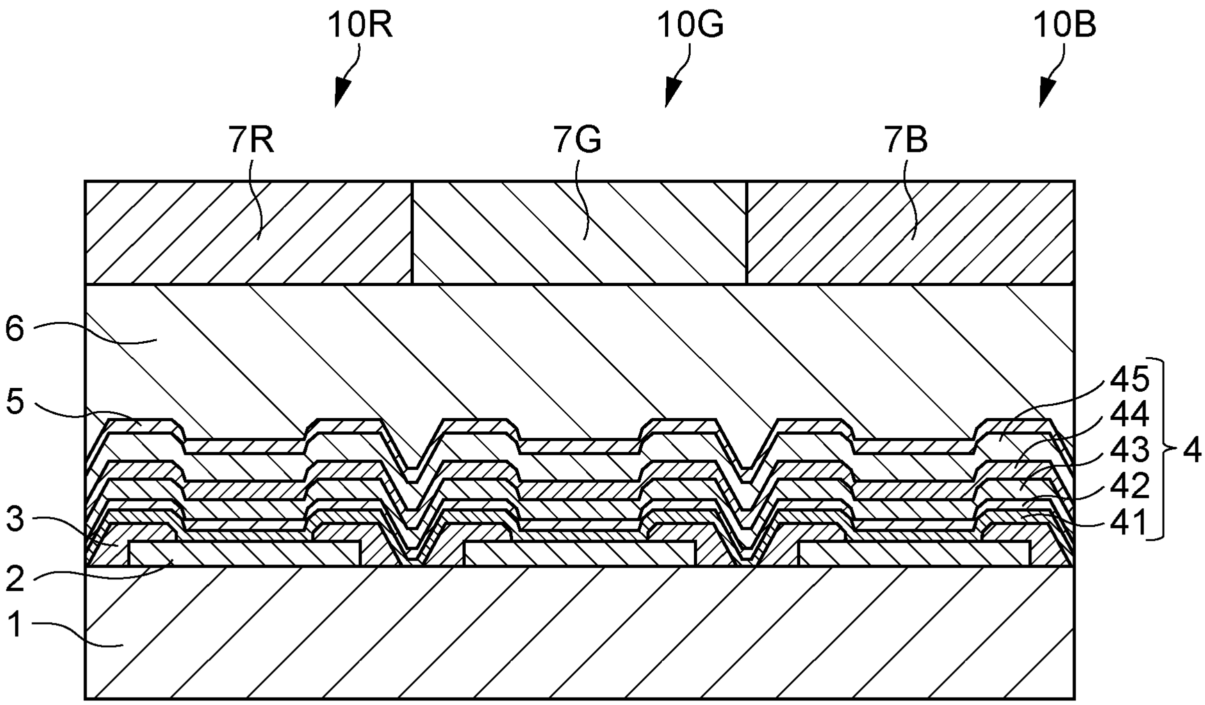
10R
10G
10B
7R
7G
7B
6
5
3
2
1
45
44
43
42
41
4

FIG. 1B

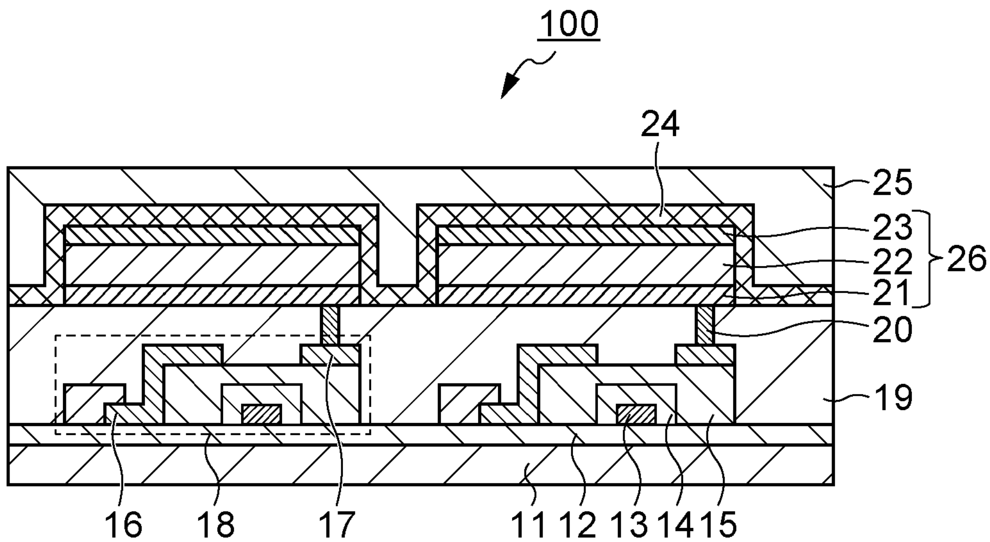
100
24
25
23
22
21
26
20
19
16
18
17
11
12
13
14
15

FIG. 2

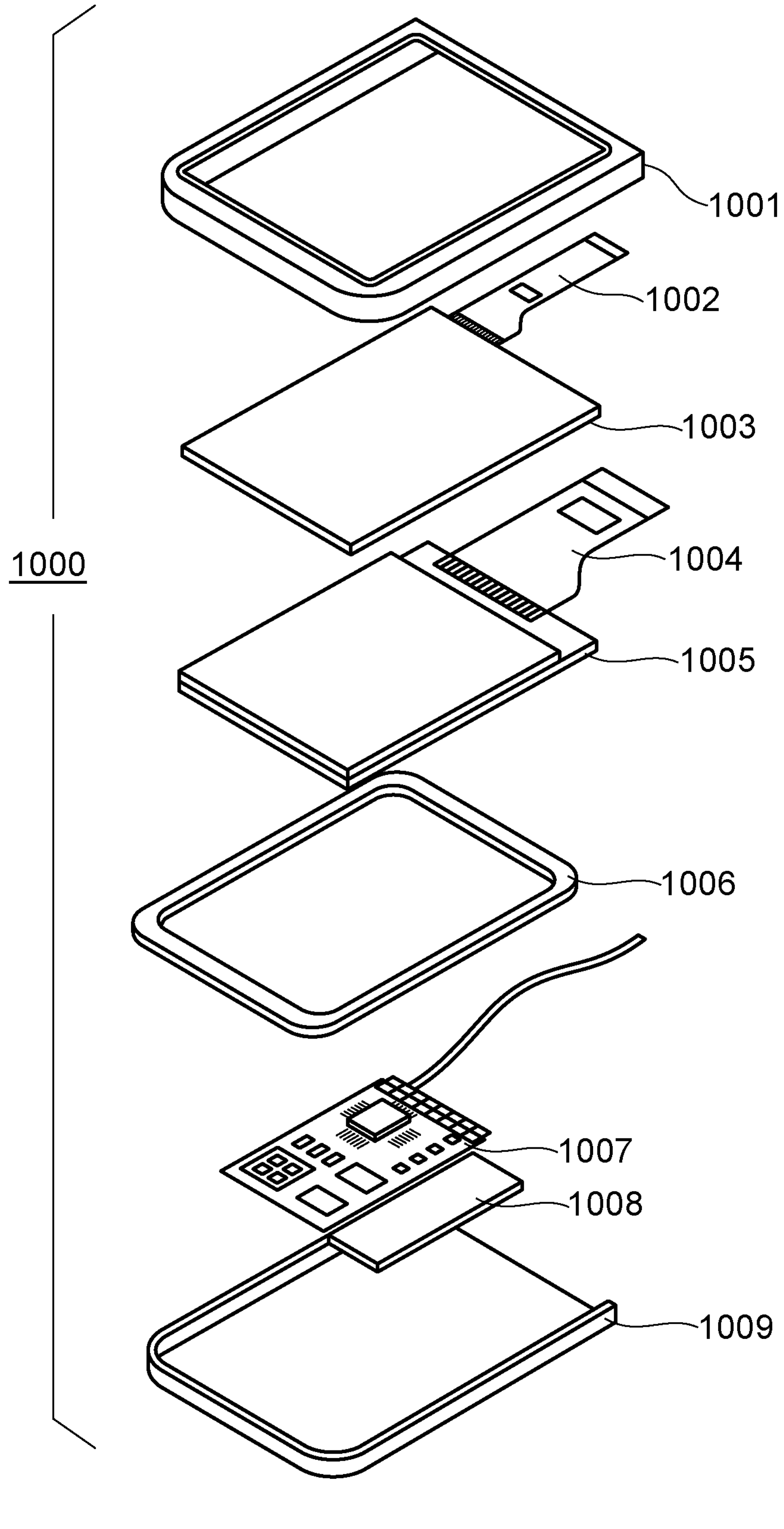

FIG. 3A

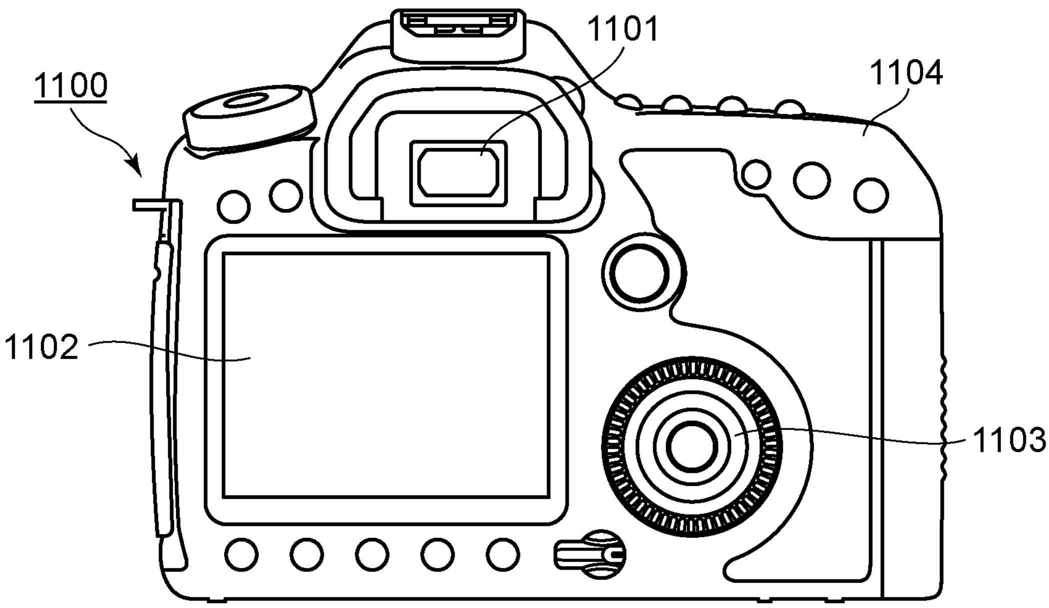


FIG. 3B

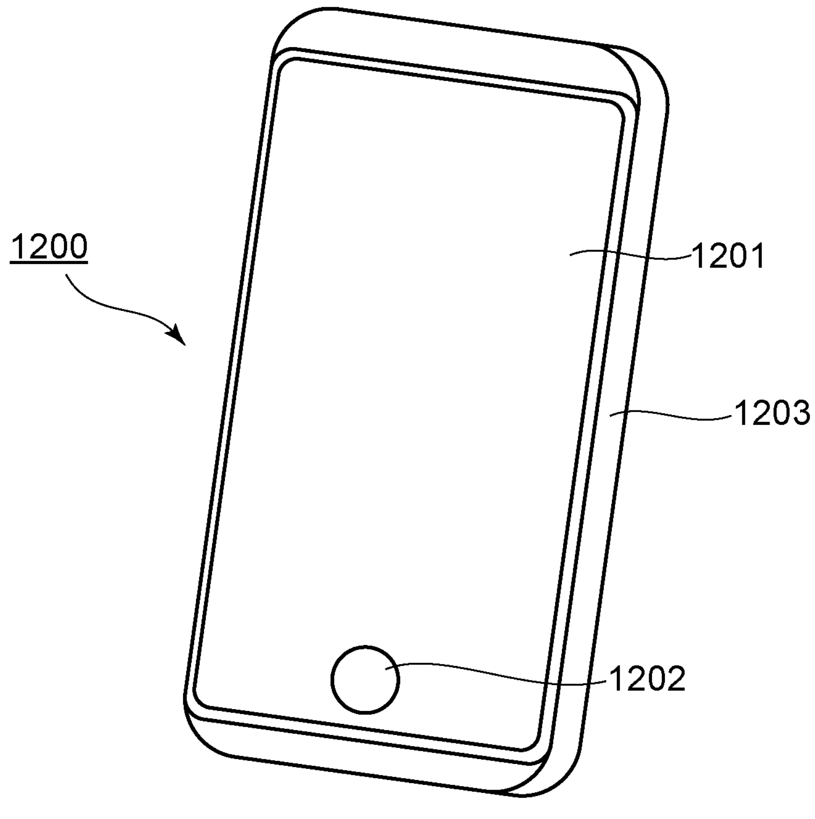

FIG. 4A

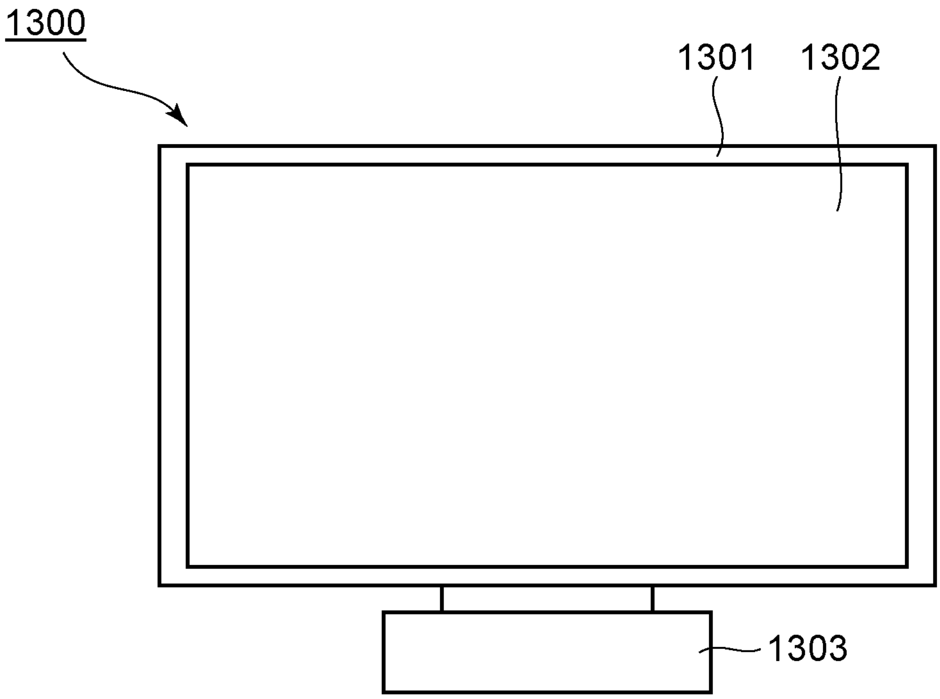


FIG. 4B

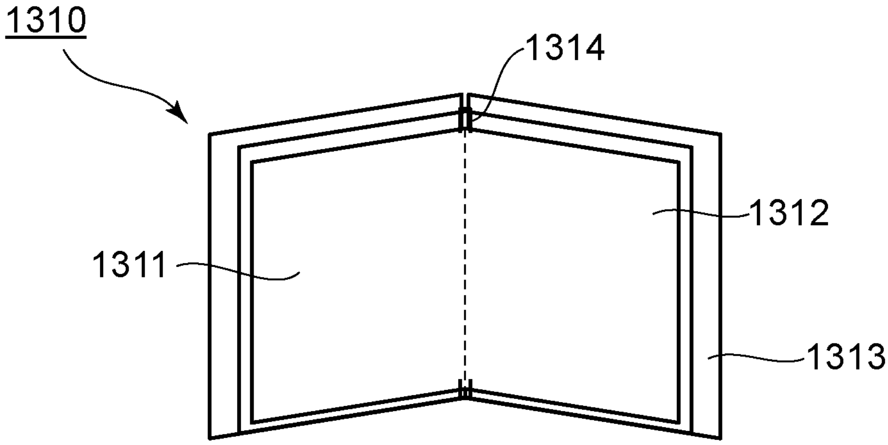

FIG. 5A

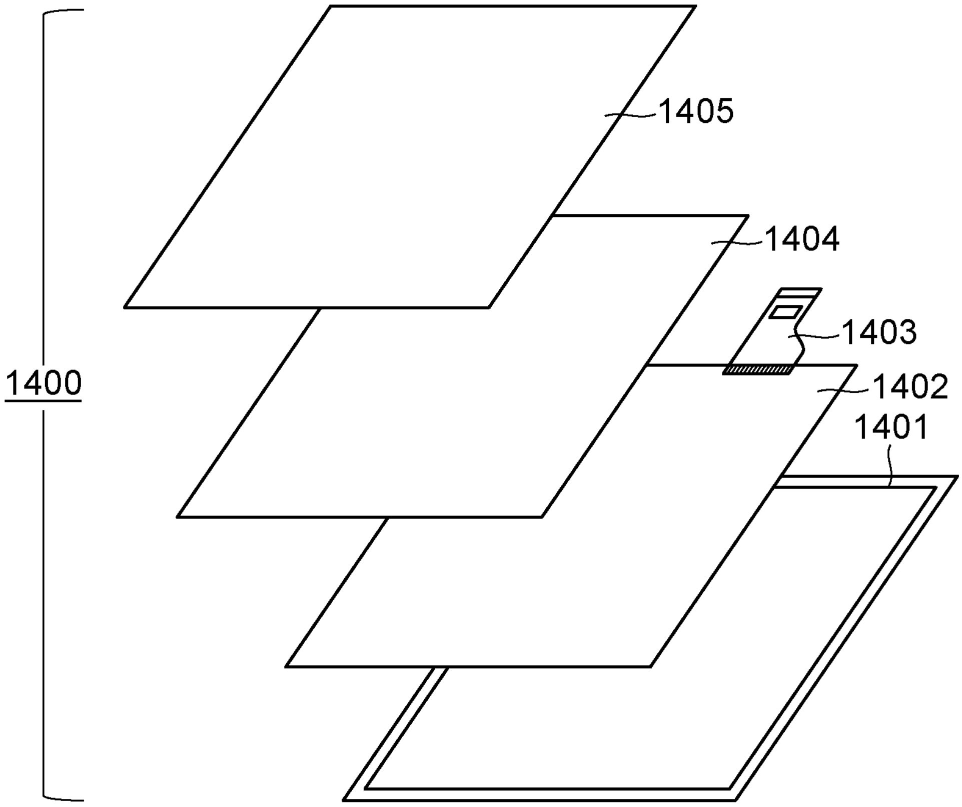


FIG. 5B

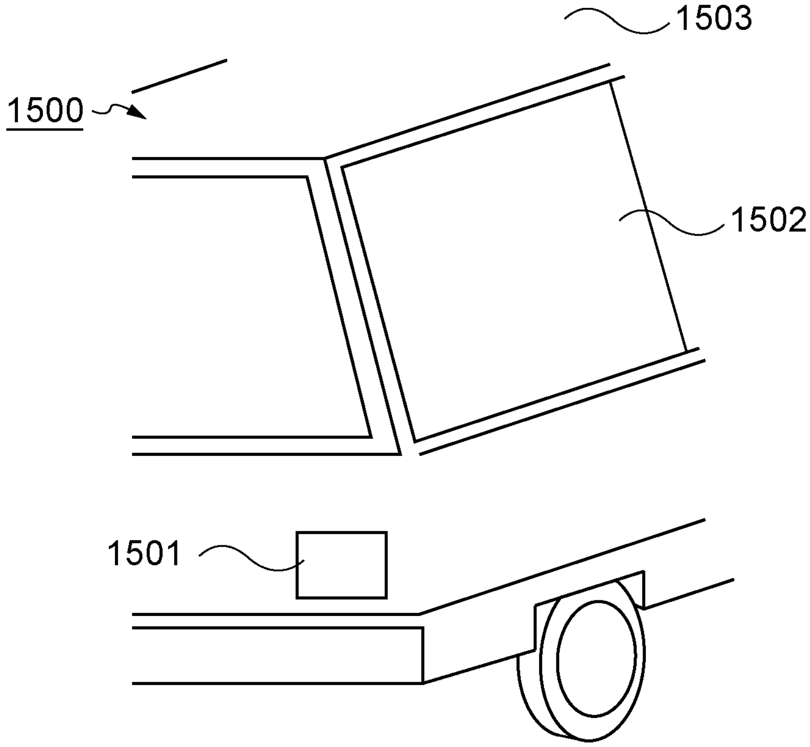

FIG. 6A

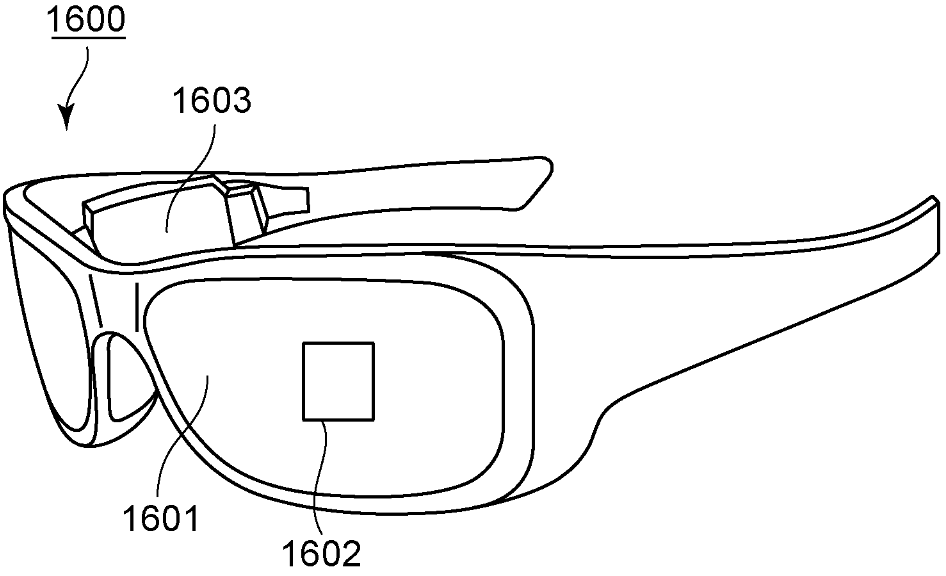


FIG. 6B

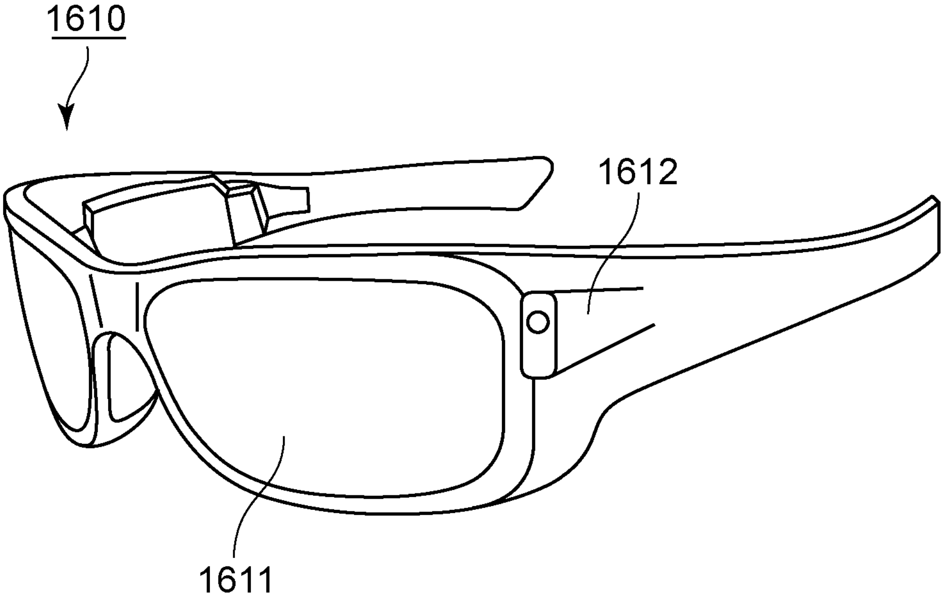

FIG. 7

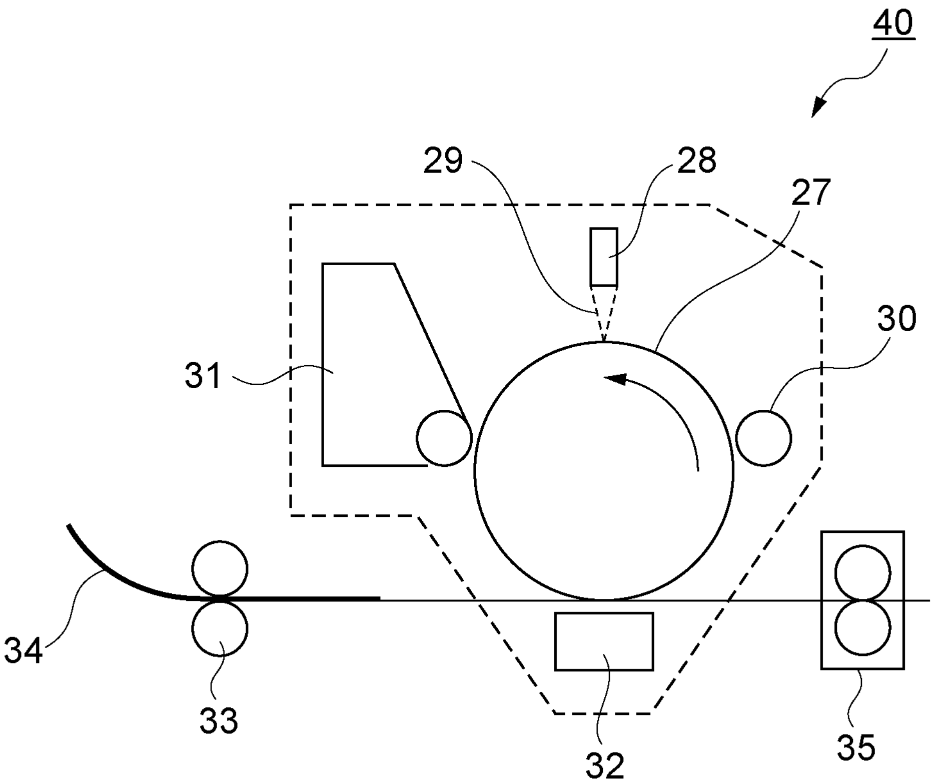

FIG. 8A

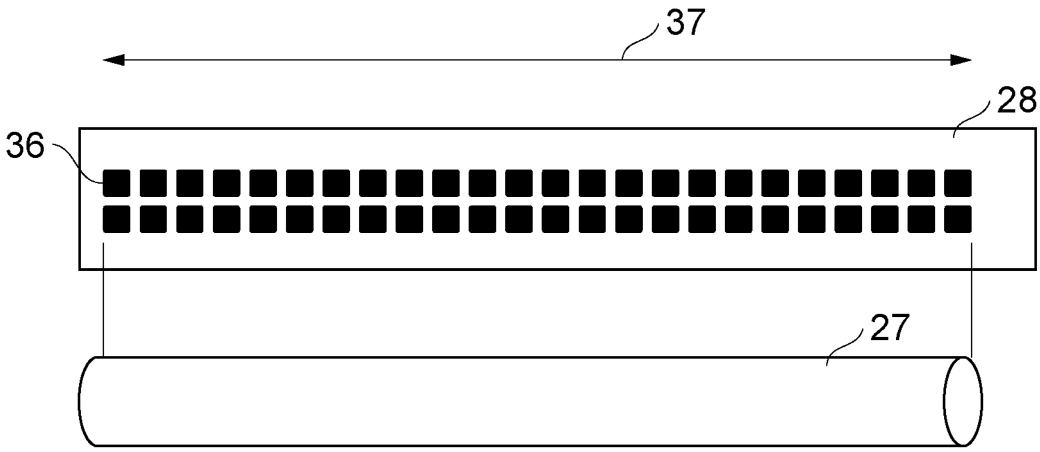


FIG. 8B

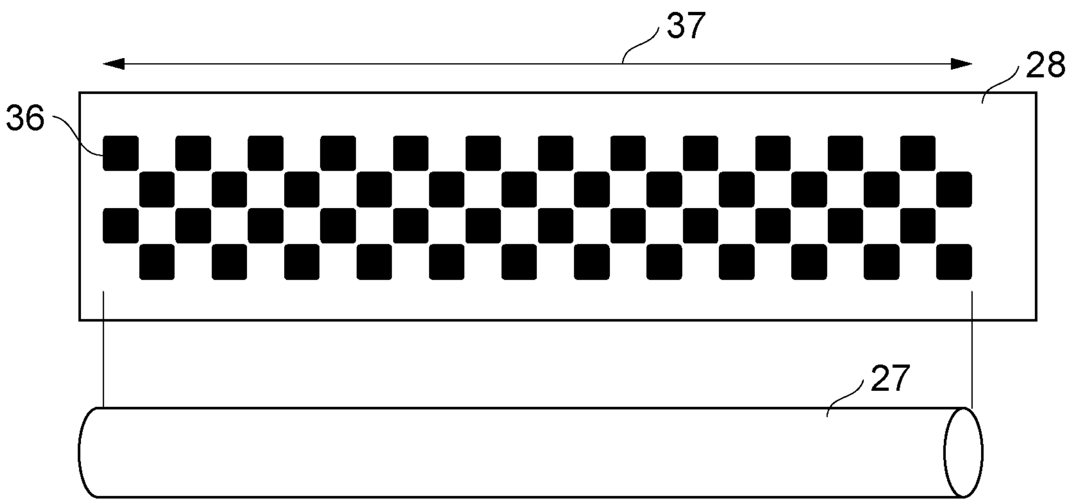

## INTERNATIONAL SEARCH REPORT

International application No.
**PCT/JP2022/039309**

**A. CLASSIFICATION OF SUBJECT MATTER**

***C07C 15/38***(2006.01)i; ***C07C 15/27***(2006.01)i; ***C07D 213/16***(2006.01)i; ***C07D 221/18***(2006.01)i; ***C07D 307/91***(2006.01)i; ***C07D 333/76***(2006.01)i; ***C07D 401/04***(2006.01)i; ***C09K 11/06***(2006.01)i; ***H05B 33/12***(2006.01)i; ***H10K 50/00***(2023.01)i
FI: C07C15/38 CSP; C07C15/27; C07D213/16; C07D221/18; C07D307/91; C07D333/76; C07D401/04; C09K11/06 690; H05B33/12 C; H05B33/12 E; H05B33/14 B

According to International Patent Classification (IPC) or to both national classification and IPC

**B. FIELDS SEARCHED**

Minimum documentation searched (classification system followed by classification symbols)

C07C15/38; C07C15/27; C07D213/16; C07D221/18; C07D307/91; C07D333/76; C07D401/04; C09K11/06; H01L51/50; H05B33/12

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | JP 2008-543086 A (UNIVERSAL DISPLAY CORP.) 27 November 2008 (2008-11-27)<br>entire text | 1-22 |
| A | WO 2012/050008 A1 (FUJIFILM CORP.) 19 April 2012 (2012-04-19)<br>entire text | 1-22 |
| A | WO 2012/033063 A1 (FUJIFILM CORP.) 15 March 2012 (2012-03-15)<br>entire text | 1-22 |
| A | JP 2017-119682 A (SEMICONDUCTOR ENERGY LAB. CO., LTD.) 06 July 2017 (2017-07-06)<br>entire text | 1-22 |
| A | JP 2020-105155 A (SAMSUNG ELECTRONICS CO., LTD.) 09 July 2020 (2020-07-09)<br>entire text | 1-22 |

☐ Further documents are listed in the continuation of Box C.　☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 December 2022** | **13 December 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2022/039309**

| Patent document cited in search report | Publication date (day/month/year) | Patent family member(s) | Publication date (day/month/year) |
|---|---|---|---|
| JP 2008-543086 A | 27 November 2008 | US 2006/0280965 A1<br>entire text<br>WO 2006/130598 A2<br>EP 1888708 B1<br>KR 10-2008-0013934 A<br>CN 101203583 A | |
| WO 2012/050008 A1 | 19 April 2012 | JP 2012-104509 A<br>KR 10-2013-0097784 A | |
| WO 2012/033063 A1 | 15 March 2012 | US 2013/0270530 A1<br>entire text<br>JP 2012-80063 A | |
| JP 2017-119682 A | 06 July 2017 | US 2017/0186971 A1<br>entire text<br>WO 2017/109637 A1<br>CN 108431010 A<br>KR 10-2018-0095919 A | |
| JP 2020-105155 A | 09 July 2020 | US 2020/0212307 A1<br>entire text<br>KR 10-2020-0083200 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006130598 A
- WO 2012050008 A
- JP 2021184897 A


### Non-patent literature cited in the description


- **M.J. FRISCH ; G.W. TRUCKS ; H.B. SCHLEGEL ; G.E. SCUSERIA ; M.A. ROBB ; J.R. CHEESEMAN ; G. SCALMANI ; V. BARONE ; B. MENNUCCI ; G.A. PETERSSON.** Gaussian 09, Revision C. 01. Gaussian, Inc., Wallingford CT, 2010